# EUROPEAN PATENT APPLICATION

(11) **EP 3 138 851 A1**
(43) Date of publication of application: **08.03.2017**
(21) Application number: 16186315.4
(22) Date of filing: 30.08.2016
(51) Int. Cl.: C07K 7/02, C08L 75/02

(54) **QUATERNARY ASSEMBLIES OF WATER-SOLUBLE NON-PEPTIDE HELICAL FOLDAMERS, THEIR USE AND PRODUCTION THEREOF**

(30) Priority: 01.09.2015 US 201562212590 P
(71) Applicant: Centre National de la Recherche Scientifique CNRS, 75794 Paris Cedex 16 (FR); UREKA Sarl, 68100 Mulhouse (FR)
(72) Inventor: Guichard, Gilles, 33170 Gradignan (FR); Collie, Gavin, Horrabridge, Yelverton, PL20 7RQ (GB); Pulka-Ziach, Karolina, 02-775 Warsaw (PL); Lombardo, Caterina, 03039 Sora (IT); Fremaux, Juliette, 33600 Pessac (FR)
(74) Representative: Kasche, André

(57) **Abstract**

The present description provides compositions and methods for producing therapeutic oligomeric compounds. In another aspect the description provides methods for administering the oligomeric compounds for the treatment and prevention of disease in a mammal. In particular, the disclosure relates to medicaments comprising various novel oligomeric compounds and pharmaceutically acceptable salts thereof. The compounds of the disclosure may optionally be administered with at least one of a pharmaceutically acceptable excipient, additional pharmacologically active agent or a combination thereof.

## Description

### CROSS-REFERENCE TO RELATED APPLICTIONS

This application claims priority to U.S. Provisional Application Serial No. 62/212,590, file on September 1, 2015, entitled "QUATERNARY ASSEMBLIES OF WATER-SOLUBLE NON-PEPTIDE HELICAL FOLDAMERS, THEIR USE AND PRODUCTION THEREOF", the entire contents of which are incorporated herein by reference in its entirety and for all purposes.

### INCORPORATION BY REFERENCE

In compliance with 37 C.F.R. § 1.52(e)(5), the sequence information contained in electronic file name: 1513195_129US2_Sequence_Listing_ST25.txt; size 2.55 KB; created on: 29 August 2016; using Patent-In 3.5, and Checker 4.4.0 is hereby incorporated herein by reference in its entirety.

### FIELD OF THE INVENTION

The present description relates to α-helicomimetic foldamers, and their synthesis. In particular, the description provides short amphiphlic α-helicomimetic foldamers bearing proteinaceous sidechains that can form a series of diverse quaternary arrangments, which can be controlled at the sequence level.

### BACKGROUND

Interactions between proteins and/or their substrates or ligands are critical for normal cell function, physiologic signal transduction, as well as for therapeutic intervention in many pathophysiologic or disease-related processes. Proteins and peptides are capable of adopting compact, well-ordered conformations, and performing complex chemical operations, e.g., catalysis, highly selective recognition, etc. The three dimensional structure is the principal determinant that governs specificity in protein-protein and/or protein-substrate interactions. Thus, the conformation of peptides and proteins is central for their biological function, pharmaceutical efficacy, and their therapeutic preparation.

Protein folding is inextricably linked to function in both proteins and peptides because the creation of an "active site" requires proper positioning of reactive groups. Consequently, there has been a long-felt need to identify synthetic polymer or oligomers, which display discrete and predictable (i.e., stable) folding and oligomerizing propensities (hereinafter referred to as "foldamers") to mimic natural biological systems. Insofar as these unnatural backbones are resistant to the action of proteases and peptidases, they are useful as probes having constrained conformational flexibility or as therapeutics with improved pharmacological properties, e.g., pharmacokinetic (PK) and/or pharmacodynamics (PD) features, such as potency and/or half-life. Whereas a naturally occurring polypeptide comprised entirely of α-amino acid residues will be readily degraded by any number of proteases and peptidases, foldamers, including chimeras of natural peptides and synthetic amino acid derivatives, mimetics or pseudopeptides, are not.

As noted above, the interest in foldamers stems in part from their resistance to enzymatic degradation. They are also interesting molecules because of their conformational behavior. The elucidation of foldamers having discrete conformational propensities akin to those of natural proteins has led to explorations of peptides constructed from β-, γ-, or δ-amino acids. γ-Peptides containing residues bearing γ-substitution or α, γ-disubstitution or α, β, γ-trisubstitution have been shown to adopt a helical conformation defined by a 14-member turn that is stabilized by C=O_{(*i*)}→NH_{(*i*+*3*)} hydrogen bonds. Both the 3₁₄ and 2.5₁₂ helical backbones have been found suitable for the design of stabilized helical peptides useful for therapeutic purposes. For example, in order to cluster polar residues on one face of the helix, amphiphilic 3₁₄-helical β-peptides have been constructed from hydrophobic-cationic-hydrophobic- or hydrophobic-hydrophobic-cationic residue triads.

Considerable effort has been applied towards the development of artificial bio-inspired systems able to form predictable and homogeneous assemblies at the nanometer scale in aqueous conditions (Zhang, F., Nangreave, J., Liu, Y. & Yan, H. Structural DNA nanotechnology: state of the art and future perspective. J. Am. Chem. Soc. 136, 11198-11211 (2014); Lai, Y.-T., King, N. P. & Yeates, T. O. Principles for designing ordered protein assemblies. Trends Cell Biol. 22, 653-661 (2012); King, N. P. et al. Computational design of self-assembling protein nanomaterials with atomic level accuracy. Science 336, 1171-1174 (2012); Lai, Y.-T., Cascio, D. & Yeates, T. O. Structure of a 16-nm cage designed by using protein oligomers. Science 336, 1129 (2012); Gradišar, H. & Jerala, R. Self-assembled bionanostructures: proteins following the lead of DNA nanostructures. J. Nanobiotechnology 12, 4 (2014); Lai, Y.-T. et al. Structure of a designed protein cage that self-assembles into a highly porous cube. Nat. Chem. 6, 1065-1071 (2014); King, N. P. et al. Accurate design of co-assembling multi-component protein nanomaterials. Nature 510, 103-108 (2014); Fletcher, J. M. et al. Self-assembling cages from coiled-coil peptide modules. Science 340, 595-599 (2013); Tebo, A. G. & Pecoraro, V. L. Artificial metalloenzymes derived from three-helix bundles. Curr. Opin. Chem. Biol. 25C, 65-70 (2015); Gradišar, H. et al. Design of a single-chain polypeptide tetrahedron assembled from coiled-coil segments. Nat. Chem. Biol. 9, 362-366 (2013); and Bromley, E. H. C., Channon, K., Moutevelis, E. & Woolfson, D. N. Peptide and protein building blocks for synthetic biology: from programming biomolecules to self-organized biomolecular systems. ACS Chem. Biol. 3, 38-50 (2008)). Although building units based on short peptides possess a number of advantages for nano-scale assembly - including synthetic availability, modularity and sequence diversity (Fletcher, J. M. et al. Self-assembling cages from coiled-coil peptide modules. Science 340, 595-599 (2013); Tebo, A. G. & Pecoraro, V. L. Artificial metalloenzymes derived from three-helix bundles. Curr. Opin. Chem. Biol. 25C, 65-70 (2015); Gradišar, H. et al. Design of a single-chain polypeptide tetrahedron assembled from coiled-coil segments. Nat. Chem. Biol. 9, 362-366 (2013); and Bromley, E. H. C., Channon, K., Moutevelis, E. & Woolfson, D. N. Peptide and protein building blocks for synthetic biology: from programming biomolecules to self-organized biomolecular systems. ACS Chem. Biol. 3, 38-50 (2008)) - one limitation resides in the intimate connection between primary sequence and secondary structure. In this respect, non-natural synthetic oligomers designed to form predictable and well-defined secondary structures akin to those found in proteins - i.e. 'foldamers'(Gellman, S. H. Foldamers: A Manifesto. Acc. Chem. Res. 31, 173-180 (1998); Goodman, C. M., Choi, S., Shandler, S. & DeGrado, W. F. Foldamers as versatile frameworks for the design and evolution of function. Nat. Chem. Biol. 3, 252-262 (2007); and Guichard, G. & Huc, I. Synthetic foldamers. Chem. Commun. 47, 5933-5941 (2011)) - obey different folding rules and thus provide new opportunities for creating self-assembled architectures with topologies similar to and beyond those of natural polypeptides. Additionally, in contrast to α-peptides, fully non-natural biopolymer backbones are likely - and in some cases proven - to be resistant to naturally occurring proteases (Johnson, L. M. & Gellman, S. H. α-Helix mimicry with α/β-peptides. Methods Enzymol. 523, 407-429 (2013); and Frackenpohl, J., Arvidsson, P. I., Schreiber, J. V. & Seebach, D. The outstanding biological stability of beta- and gamma-peptides toward proteolytic enzymes: an in vitro investigation with fifteen peptidases. Chembiochem 2, 445-455 (2001)), a highly desirable characteristic for systems intended for bio-applications.

Advances in foldamer research have led to the discovery of a wide range of oligomeric backbones predisposed to adopt well-defined secondary structures, yet few reports have described the aqueous assembly of foldamers into homogeneous tertiary or quaternary arrangements - such examples being limited to the creation of *de novo* octameric helical bundles (Daniels, D. S., Petersson, E. J., Qiu, J. X. & Schepartz, A. High-resolution structure of a beta-peptide bundle. J. Am. Chem. Soc. 129, 1532-1533 (2007); and Wang, P. S. P., Nguyen, J. B. & Schepartz, A. Design and high-resolution structure of a β3-peptide bundle catalyst. J. Am. Chem. Soc. 136, 6810-6813 (2014)) and nanofibers (Pizzey, C. L. et al. Characterization of nanofibers formed by self-assembly of beta-peptide oligomers using small angle x-ray scattering. J. Chem. Phys. 129, 095103 (2008); and Pomerantz, W. C. et al. Nanofibers and lyotropic liquid crystals from a class of self-assembling beta-peptides. Angew. Chem. Int. Ed Engl. 47, 1241-1244 (2008)) formed from short amphiphilic helical β-peptides, and hybrid α/β-peptide tetrameric helical bundles obtained by reengineering the dimerization domain of the yeast GCN4 transcription factor (Home, W. S., Price, J. L., Keck, J. L. & Gellman, S. H. Helix bundle quaternary structure from alpha/beta-peptide foldamers. J. Am. Chem. Soc. 129, 4178-4180 (2007); and Giuliano, M. W., Home, W. S. & Gellman, S. H. An alpha/beta-peptide helix bundle with a pure beta3-amino acid core and a distinctive quaternary structure. J. Am. Chem. Soc. 131, 9860-9861 (2009)). Sequence remodeling of these backbones to yield well-defined yet dissimilar topologies has not been documented, except a rearrangement from parallel to anti-parallel helix topology in the α/β peptide bundle series (Giuliano, M. W., Home, W. S. & Gellman, S. H. An alpha/beta-peptide helix bundle with a pure beta3-amino acid core and a distinctive quaternary structure. J. Am. Chem. Soc. 131, 9860-9861 (2009)). Current aqueous foldamer quaternary assemblies are thus limited in terms of: 1) backbone diversity; 2) divergence from nature; 3) diversity of topology and, importantly; 4) control. An additional - more general - limitation is the paucity of high-resolution structural data available for aqueous foldamer quaternary assemblies (Daniels, D. S., Petersson, E. J., Qiu, J. X. & Schepartz, A. High-resolution structure of a beta-peptide bundle. J. Am. Chem. Soc. 129, 1532-1533 (2007);Wang, P. S. P., Nguyen, J. B. & Schepartz, A. Design and high-resolution structure of a β3-peptide bundle catalyst. J. Am. Chem. Soc. 136, 6810-6813 (2014); Home, W. S., Price, J. L., Keck, J. L. & Gellman, S. H. Helix bundle quaternary structure from alpha/beta-peptide foldamers. J. Am. Chem. Soc. 129, 4178-4180 (2007); and Giuliano, M. W., Horne, W. S. & Gellman, S. H. An alpha/beta-peptide helix bundle with a pure beta3-amino acid core and a distinctive quaternary structure. J. Am. Chem. Soc. 131, 9860-9861 (2009)) - and water-soluble foldamers in general. As such, there is a deficiency of pre-existing structures for use as templates for the design of new aqueous quaternary (and tertiary) assemblies.

Aliphatic oligoureas are a class of non-peptide α-helicomimetic foldamer which possess several features conducive for their use as self-organizing biomimetic building units. The chemical accessibility of the urea-based monomers permits the synthesis of urea oligomers bearing, yet not limited to, any of the 20 naturally occurring amino acid side-chains (Burgess, K., Shin, H. & Linthicum, D. S. Solid-Phase Syntheses of Unnatural Biopolymers Containing Repeating Urea Units. Angew. Chem. Int. Ed. Engl. 34, 907-909 (1995); and Douat-Casassus, C., Pulka, K., Claudon, P. & Guichard, G. Microwave-enhanced solid-phase synthesis of N,N'-linked aliphatic oligoureas and related hybrids. Org. Lett. 14, 3130-3133 (2012)). Importantly, the helicity of urea oligomers is largely unaffected by the nature of the side-chains used, making these foldamers highly robust and tunable (Fischer, L. et al. The canonical helix of urea oligomers at atomic resolution: insights into folding-induced axial organization. Angew. Chem. Int. Ed Engl. 49, 1067-1070 (2010); and Violette, A. et al. N,N'-linked oligoureas as foldamers: chain length requirements for helix formation in protic solvent investigated by circular dichroism, NMR spectroscopy, and molecular dynamics. J. Am. Chem. Soc. 127, 2156-2164 (2005)). In addition, oligoureas as short as 4-6 residues in length are able to adopt stable helical structures (Nelli, Y. R., Fischer, L., Collie, G. W., Kauffmann, B. & Guichard, G. Structural characterization of short hybrid urea/carbamate (U/C) foldamers: A case of partial helix unwinding. Biopolymers 100, 687-697 (2013)), bestowing an added dimension of versatility to these foldamers as self-assembling building blocks.

The design and construction of biomimetic self-assembling systems is a challenging yet potentially highly rewarding endeavour, contributing to the development of new biomaterials, catalysts, drug-delivery systems and tools for the manipulation of biological processes. While much has been achieved by engineering self-assembling DNA-, protein- and peptide-based building units, the design of entirely new, fully non-natural folded architectures resembling biopolymers ("foldamers") with the ability to self-assemble into atomically precise nano-structures in aqueous conditions has proved exceptionally challenging. Therefore, there exists a need for fully non-natural folded architectures resembling biopolymers ("foldamers") with the ability to self-assemble into atomically precise nano-structures in aqueous conditions.

### SUMMARY

Oligoureas represent interesting classes of peptidomimetic foldamers that have previously received little attention. The present disclosure relates to the surprising and unexpected discovery that *de novo* design and subsequent sequence manipulation of non-peptide oligourea helical foldamers results in the controllable formation of diverse protein-like higher-order structures in aqueous conditions, ranging from discrete helical bundles with isolated cavities to pH-responsive super-helical nanotubes with channels or water-filled pores. The quaternary arrangements of the present disclosure represent the first examples of molecular self-assembly of fully non-peptidic foldamers into three-dimensional nanostructures in aqueous conditions, and are unprecedented in terms of topological diversity and programmability. In particular, the description provides a modular design, formation and structural elucidation at the atomic level of a series of diverse quaternary arrangements formed by the self-assembly of short amphiphilic α-helicomimetic foldamers bearing proteinaceous side-chains. Furthermore, the final quaternary assembly can be controlled at the sequence level, permitting the programmed formation of, e.g., either discrete helical bundles containing isolated cavities or pH-responsive water-filled channels with controllable pore diameters. That is, in an embodiment, the pore diameters can be altered by modifying the non-peptide oligourea peptidomimetic residues.

In one aspect, the description provides a compound comprising aliphatic oligoureas. In certain embodiments, the the aliphatic oligoureas form an oligourea helical bundle. In other embodiments, the aliphatic oligoureas are comprised of short amphiphilic α-helicomimetic foldamers with proteinaceous sidechains. In an embodiment, the short amphiphilic α-helicomimetic foldamers self-assemble into the oligourea helical bundle. In a particular embodiment, the foldamers self-assemble under aqueous conditions. Because the compounds as described herein can adopt desired secondary structures similar to native peptides, including, e.g., helicoidal structures, they can serve as, for example, receptor ligands, effector molecules, agonists, antagonists, modulators of protein-protein interactions, organocatalysts, or enzymes.

In one aspect, the description provides oligourea compounds that comprise an oligourea helical bundle and a peptide. The peptide can be a peptide α-helix. In certain embodiments, the peptide comprises an epitope that recognizes another compound, e.g. they can act as receptor ligands, effector molecules, agonists, antagonists, modulators of protein-protein interactions, organocatalysts, or enzymes.

In additional asepcts, the description provides peptide-oligourea chimeric compounds. The chimeric compound comprises a non-peptide oligourea helical foldamer and a peptide α-helix. In an embodiment, the non-peptide oligourea helical foldamer comprises a non-peptide an oligourea helical bundle. The oligourea helical bundle is coupled, joined to or contiguous with the peptide α-helix. In certain embodiments, the oligourea helical bundle is "fused" to a terminus, e.g., amino terminus, carboxy terminus or both, of an α-amino acid peptide.

In one aspect, the present disclose provides a quaternary assembly. The quaternary assembly comprising non-peptide helical foldamers. According to an embodiment, the quaternary assembly's final topology is directed by manipulation of the primary sequence. In a certain embodiment, the quaternary assembly is either a helical bundle or a super-helical (e.g., nanotubular) water-filled channel. In a particular embodiment, the helical bundle comprises a cavity or a pore defining a volume. In another embodiment, the super-helical nanotubular channel defines a water-filled channel and has a tunable diameter.

According to an embodiment, the non-peptide helical foldamer is a catalyst with tailored substrate specificity. In a particular embodiment, non-peptide helical foldamers are formed from water-soluble foldamers. According to an embodiment, the quaternary assembly has an isolated internal cavity or large water-filled pores. In an embodiment, the non-peptide helical foldamers encapsulate, at least partially, a drug or a substrate. In other embodiments, the quaternary assemblies are designed/configured for the conductance of water or (metal) ion cargo across a membrane. In a particular embodiment, the internal cavity of the oligourea helical bundle has a volume of about 500 Å³. In certain embodiments, the quaternary assembly further comprises a peptide. In certain embodiments, the non-peptide helical foldamer is "fused" to a terminus, e.g., amino terminus, carboxy terminus or both, of the peptide. The peptide can be a peptide α-helix.

In certain aspects, the description provides peptide-oligourea chimeras that adopt stable secondary structures, including, e.g., helicoidal, tertiary structure, and/or quaternary structures, wherein the chimeras comprise a peptide contiguous with or coupled to a sequence of non-peptide helical foldamers.

In certain aspects, the description provides a non-peptide helical foldamer or a oligourea helical bundle capable of binding specifically to a target, e.g., a protein such as a receptor or other polypeptide or peptide, or small molecule, similar to the native or natural peptide. In another aspect, the description provides a chimeric ligand compound comprising oligourea helical bundle or a non-peptide helical foldamers and a peptide capable of binding specifically to a target, e.g., a protein such as a receptor or other polypeptide or peptide, or small molecule, similar to the native or natural peptide. In an additional embodiments, the chimeric ligand compound peptide is contiguous with or coupled to a non-peptide helical foldamer.

In any of the embodiments described herein, the peptide-oligourea chimeric foldamer comprises non-peptide helical foldamers contiguous with or covalently linked or joined to at least one of the amino terminus (N'), the carboxyl terminus (C'), within the peptide sequence or a combination thereof, of the peptide. In a preferred embodiment, the peptide-oligourea chimeric compound comprises an oligourea portion covalently linked or joined to the C-terminus of the peptide portion.

In any aspect or embodiment described herein, the compounds can further comprise at least one additional chemical modification. In certain embodiments, the chemical modification includes at least one of, for example, acetylation, phosphorylation, methylation, glycosylation, prenylation, isoprenylation, farnesylation, geranylation, pegylation, a disulfide bond, or combination thereof.

In an additional aspect, the description provides pharmaceutically acceptable acid and base salt forms of the compounds described herein.

The oligourea foldamers and compounds as described herein, including pharmaceutically acceptable salts thereof, are useful for the preparation of a medicament and/or the treatment of disease in a subject. The compounds of the disclosure may optionally be administered with at least one of a pharmaceutically acceptable excipient, pharmacologically active agent or a combination thereof. As such, in an addition aspect the description provides compositions comprising an effective amount of a peptide-oligourea chimera or oligourea foldamers as described herein, and a pharmaceutically acceptable carrier or excipient.

The description also provides methods of treating a disease or disorder or ameliorating the effects of the same comprising the steps of administering to an individual in need thereof, a composition comprising an effective amount of a peptide-oligourea chimera, oligourea foldamers, a oligourea compound, or salt form thereof as described herein, and a pharmaceutically acceptable carrier or excipient, wherein the composition is effective for treating, preventing or ameliorating the effects of the disease or disorder.

In another aspect, the present description provides methods of making and using the compounds or the oligourea foldamer compounds as described herein. For example, the oligourea compounds or oligourea foldamers as described herein can be used as a diagnostic agent or a therapeutic agent for the treatment of a disease or condition.

In an additional aspect the present description provides methods of making oligourea compounds, e.g., oligourea foldamer compounds, or peptide-oligourea compounds as described herein. Thus, in one aspect, the present description provides for the synthesis of a non-peptide helical structure. In another aspect, the present description provides methods of making and using the compounds of the disclosure. For example, in one embodiment, the description provides a method of making an oligourea compound of the disclosure comprising fabricating a oligourea helical bundle with a non-peptide oligourea peptidomimetic residue sequence selected from the group consisting of: a helical pentad repeat (*a, b, c, d, e):* oligourea peptidomimetic residues with a hydrophobic side chain in positions *a* and *d* (e.g., Leu^{u}), oligourea peptidomimetic residues with a charged residue in position *b* and *c* (e.g. Glu^{u} and Lys^{u}, respectively), and Tyr^{u} and Ala^{u} in position *e*; and a helical pentad repeat (*a, b, c, d, e):* oligourea peptidomimetic residues with a hydrophobic side chain in positions a and c (e.g. Leu^{u}), oligourea peptidomimetic residues with a hydrophobic side chain in position e (e.g., Ala^{u} and Pro^{u} residues at the e position), and oligourea peptidomimetic residues with a charged side chain in positions *b* and *d* (e.g., Glu^{u} and Lys^{u}, respectively). In another embodiment, the non-peptide oliguria peptiomimetic residue sequence is selected from the group consisting of: Leu^{U} Glu^{U} Lys^{U} Leu^{U} Tyr^{U} Leu^{U} Glu^{U} Lys^{U} Leu^{U} Ala^{U} Leu^{U} (H1); Leu^{U} Glu^{U} Leu^{U} Lys^{U} Pro^{U} Leu^{U} Glu^{U} Leu^{U} Lys^{U} Ala^{U} (H2); Leu^{U} Glu^{U} Lys^{U} Leu^{U} Tyr^{U} Asn^{U} Glu^{U} Lys^{U} Leu^{U} Ala^{U} Leu^{U} (H3); Ser^{U} Glu^{U} Lys^{U} Leu^{U} Tyr^{U} Leu^{U} Glu^{U} Lys^{U} Leu^{U} Ala^{U} Leu^{U} (H4); Ala^{U} Leu^{U} Lys^{U} Leu^{U} Glu^{U} Tyr^{U} Leu^{U} Glu^{U} Leu^{U} Lys^{U} Ala^{U} Leu^{U} (H5); Leu^{u} Glu^{u} Lys^{u} Leu^{u} Tyr^{u} Leu^{u} Asn^{u} Lys^{u} Leu^{u} Ala^{u} Leu^{u} (H6); and Leu^{u} Glu^{u} Lys^{u} Leu^{u} Tyr^{u} Leu^{u} Gln^{u} Lys^{u} Leu^{u} Ala^{u} Leu^{u} (H7).

The preceding general areas of utility are given by way of example only and are not intended to be limiting on the scope of the present disclosure and appended claims. Additional objects and advantages associated with the compositions, methods, and processes of the present disclosure will be appreciated by one of ordinary skill in the art in light of the instant claims, description, and examples. For example, the various aspects and embodiments of the disclosure may be utilized in numerous combinations, all of which are expressly contemplated by the present description. These additional advantages objects and embodiments are expressly included within the scope of the present disclosure. The publications and other materials used herein to illuminate the background of the invention, and in particular cases, to provide additional details respecting the practice, are incorporated by reference, and for convenience are listed in the appended bibliography.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

The accompanying drawings, which are incorporated into and form a part of the specification, illustrate several embodiments of the present disclosure and, together with the description, serve to explain the principles of the disclosure. The drawings are only for the purpose of illustrating an embodiment of the disclosure and are not to be construed as limiting the disclosure. Further objects, features and advantages of the disclosure will become apparent from the following detailed description taken in conjunction with the accompanying figures showing illustrative embodiments of the disclosure, in which:

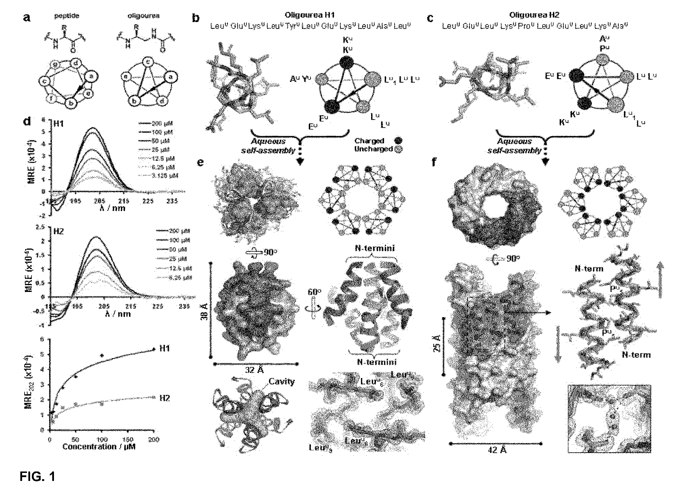

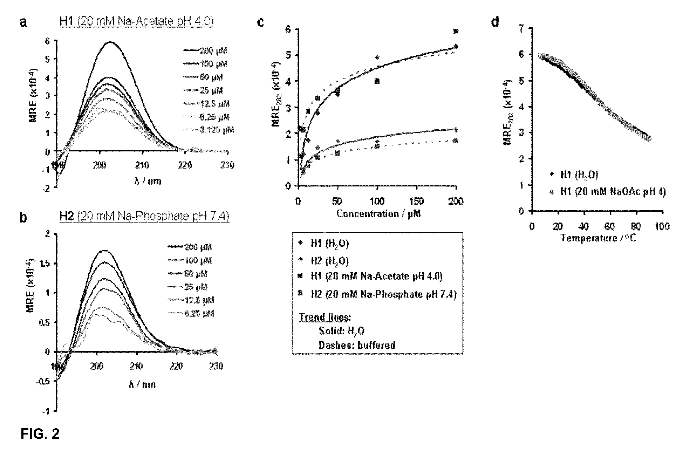

**Figure 1****. Quaternary assemblies formed from short water-soluble oligourea foldamers:** Fig. 1A. Helical-wheel representations of α-peptides and aliphatic oligoureas. Fig. 1B. Primary sequence of **H1** (top), view down helical axis of the crystal structure of **H1** (bottom left) and helical wheel showing side-chain distribution of **H1** (bottom right). Superscript 'u' denotes urea-based residue. Fig. 1C Equivalent information as in B for oligourea **H2.** Fig. 1D Variable-concentration circular dichroism (CD) analysis of oligoureas **H1** and **H2** (in pure water) (MRE: molar residual ellipticity [deg.cm².dmol⁻¹.residue⁻¹], MRE₂₀₂: molar residual ellipticity at 202 nm). Trend lines are shown to guide the eye only, and are not fit to mathematical models. Equivalent CD studies of **H1** and **H2** performed in suitable buffered conditions yield data highly similar to data collected in pure water (See Fig. 2). Fig. 1E. Crystal structure of a hexameric helical bundle formed from **H1.** Leu-type side-chains are shown as green spheres (top left). Middle panels: helices colored according to common chain orientation. Bottom left: internal cavity delineated in green (volume, 491.3 Å³, calculated using SURFNET). Bottom right: electron density surrounding interlocked Leu-type (Leu^{u}) side-chains of hydrophobic core (2Fₒ-F_{c} map, σ level: 1.4, resolution: 1.25 Å). (F) Crystal structure of channel-type assembly formed from oligourea **H2.** The two individual super-helix chains of the channel are colored separately. Inset: electron density surrounding a single mobile water molecule (red spheres) within the pore region of **H2** channel (2Fₒ-F_{c} map, σ level: 1.4, resolution: 1.40 Å).
**Figure 2****.** Circular dichroism (CD) analysis of oligoureas **H1** and **H2** in buffered conditions. Fig. 1A. Variable concentration CD spectra of **H1** in 20 mM sodium acetate (Na-acetate) pH 4.0. Concentration range measured: 3.125 µM to 200 µM. MRE: molar residual ellipticity (deg.cm².dmol⁻¹.residue⁻¹). Fig. 1B. Equivalent experiments as (a) performed on **H2** in 20 mM sodium phosphate (Na-Phosphate) at pH 7.4 (3.125 µM not measured due to weak signal intensity). Fig. 1C. Plots of oligourea concentration vs MRE₂₀₂ (molar residual ellipticity at 202 nm) for oligoureas **H1** (blue markers) and **H2** (green markers) measured in pure water (solid lines) and buffered conditions (dashed lines). Trend lines are not fit to specific mathematical models, and are shown to guide the eye only. Fig. 1D. Comparison of CD-monitored thermal melting profiles of **H1** in pure water (black) and 20 mM sodium acetate (NaOAc) pH 4.0 (grey). Fitting these melting curves to a simple two-state Boltzmann unfolding model provides Tₘ values of 45.9 °C and 48.9 °C for **H1** measure in pure water and 20 mM sodium acetate (pH 4), respectively (adjusted R-square values: 0.99943 [pure water] and 0.99871 [sodium acetate buffer]).

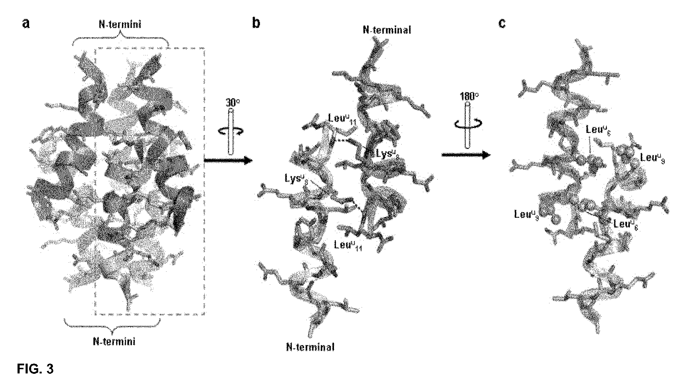

**Figure 3****.** Crystal structure of oligourea **H1:** intermolecular interactions of hexameric helical-bundle formation. Fig. 3A. Hexameric helical bundle formed from oligourea **H1.** Helices colored according to common helix orientation. Fig. 3B. Helix-dimer building block of helical bundle. The hexameric helical bundle is formed from three identical helical dimers. Fig. 3B. shows the hydrophilic dimer interface. Key inter-helical hydrogen bonds between the amine of Lys^{u}₈ and the free carbonyl group of Leu^{u}₁₁ are indicated (dashes). Fig. 3C. Hydrophobic Leu^{u}-rich interface of the helical dimer, highlighting the knobs-into-holes type packing of the Leu^{u} side-chains (shown as spheres).

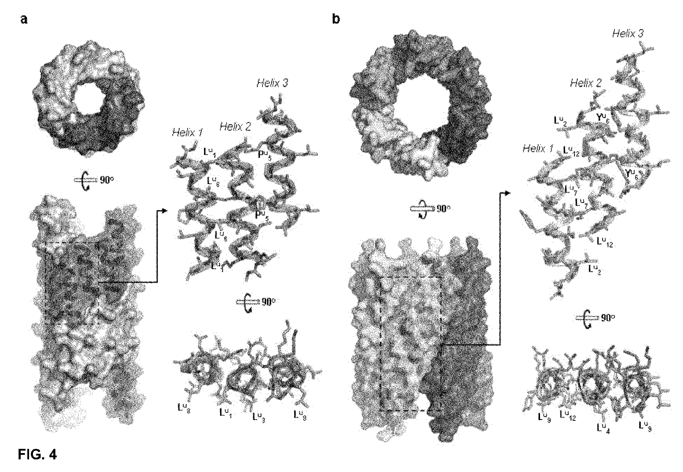

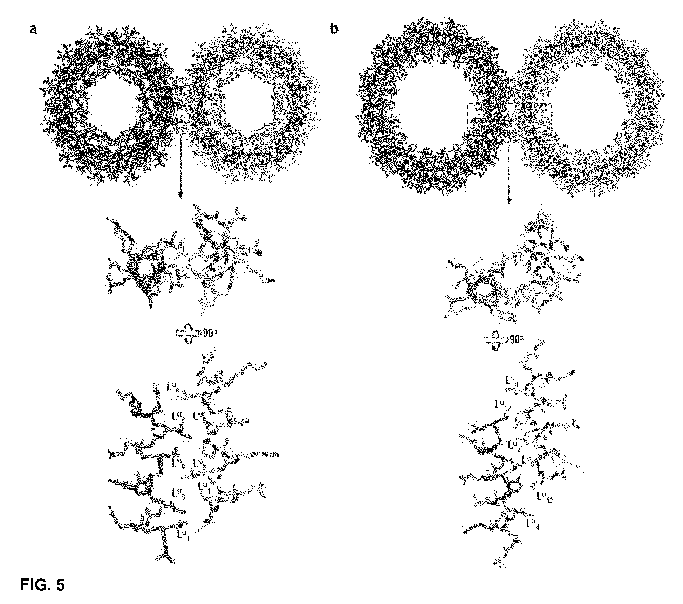

**Figure 4****.** Crystal structures of **H2** and **H5:** comparison of the construction of channel-type assemblies formed from oligoureas **H2** Fig. 4A and **H5** 4B. Despite differing considerably in sequence, the common distribution of charged and uncharged residues around the helical circumferences of oligoureas **H2** and **H5** results in a surprisingly similar mechanism of inter-helical interactions for channels **H2** and **H5,** involving helices packing laterally in an anti-parallel staggered fashion through hydrophobic interactions, creating extended super-helices. Super-helix chains are colored separately in 4A and 4B. Inter-channel hydrophobic packing interactions are illustrated and discussed in Figure 5.
**Figures 5A and 5B****.** Crystal structures of **H2** (5A) and **H5** (5B) showing details of inter-channel hydrophobic packing interactions. Individual neighboring channels of both channel-type arrangements (i.e. **H2** and **H5**) interact through dense hydrophobobic packing contacts primarily involving the precise inter-locking of Leucine-type (L^{u}) side-chains.
**Figure 6A and 6B****.** Crystal structures of **H2** (6A) and **H5** (6B) illustrating electrostatic contacts within the charged pores of the channel-type quaternary assemblies. Inter-helical salt-bridges within the interior channel pores of both channel crystals structures link adjacent as well as distant helices forming 'zipper-type' salt-bridge networks of electrostatic contacts. Distinct oligourea molecules are distinguished by carbon color.

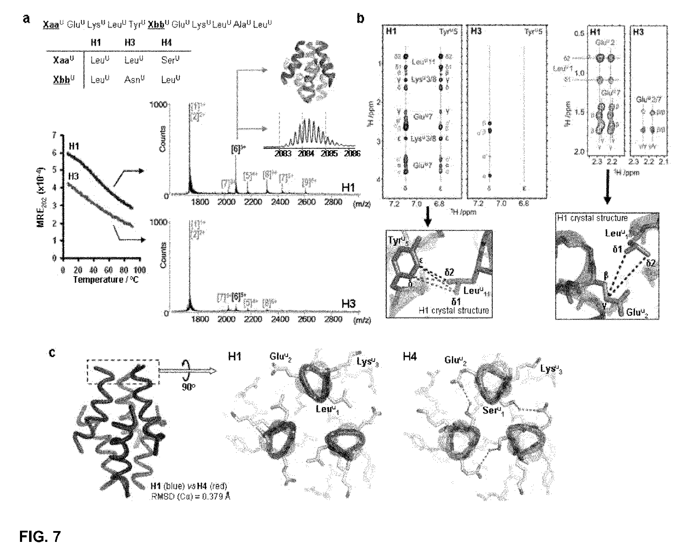

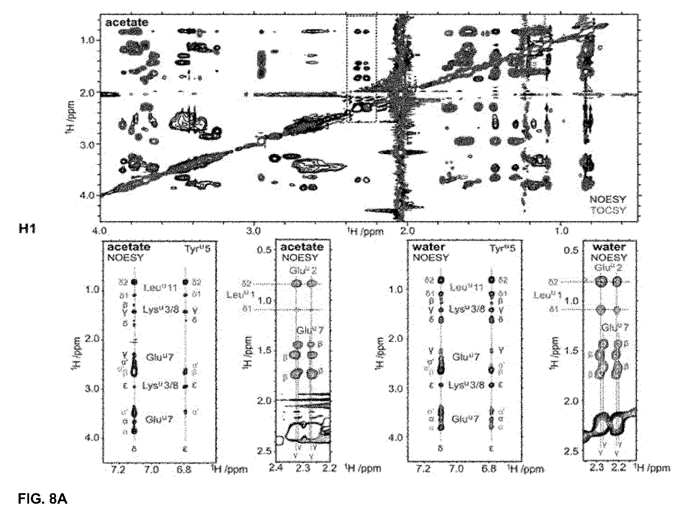

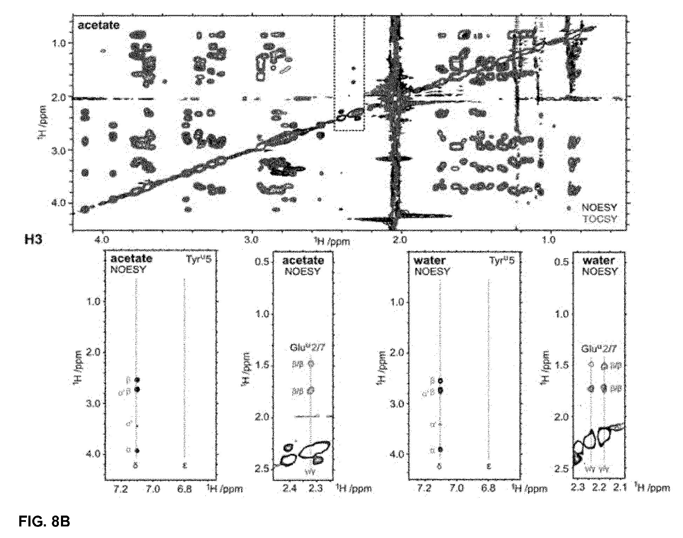

**Figure 7****. Solution and solid-state studies of oligourea helical-bundle formation.** Fig. 7A Top: sequences of oligoureas **H1, H3** and **H4.** Superscript 'u' denotes urea-based residue. Bottom: circular dichroism (CD) monitored thermal melting of oligoureas **H1** (blue) and **H3** (negative control - green), and accompanying native electrospray ionization mass spectrometry (ESI-MS) analysis. The major isotopic distribution at *m*/*z* = 2083.4 for **H1** shows isotope spacing of 0.2 *m*/*z* (inset), and therefore corresponds unambiguously to a hexameric species ([**H1**_{6]}⁵⁺). Ion counts for these spectra are normalized (to 1000 counts) to permit comparison between **H1** and **H3.** Foldamers were analyzed at a concentration of 200 µM in pure water for CD studies and 100 µM in 20 mM ammonium acetate for ESI-MS studies. MRE₂₀₂: molar residual ellipticity at 202 nm (with units of deg.cm².dmor⁻¹.residue⁻¹). Fig. 7B. NOESY spectra (200 ms mixing time) of **H1** and **H3** (measured at 200 µM foldamer concentration in 100 % D₂O at 20 °C), showing strong evidence of intermolecular interactions for **H1,** and an absence of intermolecular interactions for **H3.** Red labels indicate unambiguous intermolecular interactions. The intermolecular interactions of **H1** correlate excellently with the crystal structure (insets, NOE peaks shown as dashes). Note slight difference in Glu^{u}₂ rotamer conformation between NMR and X-ray data. NOESY experiments performed on **H1** and **H3** in buffered conditions (20 mM sodium acetate pH 4.0, 98 % D₂O) yield spectra almost identical to spectra recorded in pure water (see Figure 8). Fig. 7C. Structural alignment of crystal structures of helical bundles formed from **H1** (blue) and **H4** (red), plus separate views onto N-tenninal junctions of each bundle (middle and right panels).
**Figure 8****.** Through-bond (¹H,¹H-TOCSY) and through-space (¹H,¹H-NOESY) spectra for the characterization of H1 (top) and H3 (bottom) in solution. Overlay of the NOESY (200 ms mixing time, black spectra) and TOCSY (80 ms mixing time, red spectra) demonstrate that only H1 has NOE crosspeaks between ¹H nuclei that are long-range and not simply constrained by one or two bonds. For H3 only the geminal ¹Hα' nuclei bound to the backbone urea Cα' show significant NOE crosspeaks (∼ 2.8 ppm and ∼ 3.2 ppm). Close-up views of the NOESY spectra for the Tyr^{u}5 ¹Hδ and ¹Hε aromatic protons and the Glu^{u 1}Hγ aliphatic protons show a similar behavior for H1 and H3 in sodium acetate buffer (20 mM sodium acetate, pH 4.0, 98% ²H₂O) as compared to water (100% ²H₂0). The water spectra are the same as those in Figure 2b. Note: side-chain atomic nomenclature here follows that of natural amino acid side-chains.

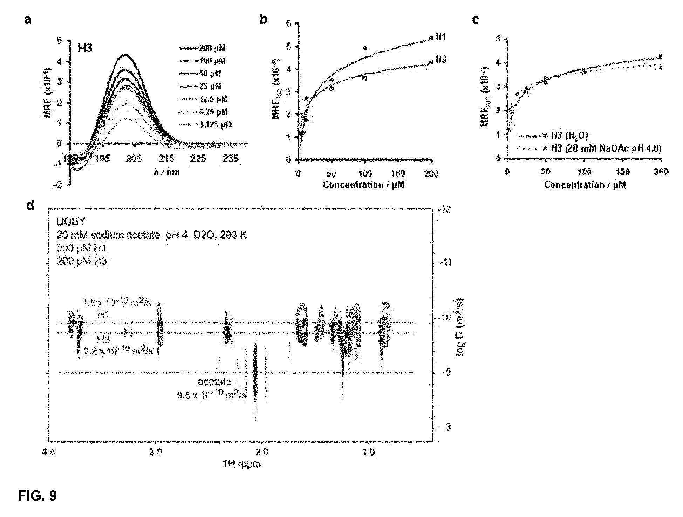

**Figure 9****.** Circular dichroism (CD) and DOSY analysis of oligourea **H3** and comparison to **H1.** Fig. 9A. Variable concentration CD spectra of **H3** in double-distilled H₂O. Concentration range measured: 3.125 µM to 200 µM. MRE: molar residual ellipticity (deg.cm².dmor¹.residue⁻¹). Fig. 9B. Plots of oligourea concentration *vs* MRE₂₀₂ (molar residual ellipticity at 202 nm) for oligoureas **H1** (blue diamonds) and **H3** (red squares). Fig. 9C. Comparison of the effect of buffer on the variable-concentration CD profile of **H3.** Square symbols plus solid line corresponds to spectra of **H3** recorded in water only, triangles plus dashed line corresponds to spectra of **H3** recorded in 20 mM sodium acetate (NaOAc) at pH 4.0. As with **H1** and **H2,** the variable-concentration CD profile of **H3** in pure water is very similar to that of **H3** obtained from buffered conditions. Fig. 9D. DOSY NMR analysis of 200 µM **H1** or **H3** in 20 mM sodium acetate (pH 4.0), 98 % D₂O, and at a temperature of 293 K. The two spectra were collected under identical conditions and used a diffusion time of 250 ms and a diffusion gradient strength of 3 ms with a linear distribution of 16 points using gradient powers from 2 % to 95 %. The average diffusion for each molecule is illustrated with a dotted line, and the numerical values are from a curve fit using TopSpin 2.1 (Bruker) of the methyl region at ∼ 0.8 ppm for **H1** and **H3,** or the main peak of acetate at 2.08 ppm. The acetate exhibited the same diffusion rate in both samples.

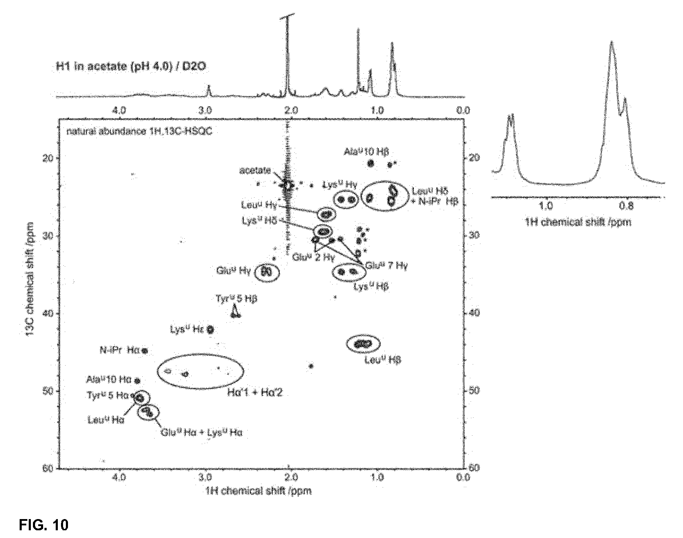

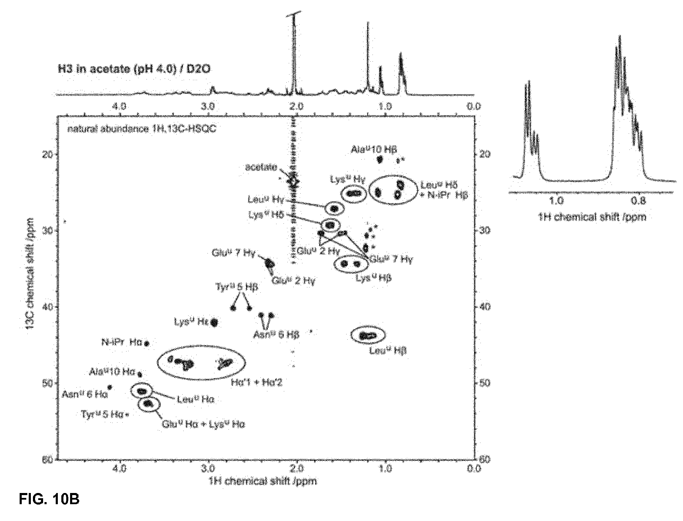

**Figure 10****.** Annotated natural abundance ¹H,¹³C-HSQC spectra for **H1** (top) and H3 (bottom). Each sample contains 200 µM oligourea monomers resuspended in 20 mM sodium acetate buffer, pH 4.0, with 98% ²H₂O. The spectra were collected at 800 MHz at a temperature of 293 K. Chemical shift assignments were based on ¹H,¹H-TOCSY (80 ms mixing time) and ¹H,¹H-NOESY (200 ms mixing time) spectra. Above each HSQC is a representative ¹H one-dimensional spectrum, with a closeup view of the ¹H methyl region shown to the right. Note that the line-widths of the methyl groups in **H1** are significantly broadened as compared to **H3,** which is consistent with the formation of an assembled multimeric complex with **H1,** whereas **H3** has narrow line-widths as expected for a monomeric species. There is a high degree of degeneracy in most residues, as noted for example by the narrow range of the backbone Leu^{u 1}Hα-¹³Cα chemical shifts as well as the remaining Leu^{u} aliphatic nuclei for the five Leu^{u} sidechains in **H1** and the four Leu^{u} in **H3.** Peaks with an asterisk derive from impurities in the sodium acetate buffer. Side-chain atomic nomenclature here follows that of natural amino acid side-chains.

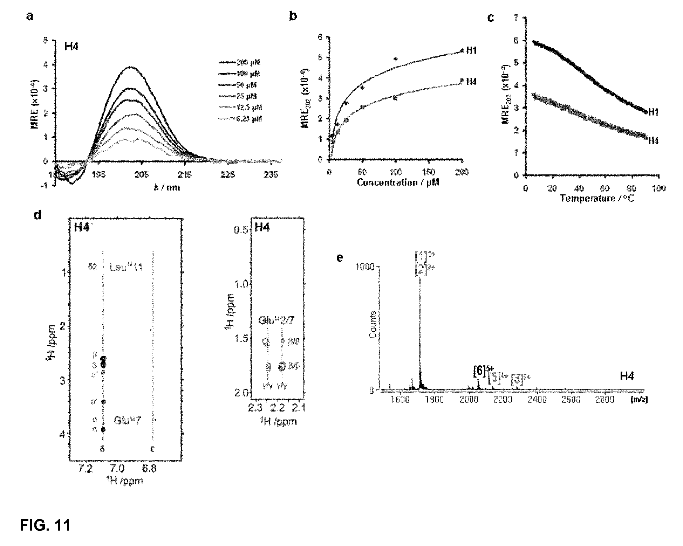

**Figure 11****.** Solution studies of oligourea **H4.** Fig. 11A. Variable concentration circular dichroism (CD) spectra of **H4** in double-distilled H₂O. Concentration range measured: 6.25 µM to 200 µM. MRE: molar residual ellipticity (deg.cm².dmol⁻¹.residue⁻¹). Fig. 11B. Plots of oligourea concentration vs MRE₂₀₂ (molar residual ellipticity at 202 nm) for oligoureas **H1** (blue diamonds) and **H4** (green squares). Fig. 11C. Comparison of CD-monitored thermal melting of oligoureas **H1** (blue) and **H4** (green). Foldamers were analyzed at a concentration of 200 µM in double-distilled H₂O. The CD signal at a wavelength of 202 nm was monitored for these experiments. Fig. 11D. NOESY NMR spectra of **H4** (200 µM at 20 °C in 100 % D₂O): a weak peak corresponding to intermolecular interactions between the δ2 proton of Leu^{u}₁₁ and the δ proton of Tyr^{u}₅ can be seen (red label), providing some, albeit weak, evidence of the presence of specific intermolecular interactions for this foldamer. Fig. 11E. Native electrospray ionization mass spectrometric analysis of a 100 µM solution of **H4** in 20 mM aqueous ammonium acetate: the predominant multimer peak (with a stoichiometry greater than dimer) corresponds to a hexameric species (m/z = 2050.8 Da, with a charge state of 5⁺).

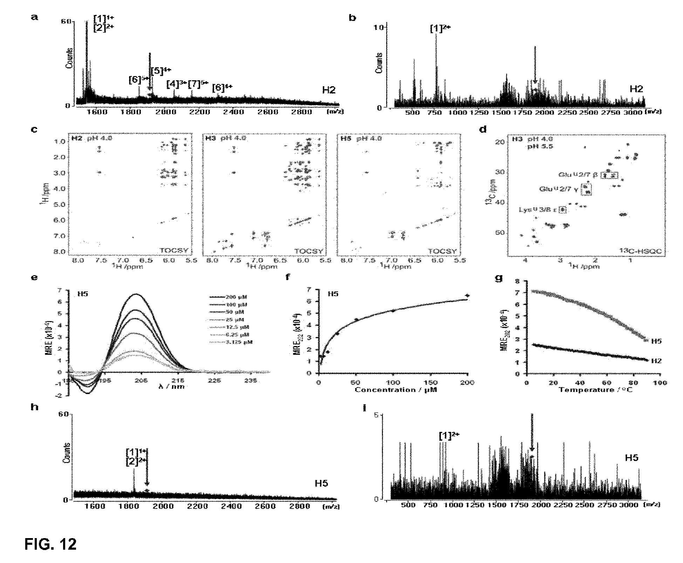

**Figure 12****.** Solution studies of channel-forming oligoureas **H2** and **H5.** Fig. 12A. Native electrospray ionization (ESI) mass spectrum of **H2** at 100 µM in 20 mM aqueous ammonium acetate. The ESI mass spectrum reveals extremely low levels of multimeric species (note the ion count on the Y axis, which is normalized to 60 to permit comparison to **H5**) with a concomitant high level of background 'noise'. MS-MS analysis of an *m*/*z* arbitrarily selected in the noisy region (indicated by arrow and diamond) reveals this background signal to be composed of **H2** monomers Fig. 12B, suggesting that **H2** exists predominantly in a multimerized/aggregated yet polydisperse form in these conditions. Fig. 12C. TOCSY NMR spectra of oligoureas **H2, H3** and **H5** at pH 4. At pH values above 5.5, the NMR spectra of **H2** show no clear signals, consistent with the severe line-broadening due to the large size of the assembled channels. In contrast, NMR spectra of **H2** at pH 4.0 (left TOCSY spectra) reveals the presence of small multimers or monomers with properties more similar to the monomeric **H3** (middle). The spectra of **H5** at pH 4.0 (right) also shows the presence of small multimers that appear to be on average larger than for **H2.** Fig. 12D. Using natural abundance 1H,13C-HSQC spectra for **H3** at both pH 4.0 and pH 5.5, the only chemical shift differences are observed for the glutamate-urea side-chains, consistent with a change in protonation state of the carboxylate group. We would anticipate the protonation state of the glutamate-urea side-chains of oligoureas **H2** and **H5** to respond in a similar manner to equivalent changes in pH. Fig. 12E. Variable concentration circular dichroism (CD) spectra of **H5** in double-distilled H₂O. Concentration range measured: 3.125 µM to 200 µM. MRE: molar residual ellipticity (deg.cm².dmor¹.residue⁻¹). Fig. 12F. Plot of oligourea concentration vs MRE₂₀₂ (molar residual ellipticity at 202 nm) of oligourea **H5.** Fig. 12G. Comparison of CD-monitored thermal melting of **H2** (blue data points) and **H5** (magenta data points) (foldamers analysed at a concentration of 200 µM in double-distilled H₂O). Fig. 12H. ESI-MS analysis of **H5** under the same conditions as **H2** (i.e. as in [a]) reveals a total absence of discrete multimeric species with stoichiometries above dimer. In addition, there is a high level of background 'noise'. The corresponding MS-MS spectrum of a arbitrarily selected *w*/*z* (indicated by arrow and diamond) is shown in Fig. 12I, indicating that the background signal in 12H is composed of aggregated **H5** monomers.

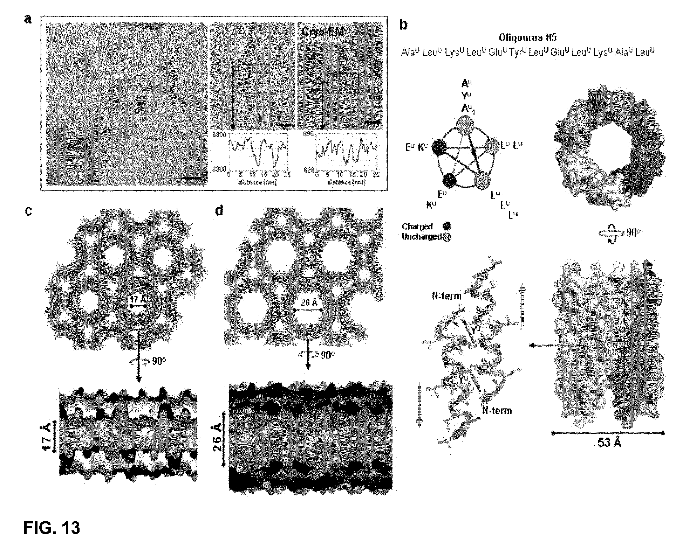

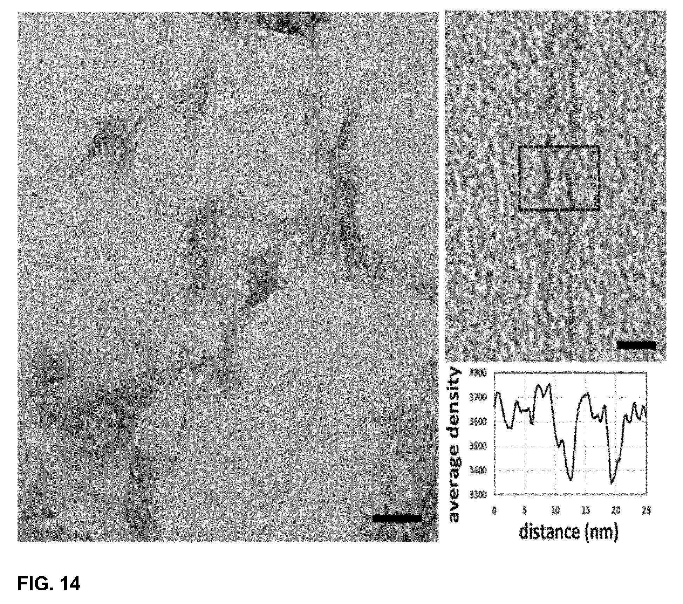

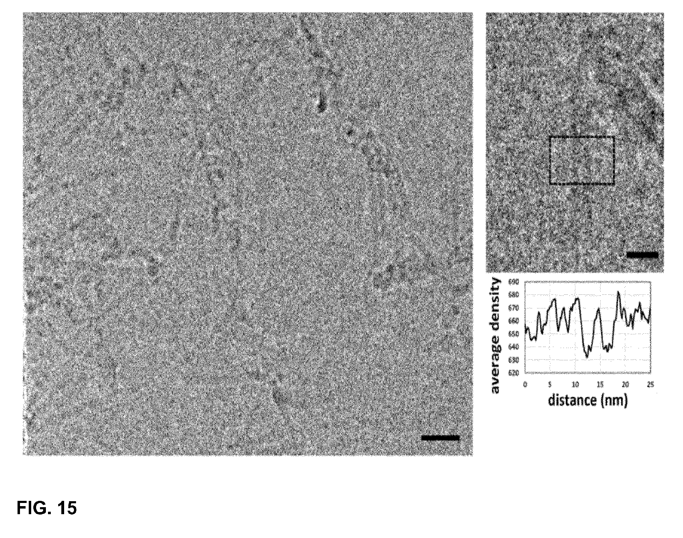

**Figure 13****. Solution and solid-state studies of channel-forming oligourea foldamers** H2 **and** H5. Fig. 13A. Negative staining electron microscopy (EM) and cryo-EM analysis of **H2** (at a concentration of 200 µM in 50 mM sodium HEPES buffer, pH 7.5). Left: wide-field negative staining EM image of **H2** revealing the presence of extended tubule structures (scale bar: 50 nm). Middle: magnification of a single tubule from the left panel with accompanying density analysis. Right: cryo-EM analysis of **H2** (at 200 µM in 50 mM sodium HEPES buffer, pH 7.5) plus accompanying density analysis revealing the presence of tubule structures comparable to those seen by negative staining EM. Scale bars for middle and right panels: 10 nm. Y axes of plots denote average density. For additional EM images see Figs. 14 and 15. Fig. 13B. Primary sequence and crystal structure of a 12-mer oligourea foldamer **(H5)** bearing a distribution of charged and uncharged side-chains analogous to oligourea **H2** (superscript 'u' denotes urea-based residue). The crystal structure of **H5** reveals a channel-type assembly constructed from six super-helices (individual super-helix chains colored separately) - comparable yet somewhat larger than the **H2**-channel. Fig. 13C. Crystal packing of **H2** showing the mechanism by which multiple channels interact in the crystal state: individual channels pack into extended arrays *via* their hydrophobic exterior surfaces. The cross-section shows the dimensions and overall water structure of the internal pore. Residues are colored according to the helical wheel diagram in Figure 1c. Waters shown as red spheres. Fig. 13D. Equivalent crystal packing of **H5,** highlighting the increased internal and external channel dimensions compared to **H2.** Color scheme as for helical wheel diagram in panel 13B. Additional details of channel packing interactions can be found in Figs. 4 and 5.
**Figure 14**. Negative staining electron microscopy analysis of **H2:** enlarged view of Figure. 3a, left and middle panels. **H2** analyzed at a concentration of 200 µM in a buffer composed of 50 mM sodium HEPES, pH 7.5.
**Figure 15****.** Cryo-electron microscopy analysis of H2: enlarged view of Fig. 3a, right panel. H2 analyzed at a concentration of 200 µM in a buffer composed of 50 mM sodium HEPES, pH 7.5.
**Figure 16****.** Control of quaternary arrangement of oligourea foldamers through manipulation of primary sequence.
**Figure 17****.** ¹H NMR (300MHz) of compound **(3).**
**Figure 18****.** ¹³C NMR (75MHz) of compound **(3).**
**Figure 19****.** ¹H NMR (300MHz) of compound (**4**).
**Figure 20****.** ¹³C NMR (75MHz) of compound **(4).**
**Figure 21****.** ¹H NMR (300MHz) of compound **(11).**
**Figure 22****.** ¹H NMR (300MHz) of compound (**17**).
**Figure 23****.** Chemical structure, HPLC profile and electrospray ionization mass spectrum of oligourea **H2.**
**Figure 24****.** ¹H NMR (300MHz) of compound (**20**).
**Figure 25****.** ¹³C NMR (75MHz) of compound (**20**).
**Figure 26****.** ¹H NMR (300MHz) of compound **(21).**
**Figure 27****.** ¹³C NMR (75MHz) of compound **(21).**
**Figure 28****.** ¹H NMR (300MHz) of compound **(22).**
**Figure 29****.** ¹³C NMR (75MHz) of compound **(22).**
**Figure 30****.** ¹H NMR (300MHz) of compound (**23**).
**Figure 31****.** ¹³C NMR (75MHz) of compound **(23).**
**Figure 32****.** Chemical structure, HPLC profile and electrospray ionization mass spectrum of oligourea **H1.**
**Figure 33****.** Chemical structure, HPLC profile and electrospray ionization mass spectrum of oligourea **H3.**
**Figure 34****.** Chemical structure, HPLC profile and electrospray ionization mass spectrum of oligourea **H4.**
**Figure 35****.** Chemical structure, HPLC profile and electrospray ionization mass spectrum of oligourea **H5.**
**Figure 36****.** Comparison of hexamer (H1) and octamer (H1+) dimer.

### DETAILED DESCRIPTION

The following is a detailed description of the disclosure provided to aid those skilled in the art in practicing the present disclosure. Those of ordinary skill in the art may make modifications and variations in the embodiments described herein without departing from the spirit or scope of the present disclosure. Unless otherwise defined, all technical and scientific terms used herein have the same meaning as commonly understood by one of ordinary skill in the art to which this disclosure belongs. The terminology used in the description of the disclosure herein is for describing particular embodiments only and is not intended to be limiting of the disclosure. All publications, patent applications, patents, figures and other references mentioned herein are expressly incorporated by reference in their entirety.

U.S. Provisional Patent Application No. 61/868,128 filed 21 August 2013 titled: Oligourea Foldamer Organocatalysts and Methods of Their Use; U.S. Provisional Patent Application No. 61/887,651 filed 07 October 2013 titled: Peptide-Oligourea Chimeric Compounds and Methods of Their Use; U.S. Patent Application No. 14/465680 filed 21 August 2014 titled: Peptide-oligourea Chmerica Compounds and Methods of their Use; and Collie, G.W., et al. (Gavin W. Collie, Karolina Pulka-Ziach, Caterina M. Lombardo, Juliette Fremaux, Frédéric Rosu, Marion Decossas, Laura Mauran, Olivier Lambert, Valérie Gabelica, Cameron D. Mackereth and Gilles Guichard. Shaping quaternary assemblies of water-soluble non-peptide helical foldamers by sequence manipulation. Nature Chemistry. _, _-_ (2015)), are hereby incorporated by reference in their entirety for all purposes. Furthermore, the disclosures of each and every patent, patent application, and publication cited herein are hereby incorporated herein by reference in their entirety.

The present disclosure related to non-peptide helical foldamers with the ability to self-assemble to an oligourea helical bundle and/or a super-helical nanotubular structure with an internal channel or pore. According to an embodiment, the final topology of a quaternary assembly is directed by manipulation of the primary sequence. In a certain embodiment, the quaternary assembly is either finite helical bundles or super-helical tube with water-filled channels. In a particular embodiment, the finite helical bundles or super-helical water-filled channels have tunable dimensions, e.g., volume or diameter, respectively. According to an embodiment, the foldamer is a catalyst with tailored substrate specificity. As structure and function are intimately linked, the creation of new and unique foldamer architectures is an important step towards this goal. The higher-order assemblies described herein are unique in terms of backbone composition and quaternary topology.

The channel-type assemblies described here represent the first examples of such entities formed from water-soluble foldamers to be characterized at atomic resolution. The stoichiometry (and topology) of the helical bundle reported herein (hexameric) is rare among both natural and non-natural helical bundles (Zaccai, N. R. et al. A de novo peptide hexamer with a mutable channel. Nat. Chem. Biol. 7, 935-941 (2011)). Importantly, these unique quaternary assemblies are accompanied by unique structural characteristics (isolated internal cavities and large water-filled pores) which could be tailored to specific function(s). For example, in an embodiment, the non-peptide helical foldamers encapsulate a drug or a substrate (Yadav, M. K. et al. Structure-based engineering of internal cavities in coiled-coil peptides. Biochemistry 44, 9723-9732 (2005); Liu, R., Loll, P. J. & Eckenhoff, R. G. Structural basis for high-affinity volatile anesthetic binding in a natural 4-helix bundle protein. FASEB J. 19, 567-576 (2005); and Ghirlanda, G. et al. Volatile anesthetic modulation of oligomerization equilibria in a hexameric model peptide. FEBS Lett. 578, 140-144 (2004)), or are designed/configured for the conductance of a substance , e.g., water or (metal) ion cargo, across a membrane (Joh, N. H. et al. De novo design of a transmembrane Zn2+-transporting four-helix bundle. Science 346, 1520-1524 (2014)). Several examples of internal cavities engineered into peptide coiled-coils have been reported, with cavity volumes in the range of 200-300 Å³ for the few structures for which high-resolution data is available (Yadav, M. K. et al. Structure-based engineering of internal cavities in coiled-coil peptides. Biochemistry 44, 9723-9732 (2005); and Liu, R., Loll, P. J. & Eckenhoff, R. G. Structural basis for high-affinity volatile anesthetic binding in a natural 4-helix bundle protein. FASEB J. 19, 567-576 (2005)). With volumes of about 500 Å³, the cavities observed in the crystal structures of **H1** and **H4** reported here are significantly larger and thus, more amenable to future development for various applications, such as the design of selective catalysts (Tegoni, M., et al. Designing a functional type 2 copper center that has nitrite reductase activity within α-helical coiled coils. Proc. Natl. Acad. Sci. U. S. A. 109, 21234-21239 (2012); and Faiella, M. et al. An artificial di-iron oxo-protein with phenol oxidase activity. Nat. Chem. Biol. 5, 882-884 (2009)).

Unlike previously reported aqueous self-assembling foldamers (Daniels, D. S., et al. High-resolution structure of a beta-peptide bundle. J. Am. Chem. Soc. 129, 1532-1533 (2007); Wang, P. S. P., et al. Design and high-resolution structure of a β3 -peptide bundle catalyst. J. Am. Chem. Soc. 136, 6810-6813 (2014); Pizzey, C. L. et al. Characterization of nanofibers formed by self-assembly of beta-peptide oligomers using small angle x-ray scattering. J. Chem. Phys. 129, 095103 (2008); Pomerantz, W. C. et al. Nanofibers and lyotropic liquid crystals from a class of self-assembling beta-peptides. Angew. Chem. Int. Ed Engl. 47, 1241-1244 (2008); Horne, W. S., et al. Helix bundle quaternary structure from alpha/beta-peptide foldamers. J. Am. Chem. Soc. 129, 4178-4180 (2007); and Giuliano, M. W., et al. An alpha/beta-peptide helix bundle with a pure beta3-amino acid core and a distinctive quaternary structure. J. Am. Chem. Soc. 131, 9860-9861 (2009)), the presently described system allows for the control of the final quaternary assembly by manipulating the primary sequence, permitting the creation of dramatically different yet equally unique three-dimensional structures (discrete helical bundles or extended channel-type assemblies of defined pore diameter) with a high degree of precision. Due to: 1) the simplicity of the design; 2) the short length of the helical components; and 3) the tunability of the reported assemblies (a result of the independence of oligourea helicity on primary sequence), the approach described herein can be easily implemented for the creation of structures with a broader range of topologies (and stoichiometries), functional properties and, consequently, applications. In addition, the similarity in screw-sense, pitch and polarity between peptide α-helices and oligourea 2.5-helices allow these two backbones to be combined, permitting key beneficial features of both species - such as natural epitope recognition of α-peptides and the innate helical stability of oligoureas - to be exploited in a single chimeric construct (Fremaux, J. et al. α-Peptide/Oligourea Chimeras: Stabilization of Short α-helices by Non Peptide Helical Foldamers. Angew. Chem. Int. Ed. Engl. (2015). DOI: 10.1002/anie.201500901R201500901). These goals will be aided significantly by the wealth of high-resolution structural information (X-ray and high-field NMR) generated for the assemblies reported herein, permitting a rational as well as directed approach for the development of new aqueous quaternary assemblies of equal potential utility.

In one aspect, the description provides short oligourea sequences that result in amphiphilic structures with well-defined and distinct hydrophobic and charged regions when folded as canonical 2.5-helices. In certain embodiments, the sequestration of hydrophobic side-chains provide the driving force for self-assembly in acqueous conditions and the complementary charged side-chains facilitate inter-helical interactions. In a particular embodiment, the oligourea has a primary sequence selected from H1 or H2, as shown in Figure 1B or Figure 1C.

In additional aspects, the description provides oligourea compounds comprising an oligourea helical bundle. In a particular embodiment, the oligourea residues are coupled, joined to or contiguous with a peptide. In certain embodiments, the oligourea residues are "fused" to a terminus, e.g., amino terminus, carboxy terminus or both, of an α-amino acid peptide. In an embodiment, the peptide is a peptide α-helix.

The present description relates to the surprising and unexpected discovery that short amphiphilic α-helicomimetic foldamers with proteinaceous side-chains can self-assemble under acqueous conditions to form higher order structures, e.g., oligourea helical bundles and super-helical nanotubes.

In another aspect, the description provides peptide-oligourea chimeric foldamer compounds comprising an oligourea helical bundle comprising non-peptide oligourea peptidomimetic residues; and a peptide. In a certain embodiment, the non-peptide oligourea foldamers include non-peptide oligourea peptidomimetic residues. In a particular embodiment, the peptide includes a peptide α-helix. The chimeric compounds described herein improve at least one of potency, specificity, stability, pharmacokinetic (PK) and/or pharmacodynamics (PD) profile or combinations thereof, of the peptide precursor, e.g., alpha peptide precursor. The peptidomimetics as described herein are resistant or wholly immune to peptidase and protease degradation and are conformationally restrained. Thus, they are useful as tools to model peptide and protein conformations in aqueous solutions. The compounds are also useful as non-enzymatically degradable probes to mimic protein behavior in solution.

In one aspect, the description provides oligourea compounds and peptide-oligourea compounds synthesized using the methods of the disclosure The description also provides pharmaceutical compositions comprising effective amounts of said compounds. In other aspects, the description provides therapeutic methods comprising the administration of an effective amount of the compounds of the disclosure to a mammal in need thereof

Where a range of values is provided, it is understood that each intervening value, to the tenth of the unit of the lower limit unless the context clearly dictates otherwise (such as in the case of a group containing a number of carbon atoms in which case each carbon atom number falling within the range is provided), between the upper and lower limit of that range and any other stated or intervening value in that stated range is encompassed within the disclosure. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

The following terms are used to describe the present invention. In instances where a term is not specifically defined herein, that term is given an art-recognized meaning by those of ordinary skill applying that term in context to its use in describing the present invention.

The articles "a" and "an" as used herein and in the appended claims are used herein to refer to one or to more than one (i.e., to at least one) of the grammatical object of the article unless the context clearly indicates otherwise. By way of example, "an element" means one element or more than one element.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of."

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of and "consisting essentially of shall be closed or semi-closed transitional phrases, respectively, as set forth in the 10 United States Patent Office Manual of Patent Examining Procedures, Section 2111.03.

As used herein in the specification and in the claims, the phrase "at least one," in reference to a list of one or more elements, should be understood to mean at least one element selected from anyone or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a nonlimiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

The terms "co-administration" and "co-administering" or "combination therapy" refer to both concurrent administration (administration of two or more therapeutic agents at the same time) and time varied administration (administration of one or more therapeutic agents at a time different from that of the administration of an additional therapeutic agent or agents), as long as the therapeutic agents are present in the patient to some extent, preferably at effective amounts, at the same time. In certain preferred aspects of the present disclosure, one or more of the present compounds described herein, are coadministered in combination with at least one additional bioactive agent. In particularly preferred aspects of the disclosure, the co-administration of compounds results in synergistic activity and/or therapy.

"Peptides" are typically short chains of amino acid monomers linked by peptide (amide) bonds, the covalent chemical bonds formed when the carboxyl group of one amino acid reacts with the amino group of another. The shortest peptides are dipeptides, consisting of 2 amino acids joined by a single peptide bond, followed by tripeptides, tetrapeptides, etc. A polypeptide is a long, continuous, and unbranched peptide chain.

The term "amino" or "amine" as used herein refers to -NH2 and substituted derivatives thereof wherein one or both of the hydrogens are independently replaced with 20 substituents selected from the group consisting of alkyl, haloalkyl, fluoro alkyl, alkenyl, alkynyl, carbocyclyl, heterocyclyl, aryl, aralkyl, hetero aryl , hetero aralkyl , alkylcarbonyl, haloalkylcarbonyl, carbocyclylcarbonyl, fluoroalkylcarbonyl, alkenylcarbonyl, heterocyclylcarbonyl, arylcarbonyl, alkynylcarbonyl, aralkylcarbonyl, heteroarylcarbonyl, heteroaralkylcarbonyl and the sulfonyl and sulfinyl groups defined above; or when both hydrogens together are replaced with an alkylene group (to form a ring which contains the nitrogen). Representative examples include, but are not limited to methylamino, acetylamino, and dimethylamino.

"Amino acid" refers to any molecule that contains both amino and carboxylic acid functional groups, and includes any of the naturally occurring amino acids (e.g. Ala, Arg, Asn, Asp, Cys, Glu, Gln, Gly, His, Hyl, Hyp, Ile, Leu, Lys, Met, Phe, Pro, Ser, Thr, Trp, Tyr, and Val) in D, L, or DL form. The side chains of naturally occurring amino acids are well known in the art and include, for example, hydrogen (e.g., as in glycine), alkyl (e.g., as in alanine, valine, leucine, isoleucine, proline), substituted alkyl (e.g., as in threonine, serine, methionine, cysteine, aspartic acid, asparagine, glutamic acid, glutamine, arginine, and lysine), alkaryl (e.g., as in phenylalanine and tryptophan), substituted arylalkyl (e.g., as in tyrosine), and heteroarylalkyl (e.g., as in histidine). The term is inclusive of various types of amino acids including α-, β-, γ-, or δ-amino acids, analogs and derivatives of the same, unless the context clearly indicates otherwise.

The term "amino acid sidechain" or "amino acid residue" shall mean, within context, a radical of a D- or L-amino acid sidechain (derived from an amino acid) which functions as a substituent on another group, often an alkylene (usually a methylene) group on R2' or R3' as otherwise described herein. Preferred amino acid sidechains for use in the present disclosure are derived from the sidechains of both natural and unnatural amino acids, preferably including, for example, alanine, β-alanine, arginine, asparagine, aspartic acid, cyclohexylalanine, cysteine, cystine, glutamic acid, glutamine, glycine, phenylalanine, histidine, isoleucine, lysine, leucine, methionine, naphthylalanine, norleucine, norvaline, proline, serine, threonine, valine, tryptophan or tyrosine, among others.

Unless the context clearly indicates otherwise, the term "any amino acid" can mean any natural or synthetic amino acid, including α-, β-, γ-, or δ-amino acids, possibly modified by the presence of one or more substituents, or combinations thereof, including analogs, derivatives, mimetics, and peptoid versions of the same. More precisely the term α-amino acid means an alpha aminated amino acid with the following general structure:

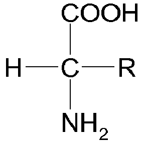

where R represents the side chain of the amino acid. In the context of the disclosure, R therefore represents the side chain of a side or non-side amino acid. The term "natural amino acid" means any amino acid which is found naturally *in vivo* in a living being. Natural amino acids therefore include amino acids coded by mRNA incorporated into proteins during translation but also other amino acids found naturally *in vivo* which are a product or by-product of a metabolic process, such as for example ornithine which is generated by the urea production process by arginase from L-arginine. In the present disclosure, the amino acids used can therefore be natural or not. Namely, natural amino acids generally have the L configuration but also, according to the disclosure, an amino acid can have the L or D configuration. Moreover, R is of course not limited to the side chains of natural amino acid but can be freely chosen.

As used herein, "urea" or carbamide is an organic compound with the chemical formula CO(NH₂)₂. The molecule has two -NH₂ groups joined by a carbonyl (C=O) functional group.

Unless indicated otherwise, the term "peptide precursor" or "parental peptide" refers, but is in no way limited to, a parental α-peptide sequence that is coupled with oligourea pseudopeptide or peptidomimetic subunits or substituting oligourea pseudopeptide subunits (i.e., exchanging one or more α-amino acids for one or more oligourea pseudopeptide subunits).

Unless indicated otherwise, the term "oligourea" refers, but is in no way limited to, a residue containing N, N'-linked urea residues including oligomers of substituted or unsubstituted N-2-ethylaminocarbamoyl or 1, 2-ethylene diamine residues.

The term "compound", as used herein, unless otherwise indicated, refers to any specific chemical compound disclosed herein and includes tautomers, regioisomers, geometric isomers, and where applicable, stereoisomers, including optical isomers (enantiomers) and other steroisomers (diastereomers) thereof, as well as pharmaceutically acceptable salts and derivatives (including prodrug forms) thereof where applicable, in context. Within its use in context, the term compound generally refers to a single compound, but also may include other compounds such as stereoisomers, regioisomers and/or optical isomers (including racemic mixtures) as well as specific enantiomers or enantiomerically enriched mixtures of disclosed compounds. The term also refers, in context to prodrug forms of compounds which have been modified to facilitate the administration and delivery of compounds to a site of activity. It is noted that in describing the present compounds, numerous substituents and variables associated with same, among others, are described. It is understood by those of ordinary skill that molecules which are described herein are stable compounds as generally described hereunder. When the bond is shown, both a double bond and single bond are represented within the context of the compound shown.

The term "hydrocarbyl" shall mean a compound which contains carbon and hydrogen and which may be fully saturated, partially unsaturated or aromatic and includes aryl groups, alkyl groups, alkenyl groups and alkynyl groups.

The term "amido" as used herein means an ammo group, as defined herein, appended to the parent molecular moiety through a carbonyl.

The term "cyano" as used herein means a -C=N group.

The term "nitro" as used herein means a -N02 group.The term "azido" as used herein means a -N3 group.

The abbreviations Me, Et, Ph, Tf, Nf, Ts, Ms, Cbz, and Boc represent methyl, ethyl, phenyl, trifluoromethanesulfonyl, nonafluorobutanesulfonyl, p-toluenesulfonyl, methanesulfonyl, carbobenzyloxy, and tert-butyloxycarbonyl, respectively.

A more comprehensive list of the abbreviations utilized by organic chemists of ordinary skill in the art appears in the first issue of each volume of the *Journal of Organic Chemistry*; this list is typically presented in a table entitled Standard List of Abbreviations, and is incorporated herein by reference.

"Alkyl" refers to a branched or unbranched alkyl group having 1-6 carbon atoms, a branched or unbranched alkenyl group having 1-6 carbon atoms, a branched or unbranched alkinyl group having 1-6 carbon atoms. The term "alkyl" shall mean within its context a linear, branch-chained or cyclic fully saturated hydrocarbon radical or alkyl group, preferably a C1-C10, more preferably a C1-C6, alternatively a C1-C3 alkyl group, which may be optionally substituted. Examples of alkyl groups are methyl, ethyl, n-butyl, sec-butyl, n-hexyl, n-heptyl, n-octyl, n-nonyl, n-decyl, isopropyl, 2-methylpropyl, cyclopropyl, cyclopropylmethyl, cyclobutyl, cyclopentyl, cyclopentylethyl, cyclohexylethyl and cyclohexyl, among others. In certain preferred embodiments, compounds according to the present disclosure which may be used to covalently bind to dehalogenase enzymes. These compounds generally contain a side chain (often linked through a polyethylene glycol group) which terminates in an alkyl group which has a halogen substituent (often chlorine or bromine) on its distil end which results in covalent binding of the compound containing such a moiety to the protein.

The term "Alkenyl" refers to linear, branch-chained or cyclic C2-C10 (preferably C2-C6) hydrocarbon radicals containing at least one C=C bond. The term "Alkynyl" refers to linear, branchchained or cyclic C2-C10 (preferably C2-C6) hydrocarbon radicals containing at least one C≡C bond.

The term "alkylene" when used, refers to a -(CH2)n- group (n is an integer generally from 0-6), which may be optionally substituted. When substituted, the alkylene group preferably is substituted on one or more of the methylene groups with a C1-C6 alkyl group (including a cyclopropyl group or a t-butyl group), more preferably a methyl group, but may also be substituted with one or more halo groups, preferably from 1 to 3 halo groups or one or two hydroxyl groups or O-(C1-C6 alkyl) groups. In certain embodiments, an alkylene group may be substituted with a urethane or alkoxy group (or other group) which is further substituted with a polyethylene glycol chain (of from 1 to 10, preferably 1 to 6, often 1 to 4 ethylene glycol units) to which is substituted (preferably, but not exclusively on the distal end of the polyethylene glycol chain) an alkyl chain substituted with a single halogen group, preferably a chlorine group. In still other embodiments, the alkylene group may be substituted with an amino acid side chain such as group obtained from an amino acid (a natural or unnatural amino acid) such as, for example, alanine, β-alanine, arginine, asparagine, aspartic acid, cysteine, cystine, glutamic acid, glutamine, glycine, phenylalanine, histidine, isoleucine, lysine, leucine, methionine, proline, serine, threonine, valine, tryptophan or tyrosine.

The term "unsubstituted" shall mean substituted only with hydrogen atoms. A range of carbon atoms which includes C0 means that carbon is absent and is replaced with H. Thus, a range of carbon atoms which is C0-C6 includes carbons atoms of 1, 2, 3, 4, 5 and 6 and for C0, H stands in place of carbon. The term "substituted" or "optionally substituted" shall mean independently (i.e., where more than substituent occurs, each substituent is independent of another substituent), one or more substituents (independently, up to five substitutents, preferably up to three substituents, often 1 or 2 substituents on a moiety in a compound according to the present disclosure and may include substituents which themselves may be further substituted) at a carbon (or nitrogen) position anywhere on a molecule within context, and independently includes as substituents hydroxyl, thiol, carboxyl, cyano (C≡N), nitro (NO2), halogen (preferably, 1, 2 or 3 halogens, especially on an alkyl, especially a methyl group such as a trifluoromethyl), an alkyl group (preferably, C1-C10 , more preferably, C1-C6), aryl (especially phenyl and substituted phenyl for example benzyl or benzoyl), alkoxy group (preferably, C1-C6 alkyl or aryl, including phenyl and substituted phenyl), thioether (C1-C6 alkyl or aryl), acyl (preferably, C1-C6 acyl), ester or thioester (preferably, C1-C6 alkyl or aryl) including alkylene ester (such that attachment is on the alkylene group, rather than at the ester function which is preferably substituted with a C1-C6 alkyl or aryl group), preferably, C1-C6 alkyl or aryl, halogen (preferably, F or Cl), amine (including a five- or six-membered cyclic alkylene amine, further including a C1-C6 alkyl amine or a C1-C6 dialkyl amine which alkyl groups may be substituted with one or two hydroxyl groups) or an optionally substituted -N(C0-C6 alkyl)C(O)(O-C1-C6 alkyl) group (which may be optionally substituted with a polyethylene glycol chain to which is further bound an alkyl group containing a single halogen, preferably chlorine substituent), hydrazine, amido, which is preferably substituted with one or two C1-C6 alkyl groups (including a carboxamide which is optionally substituted with one or two C1-C6 alkyl groups), alkanol (preferably, C1-C6 alkyl or aryl), or alkanoic acid (preferably, C1-C6 alkyl or aryl).

The term "substituted" (each substituent being independent of another substituent) shall also mean within its context of use C1-C6 alkyl, C1-C6 alkoxy, halogen, amido, carboxamido, sulfone, including sulfonamide, keto, carboxy, C1-C6 ester (oxyester or carbonylester), C1-C6 keto, urethane -O-C(O)-NR1R2 or-N(R1)-C(O)-O-R1, nitro, cyano and amine (especially including a C1-C6 alkylene-NR1R2, a mono- or di- C1-C6 alkyl substituted amines which may be optionally substituted with one or two hydroxyl groups). Each of these groups contain unless otherwise indicated, within context, between 1 and 6 carbon atoms. In certain embodiments, preferred substituents will include for example, -NH-, -NHC(O)-, -O-,=O, -(CH2)m- (here, m and n are in context, 1, 2, 3, 4, 5 or 6), -S-, -S(O)-, SO2- or -NH-C(O)-NH-, - (CH2)nOH, -(CH2)nSH, -(CH2)nCOOH, C1-C6 alkyl, -(CH2)nO-(C1-C6 alkyl), -(CH2)nC(O)-(C1-C6 alkyl), -(CH2)nOC(O)-(C1-C6 alkyl), -(CH2)nC(O)O-(C1-C6 alkyl), -(CH2)nNHC(O)-R1 -(CH2)nC(O)-NR1R2, -(OCH2)nOH, -(CH2O)nCOOH, C1-C6 alkyl, -(OCH2)nO-(C1-C6 alkyl), -(CH2O)nC(O)-(C1-C6 alkyl), -(OCH2)nNHC(O)-R1, -(CH2O)nC(O)-NR1R2, -S(O)2-RS, -S(O)-RS (RS is C1-C6 alkyl or a-(CH2)m-NR1R2 group), NO2, CN or halogen (F, Cl, Br, I, preferably F or Cl), depending on the context of the use of the substituent. R1 and R2 are each, within context, H or a C1-C6 alkyl group (which may be optionally substituted with one or two hydroxyl groups or up to three halogen groups, preferably fluorine). The term "substituted" shall also mean, within the chemical context of the compound defined and substituent used, an optionally substituted aryl or heteroaryl group or an optionally substituted heterocyclic group as otherwise described herein. Alkylene groups may also be substituted as otherwise disclosed herein, preferably with optionally substituted C1-C6 alkyl groups (methyl, ethyl or hydroxymethyl or hydroxyethyl is preferred, thus providing a chiral center), an amido group as described hereinabove, or a urethane group O-C(O)-NR1R2 group where R1 and R2 are as otherwise described herein, although numerous other groups may also be used as substituents. Various optionally substituted moieties may be substituted indepencetnly with 3 or more substituents, preferably no more than 3 substituents and preferably with 1 or 2 substituents. It is noted that in instances where, in a compound at a particular position of the molecule substitution is required (principally, because of valency), but no substitution is indicated, then that substituent is construed or understood to be H, unless the context of the substitution suggests otherwise.

"Hydroxyl" refers the functional group -OH when it is a substituent in an organic compound.

"Heterocycle" refers to a heterocyclic group having from 4 to 9 carbon atoms and at least one heteroatom selected from the group consisting of N, O or S, and may be aromatic (heteroaryl) or non-aromatic. Thus, the heteroaryl moieties are subsumed under the definition of heterocycle, depending on the context of its use. Exemplary heteroaryl groups are described hereinabove. Exemplary nonaromatic heterocyclic groups for use in the present disclosure include, for example, pyrrolidinyl, pyrrolinyl, piperidinyl, piperazinyl, N-methylpiperazinyl, imidazolinyl, pyrazolidinyl, imidazolidinyl, morpholinyl, tetrahydropyranyl, azetidinyl, oxetanyl, oxathiolanyl, pyridone, 2-pyrrolidone, ethyleneurea, 1,3-dioxolane, 1,3-dioxane, 1,4-dioxane, phthalimide and succinimide, among others.

Heterocyclic groups can be optionally substituted with 1 to 5, and preferably 1 to 3 substituents, selected from the group consisting of alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, keto, thioketo, carboxy, carboxyalkyl, thioaryloxy, thioheteroaryloxy, thioheterocyclooxy, thiol, thioalkoxy, substituted thioalkoxy, aryl, aryloxy, heteroaryl, heteroaryloxy, heterocyclic, heterocyclooxy, hydroxyamino, alkoxyamino, nitro, -SO-alkyl, -SO-substituted alkyl, -SO-aryl, -SO-heteroaryl, -SO2-alkyl, -SO2-substituted alkyl, -SO2-aryl, oxo (=O), and-SO2-heteroaryl. Such heterocyclic groups can have a single ring or multiple condensed rings. Preferred heterocyclics include morpholino, piperidinyl, and the like.

Examples of nitrogen heterocycles and heteroaryls include, but are not limited to, pyrrole, imidazole, pyrazole, pyridine, pyrazine, pyrimidine, pyridazine, indolizine, isoindole, indole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, morpholino, piperidinyl, tetrahydrofuranyl, and the like as well as N-alkoxy-nitrogen containing heterocycles.

"Heteroaryl" refers to a heterocyclic group having from 4 to 9 carbon atoms and at least one heteroatom selected from the group consisting of N, O or S with at least one ring of this group being aromatic. Heteroaryl groups having one or more nitrogen, oxygen, or sulfur atoms in the ring (moncyclic) such as imidazole, furyl, pyrrole, furanyl, thiene, thiazole, pyridine, pyrimidine, pyrazine, triazole, oxazole or fused ring systems such as indole, quinoline, indolizine, azaindolizine, benzofurazan, etc., among others, which may be optionally substituted as described above. Among the heteroaryl groups which may be mentioned include nitrogen containing heteroaryl groups such as pyrrole, pyridine, pyridone, pyridazine, pyrimidine, pyrazine, pyrazole, imidazole, triazole, triazine, tetrazole, indole, isoindole, indolizine, azaindolizine, purine, indazole, quinoline, dihydroquinoline, tetrahydroquinoline, isoquinoline, dihydroisoquinoline, tetrahydroisoquinoline, quinolizine, phthalazine, naphthyridine, quinoxaline, quinazoline, cinnoline, pteridine, imidazopyridine, imidazotriazine, pyrazinopyridazine, acridine, phenanthridine, carbazole, carbazoline, perimidine, phenanthroline, phenacene, oxadiazole, benzimidazole, pyrrolopyridine, pyrrolopyrimidine and pyridopyrimidine; sulfur-containing aromatic heterocycles such as thiophene and benzothiophene; oxygen-containing aromatic heterocycles such as furan, pyran, cyclopentapyran, benzofuran and isobenzofuran; and aromatic heterocycles comprising 2 or more hetero atoms selected from among nitrogen, sulfur and oxygen, such as thiazole, thiadizole, isothiazole, benzoxazole, benzothiazole, benzothiadiazole, phenothiazine, isoxazole, furazan, phenoxazine, pyrazoloxazole, imidazothiazole, thienofuran, furopyrrole, pyridoxazine, furopyridine, furopyrimidine, thienopyrimidine and oxazole, among others, all of which may be optionally substituted.

"Substituted heteroaryl" refers to a heterocyclic group having from 4 to 9 carbon atoms and at least one heteroatom selected from the group consisting of N, O or S with at least one ring of this group being aromatic and this group being substituted with one or more substituents selected from the group consisting of halogen, alkyl, carbyloxy, carbylmercapto, alkylamino, amido, carboxyl, hydroxyl, nitro, mercapto or sulfo, whereas these generic substituent group have meanings which are identical with definitions of the corresponding groups as defined in this legend.

The term "thiol" refers to the group -SH.

The term "thioalkoxy" refers to the group -S-alkyl.

"Amidine" refers to a functional group that has two amine groups attached to the same carbon atom with one carbon-nitrogen double bond: HN=CR'-NH"2.

"Alkoxyl" refers to an alkyl group linked to oxygen thus: R-O-, where R is an alkyl.

"Substituted alkyl" refers to a branched or unbranched alkyl, alkenyl or alkinyl group having 1-10 carbon atoms and having substituted by one or more substituents selected from the group consisting of hydroxyl, mercapto, carbylmercapto, halogen, carbyloxy, amino, amido, carboxyl, cycloalkyl, sulfo or acyl. These substituent generic groups having the meanings being identical with the definitions of the corresponding groups as defined herein.

"Halogen" refers to fluorine, bromine, chlorine, and iodine atoms.

"Acyl" denotes the group --C(O)Rₑ, where Rₑ is hydrogen, alkyl, substituted alkyl, aryl, substituted aryl, arylalkyl, substituted arylalkyl, cycloalkyl, substituted cycloalkyl whereas these generic groups have meanings which are identical with definitions of the corresponding groups as defined in this legend.

"Acloxy" denotes the group --OAc, where Ac is an acyl, substituted acyl, heteroacyl or substituted heteroacyl whereas these generic groups have meanings which are identical with definitions of the corresponding groups as defined in this legend.

"Alkylamino" denotes the group --NR_{f}R_{g}, where R_{f} and R_{g}, that are independent of one another, represent hydrogen, alkyl, substituted alkyl, aryl, substituted aryl, heteroaryl or substituted heteroaryl, whereas these generic substituents have meanings which are identical with definitions of the corresponding groups defined herein.

"Aryl" refers to an aromatic carbocyclic group having from 1 to 18 carbon atoms and being a substituted (as otherwise described herein) or unsubstituted monovalent aromatic radical having a single ring (e.g., benzene, phenyl, benzyl) or condensed (fused) rings, wherein at least one ring is aromatic (e.g., naphthyl, anthracenyl, phenanthrenyl, etc.) and can be bound to the compound according to the present disclosure at any available stable position on the ring(s) or as otherwise indicated in the chemical structure presented. Other examples of aryl groups, in context, may include heterocyclic aromatic ring systems.

"Substituted aryl" refers to an aromatic carbocyclic group having from 1 to 18 carbon atoms and being composed of at least one aromatic ring or of multiple condensed rings at least one of which being aromatic. The ring(s) are optionally substituted with one or more substituents selected from the group consisting of halogen, alkyl, hydroxyl, carbylmercapto, alkylamino, carbyloxy, amino, amido, carboxyl, nitro, mercapto or sulfo, whereas these generic substituent group have meanings which are identical with definitions of the corresponding groups as defined in this legend.

"Carboxyl" denotes the group --C(O)OR, where R is hydrogen, alkyl, substituted alkyl, aryl, substituted aryl, heteroaryl or substituted heteroaryl , whereas these generic substituents have meanings which are identical with definitions of the corresponding groups defined herein.

"Cycloalkyl" refers to a monocyclic or polycyclic alkyl group containing 3 to 15 carbon atoms.

"Substituted cycloalkyl" refers to a monocyclic or polycyclic alkyl group containing 3 to 15 carbon atoms and being substituted by one or more substituents selected from the group consisting of halogen, alkyl, substituted alkyl, carbyloxy, carbylmercapto, aryl, nitro, mercapto or sulfo, whereas these generic substituent groups have meanings which are identical with definitions of the corresponding groups as defined in this legend.

"Heterocycloalkyl" refers to a monocyclic or polycyclic alkyl group containing 3 to 15 carbon atoms which at least one ring carbon atom of its cyclic structure being replaced with a heteroatom selected from the group consisting of N, O, S or P.

"Substituted heterocycloalkyl" refers to a monocyclic or polycyclic alkyl group containing 3 to 15 carbon atoms which at least one ring carbon atom of its cyclic structure being replaced with a heteroatom selected from the group consisting of N, O, S or P and the group is containing one or more substituents selected from the group consisting of halogen, alkyl, substituted alkyl, carbyloxy, carbylmercapto, aryl, nitro, mercapto or sulfo, whereas these generic substituent group have meanings which are identical with definitions of the corresponding groups as defined in this legend.

The term "alkenyl" refers to a monoradical of a branched or unbranched unsaturated hydrocarbon group preferably having from 2 to 40 carbon atoms, more preferably 2 to 10 carbon atoms and even more preferably 2 to 6 carbon atoms. Preferred alkenyl groups include ethenyl (-CH=CH2), n-propenyl (-CH2CH=CH2), iso-propenyl (-C(CH3)=CH2), and the like.

"Imidazole" refers to a heterocyclic base of the general formula: C₃H₄N₂.

"Aralkyl group" refers to, for example, a C1 -C6 alkyl group which is attached to 1 or 2 aromatic hydrocarbon rings having from 6 to 10 carbon atoms and which has a total of 7 to 14 carbon atoms, such as the benzyl, alpha-naphthylmethyl, indenylmethyl, diphenylmethyl, 2-phenethyl, 2-alpha-naphthylethyl, 3-phenylpropyl, 3-alpha-naphthylpropyl, phenylbutyl, 4-alpha-naphthylbutyl or 5-phenylpentyl groups.

"Guanidine" refers generally to the amidine of amidocarbonic acid and has the general formula of: C(NH₂)₃.

The terms "aralkyl" and "heteroarylalkyl" refer to groups that comprise both aryl or, respectively, heteroaryl as well as alkyl and/or heteroalkyl and/or carbocyclic and/or heterocycloalkyl ring systems according to the above definitions.

In one aspect, the description provides for oligourea compounds comprising aliphatic oligoureas. In certain embodiments, the aliphatic oligoureas form a oligourea helical bundle. In another embodiment, the aliphatic oligoureas are comprised of short amphiphilic α-helicomimetic foldamers with proteinaceous sidechains. In an embodiment, the short amphiphilic α-helicomimetic foldamers self-assemble into the oligourea helical bundle. Furthermore, the foldamers can self-assemble under aqueous conditions. Because the compounds as described herein can adopt desired secondary structures similar to native peptides, including, e.g., helicoidal structures, they can serve as, for example, receptor ligands, effector molecules, agonists, antagonists, modulators of protein-protein interactions, organocatalysts, or enzymes.

### Oligourea Compounds

In certain aspects, the present description provides compounds comprising non-peptide oligourea helical foldamers. In particular, the non-peptide oligourea helical foldamers self-assemble under acqueous conditions to form oligourea helical bundles or super-helical nanotubes. In an embodiment, the oligourea helical bundle has an isolated cavity within the hydrophobic core of the bundle. In another embodiment, the oligourea nanotube has a pH-responsive water-filled channel or pore within the hydrophobic core of the bundle. Furthermore, the diameter of the channel or pore can be controlled through the modification of the non-peptide oligourea peptidomimetic residue sequence, which is described in greater detail with regard to the figures. In an alternative embodiment, the oligourea helical bundle has a pH-responsive super-helical nanotube with water-filled channel or pore within the hydrophobic core.

In certain embodiments, an interior channel or pore of the oligourea super-helical nanotube has an internal diameter of in a range of about 10 Å to about 30 Å. Alternatively, an interior pore internal diameter is in a range of about 12.5 Å to about 30 Å, about 12.5 Å to about 27.5 Å, about 12.5 Å to about 25 Å, about 12.5 Å to about 22.5 Å. About 12.5 Å to about 20 Å, about 12.5 Å to about 17.5 Å, about 12.5 Å to about 15 Å, about 15 Å to about 30 Å, about 15 Å to about 27.5 Å, about 15 Å to about 25 Å, about 15 Å to about 22.5 Å, about 15 Å to about 20 Å, about 15 Å to about 17.5 Å, about 17.5 Å to about 30 Å, about 17.5 Å to about 27.5 Å, about 17.5 Å to about 25 Å, about 20 Å to about 22.5 Å, about 20 Å to about 30 Å, about 20 Å to about 27.5 Å, to about 20 Å to about 25 Å, about 20 Å to about 22.5 Å, about 22.5 Å to about 30 Å, about 22.5 Å to about 27.5 Å, or about 22.5 Å to about 25 Å. In particular embodiments, the internal diameter of the internal pore of the oligourea helical bundle is 10 Å, 11 Å, 12 Å, 13 Å, 14 Å, 15 Å, 16 Å, 17 Å, 18 Å, 19 Å, 20 Å, 21 Å, 22 Å, 23 Å, 24 Å, 25 Å, 26 Å, 27 Å, 28 Å, 29 Å, or 30 Å.

In particular embodiments, the oligourea super-helical nanotube has a diameter in a range of about 2 nm to about 12 nm. Alternatively, the diameter can be in a range of about 2 nm to about 11 nm, about 2 nm to about 10 nm, about 2 nm to about 9 nm, about 2 nm to about 8 nm, about 2 nm to about 7 nm, about 2 nm to about 6 nm, about 2 nm to about 5 nm, about 3 nm to about 12 nm, about 3 nm to about 11 nm, about 3 nm to about 10 nm, about 3 nm to about 9 nm, about 3 nm to about 8 nm, about 3 nm to about 7 nm, about 3 nm to about 4 nm, about 4 nm to about 12 nm, about 4 nm to about 11 nm, about 4 nm to about 10 nm, about 4 nm to about 9 nm, about 4 nm to about 8 nm, about 4 nm to about 7nm, about 4 nm to about 6 nm, about 4 nm to about 5 nm, about 5 nm to about 12 nm, about 5 nm to about 11 nm, about 5 nm to about 10 nm, about 5 nm to about 9 nm, about 5 nm to about 8 nm, about 5 nm to about 7 nm, about 5 nm to about 6 nm, about 6 nm to about 12 nm, about 6 nm to about 11 nm, about 6 nm to about 10 nm, about 6 nm to about 9 nm, about 6 nm to about 8 nm, about 6 nm to about 7 nm, about 7 nm to about 12 nm, about 7 nm to about 11 nm, about 7 nm to about 10 nm, about 7 nm to about 9 nm, about 7 nm to about 8 nm, about 8 nm to about 12 nm, about 8 nm to about 11 nm, about 8 nm to about 10 nm, about 8 nm to about 9 nm, about 9 nm to about 12 nm, about 9 nm to about 11 nm, about 9 nm to about 10 nm, about 10 nm to about 12 nm, about 10 nm to about 11 nm, or about 11 nm to about 12 nm. In certain embodiments, the diameter of the oligourea helical bundle is about 2 nm, about 3 nm, about 4 nm, about 5 nm, about 6 nm, about 7 nm, about 8 nm, about 9 nm, about 10 nm, about 11 nm, or about 12 nm.

In some embodiments, the non-peptide oliguria peptiomimetic residue sequence is selected from the group consisting of: Leu^{U} Glu^{U} Lys^{U} Leu^{U} _{T}yr^{U} Leu^{U} Glu^{U} Lys^{U} Leu^{U} Ala^{U} Leu^{U} (H1) (SEQ ID NO: 1); Leu^{U} Glu^{U} Leu^{U} Lys^{U} Pro^{U} Leu^{U} Glu^{U} Leu^{U} Lys^{U} Ala^{U} (H2) (SEQ ID NO: 2); Leu^{U} Glu^{U} Lys^{U} Leu^{U} Tyr^{U} Asn^{U} Glu^{U} Lys^{U} Leu^{U} Ala^{U} Leu^{U} (H3) (SEQ ID NO: 3); Ser^{U} Glu^{U} Lys^{U} Leu^{U} Tyr^{U} Leu^{U} Glu^{U} Lys^{U} Leu^{U} Ala^{U} Leu^{U} (H4) (SEQ ID NO: 4); Ala^{U} Leu^{U} Lys^{U} Leu^{U} Glu^{U} Tyr^{U} Leu^{U} Glu^{U} Leu^{U} Lys^{U} Ala^{U} Leu^{U} (H5) (SEQ ID NO: 5); Leu^{u} Glu^{u} Lys^{u} Leu^{u} Tyr^{u} Leu^{u} Asn^{u} Lys^{u} Leu^{u} Ala^{u} Leu^{u} (H6) (SEQ ID NO: 6); and Leu^{u} Glu^{u} Lys^{u} Leu^{u} Tyr^{u} Leu^{u} Gln^{u} Lys^{u} Leu^{u} Ala^{u} Leu^{u} (H7) (SEQ ID NO: 7).

In certain embodiments, the non-peptide oligourea helical foldamer is an aliphatic oligourea. In a particular embodiment, the non-peptide oligourea helical foldamer is a short amphiphilic α-helicomimetic foldamer with proteinaceous side-chains.

In a particular embodiment, the oligourea foldamer has a non-peptide oligourea peptidomimetic residue sequence selected from the group consisting of: Leu^{u} Glu^{u} Lys^{u} Leu^{u} Tyr^{u} Leu^{u} Glu^{u} Lys^{u} Leu^{u} Ala^{u} Leu^{u} (H1); Leu^{u} Glu^{u} Leu^{u} Lys^{u} Pro^{u} Leu^{u} Glu^{u} Leu^{u} Lys^{u} Ala^{u} (H2); Leu^{u} Glu^{u} Lys^{u} Leu^{u} Tyr^{u} Asn^{u} Glu^{u} Lys^{u} Leu^{u} Ala^{u} Leu^{u} (H3); Ser^{u} Glu^{u} Lys^{u} Leu^{u} Tyr^{u} Leu^{u} Glu^{u} Lys^{u} Leu^{u} Ala^{u} Leu^{u} (H4); and Ala^{u} Leu^{u} Lys^{u} Leu^{u} Glu^{u} Tyr^{u} Leu^{u} Glu^{u} Leu^{u} Lys^{u} Ala^{u} Leu^{u} (H5). Alternatively, the oligourea foldamer has a non-peptide peptidomimetic residue sequence that follows one of the following two helical pentad repeat sequences. Helical pentad repeat sequence 1: oligourea peptidomimetic residues with a hydrophobic side chain in positions *a* and *d* (e.g., Leu^{u}); oligourea peptidomimetic residues with a charged residue in position *band c* (e.g. Glu^{u} and Lys^{u}, respectively); and Tyr^{u} and Ala^{u} in position *e.* Helical pentad repeat sequence 2: oligourea peptidomimetic residues with a hydrophobic side chain in positions a and c (e.g. Leu^{u}); oligourea peptidomimetic residues with a hydrophobic side chain in position e (e.g., Ala^{u} and Pro^{u} residues at the *e* position); and oligourea peptidomimetic residues with a charged side chain in positions *b* and *d* (e.g., Glu^{u} and Lys^{u}, respectively).

In additional embodiments, the oligourea compound includes at least one of: a plurality of oligourea helical bundles (e.g., two, three, four, etc.); a plurality of non-peptide oligourea helical foldamers (e.g., two, three, four, etc.); and a plurality of peptides (e.g., two, three, four, etc.). In certain embodiments, the oligourea compound includes two oligourea helical bundles. In another embodiment, the oligourea helical bundle comprises one or more non-peptide oligourea helical foldamer (e.g., one foldamer, two foldamers, three foldamers, 4 foldamers, etc.).

In a particular embodiment, the non-peptide oligourea peptidomimetic residue sequence is selected from the group consisting of: Leu^{u} Glu^{u} Lys^{u} Leu^{u} Tyr^{u} Leu^{u} Glu^{u} Lys^{u} Leu^{u} Ala^{u} Leu^{u} (H1); Leu^{u} Glu^{u} Leu^{u} Lys^{u} Pro^{u} Leu^{u} Glu^{u} Leu^{u} Lys^{u} Ala^{u} (H2); Leu^{u} Glu^{u} Lys^{u} Leu^{u} Tyr^{u} Asn^{u} Glu^{u} Lys^{u} Leu^{u} Ala^{u} Leu^{u} (H3); Ser^{u} Glu^{u} Lys^{u} Leu^{u} Tyr^{u} Leu^{u} Glu^{u} Lys^{u} Leu^{u} Ala^{u} Leu^{u} (H4); and Ala^{u} Leu^{u} Lys^{u} Leu^{u} Glu^{u} Tyr^{u} Leu^{u} Glu^{u} Leu^{u} Lys^{u} Ala^{u} Leu^{u} (H5). Alternatively, the non-peptide oligourea helical foldamer has a non-peptide peptidomimetic residue sequence that follows one of the following two helical pentad repeat sequences. Helical pentad repeat sequence 1: oligourea peptidomimetic residues with a hydrophobic side chain in positions *a* and *d* (e.g., Leu^{u}); oligourea peptidomimetic residues with a charged residue in position *b* and *c* (e.g. Glu^{u} and Lys^{u}, respectively); and Tyr^{u} and Ala^{u} in position *e.* Helical pentad repeat sequence 2: oligourea peptidomimetic residues with a hydrophobic side chain in positions a and c (e.g. Leu^{u}); oligourea peptidomimetic residues with a hydrophobic side chain in position e (e.g., Ala^{u} and Pro^{u} residues at the *e* position); and oligourea peptidomimetic residues with a charged side chain in positions *b and d* (e.g., Glu^{u} and Lys^{u}, respectively).

In a certain embodiments, the oligourea helical bundle has an internal cavity with a volume in a range of about 300 to about 1000 Å³. In another embodiment, the volume of the internal cavity is in a range of 300 to about 650 Å³, about 300 to about 600 Å³, about 300 to about 575 Å³, about 300 to about 550 Å³, about 300 to about 500 Å³, about 300 to about 475 Å³, about 300 to about 450 Å³, about 300 to about 425 Å³, about 350 to about 700 Å³, about 350 to about 650 Å³, about 350 to about 600 Å³, about 350 to about 575 Å³, about 350 to about 550 Å³, about 350 to about 525 Å³, about 350 to about 500 Å³, about 350 to about 475 Å³, about 350 to about 450 Å³, about 350 to about 425 Å³, about 350 to about 400 Å³, about 375 to about 700 Å³, about 375 to about 650 Å³, about 375 to about 600 Å³, about 375 to about 575 Å³, about 375 to about 550 Å³, about 375 to about 525 Å³, about 375 to about 500 Å³, about 375 to about 400 Å³, about 400 to about 700 Å³, about 400 to about 650 Å³, about 400 to about 600 Å³, about 400 to about 575 Å³, about 400 to about 550 Å³, about 400 to about 525 Å³, about 400 to about 500 Å³, about 400 to about 475 Å³, about 425 to about 700 Å³, about 425 to about 650 Å³, about 425 to about 600 Å³, about 425 to about 575 Å³, about 425 to about 550 Å³, about 425 to about 525 Å³, about 500 to about 700 Å³, about 425 to about 475 Å³, about 450 to about 700 Å³, about 450 to about 650 Å³, about 450 to about 600 Å³, about 450 to about 575 Å³, about 450 to about 550 Å³, about 450 to about 525 Å³, about 450 to about 500 Å³, about 475 to about 700 Å³, about 475 to about 650 Å³, about 475 to about 600 Å³, about 475 to about 550 Å³, about 475 to about 525 Å³, about 500 to about 700 Å³, about 500 to about 650 Å³, about 500 to about 625 Å³, about 500 to about 600 Å³, about 500 to about 575 Å³, or about 500 to about 575 Å³. In particular embodiments, the volume of the internal cavity is 300 Å³, 325 Å³, 350 Å³, 375 Å³, 400 Å³, 425 Å³, 450 Å³, 475 Å³, 500 Å³, 525 Å³, 550 Å³, 575 Å³, 600 Å³, 625 Å³, 650 Å³, 675 Å³, 600 Å³, 625 Å³, 650 Å³, 675 Å³, or 700 Å³.

In certain embodiments, the oligourea compound has a secondary structure similar to a native peptide. As such, the secondary structure can act in a fashion similar to that of the native peptide. For example, the secondary structure of the oligourea compound can be biologically active. By way of another example, the secondary structure can act as a receptor ligand, an effector molecule, an agonist, an antagonist, a modulator of protein-protein interactions, an organocatalyst, or an enzyme.

Alteratively, the oligourea compound may have a secondary structure that provides a function not found in nature. For example, the oligourea compound may be a catalyst with tailored substrate specificity.

The present description describes the surprising and unexpected discovery that non-peptide oligourea foldamers comprising non-peptide oligourea peptidomimetic residues demonstrate the ability to self-assemble oligourea helical bundles and super-helical nanotubes under acqueous conditions.

Furthermore, in another aspect, peptide-oligourea chimeric compounds are described herein. The chimeric compound comprising an oligourea helical bundle and a peptide. In a particular embodiment, the peptide is fused to, contiguous with, or conjugated to the oligourea helical bundle. In a particular embodiment, the peptide is a peptide α-helix. The chimeric compounds as described herein improve at least one PK ane/or PD characteristic of the natural peptide. Because the chimeric compounds as described herein can adopt desired secondary structures similar to native peptides, including, e.g., helicoidal structures, they can serve as, for example, receptor ligands, effector molecules, agonists, antagonists, modulators of protein-protein interactions, organocatalysts or enzymes. Therefore, in certain aspects, the present description provides chimeric compounds, methods of making, and using the same. These aspectis will be discussed next.

### Peptide-Oligourea Chimeric Compounds

In certain aspects, the present description provides compounds comprising peptide/oligourea chimeras (herein, "chimeras" or "chimeric compounds"). In particular, the description provides chimeric compounds comprising a portion or sequence of amino acids (i.e., a peptide) covalently coupled to an oligourea helix bundle. In an embodiment, the oligourea helical bundle self-assembles into its three-dimensional nanostructure under aqueous conditions. In a particular embodiment, the peptide is a peptide α-helix. In a certain embodiment, the peptide is a α-peptide (i.e., make of alpha amino acids). In certain embodiments, the non-peptide oligourea helical foldamer is an aliphatic oligourea. In a particular embodiment, the non-peptide oligourea helical foldamer is a short amphiphilic α-helicomimetic foldamer with proteinaceous side-chains.

In an embodiment, the oligourea helical bundle has an isolated cavity within the hydrophobic core of the bundle. In another embodiment, the oligourea foldamer forms a super-helical nanotube comprising a pH-responsive water-filled channel within the hydrophobic core of the helix. Furthermore, the diameter of the pores can be controlled through the modification of the non-peptide oligourea peptidomimetic residue sequence, which is described in greater detail below. In an alternative embodiment, the oligourea super-helical nanotube has a pH-responsive super-helical channel with water-filled pores within the hydrophobic core of the bundle.

In a particular embodiment, the oligourea helical foldamer has a non-peptide oligourea peptidomimetic residue sequence selected from the group consisting of: Leu^{u} Glu^{u} Lys^{u} Leu^{u} Tyr^{u} Leu^{u} Glu^{u} Lys^{u} Leu^{u} Ala^{u} Leu^{u} (H1); Leu^{u} Glu^{u} Leu^{u} Lys^{u} Pro^{u} Leu^{u} Glu^{u} Leu^{u} Lys^{u} Ala^{u} (H2); Leu^{u} Glu^{u} Lys^{u} Leu^{u} Tyr^{u} Asn^{u} Glu^{u} Lys^{u} Leu^{u} Ala^{u} Leu^{u} (H3); Ser^{u} Glu^{u} Lys^{u} Leu^{u} Tyr^{u} Leu^{u} Glu^{u} Lys^{u} Leu^{u} Ala^{u} Leu^{u} (H4); and Ala^{u} Leu^{u} Lys^{u} Leu^{u} Glu^{u} Tyr^{u} Leu^{u} Glu^{u} Leu^{u} Lys^{u} Ala^{u} Leu^{u} (H5). Alternatively, the oligourea helical foldamer has a non-peptide peptidomimetic residue sequence that follows one of the following two helical pentad repeat sequences. Helical pentad repeat sequence 1: oligourea peptidomimetic residues with a hydrophobic side chain in positions *a* and *d* (e.g., Leu^{u}); oligourea peptidomimetic residues with a charged residue in position *b* and *c* (e.g. Glu^{u} and Lys^{u}, respectively); and Tyr^{u} and Ala^{u} in position *e.* Helical pentad repeat sequence 2: oligourea peptidomimetic residues with a hydrophobic side chain in positions a and c (e.g. Leu^{u}); oligourea peptidomimetic residues with a hydrophobic side chain in position e (e.g., Ala^{u} and Pro^{u} residues at the *e* position); and oligourea peptidomimetic residues with a charged side chain in positions *b* and *d* (e.g., Glu^{u} and Lys^{u}, respectively).

In a particular embodiment, the non-peptide oligourea peptidomimetic residue sequence is selected from the group consisting of: Leu^{u} Glu^{u} Lys^{u} Leu^{u} Tyr^{u} Leu^{u} Glu^{u} Lys^{u} Leu^{u} Ala^{u} Leu^{u} (H1); Leu^{u} Glu^{u} Leu^{u} Lys^{u} Pro^{u} Leu^{u} Glu^{u} Leu^{u} Lys^{u} Ala^{u} (H2); Leu^{u} Glu^{u} Lys^{u} Leu^{u} Tyr^{u} Asn^{u} Glu^{u} Lys^{u} Leu^{u} Ala^{u} Leu^{u} (H3); Ser^{u} Glu^{u} Lys^{u} Leu^{u} Tyr^{u} Leu^{u} Glu^{u} Lys^{u} Leu^{u} Ala^{u} Leu^{u} (H4); and Ala^{u} Leu^{u} Lys^{u} Leu^{u} Glu^{u} Tyr^{u} Leu^{u} Glu^{u} Leu^{u} Lys^{u} Ala^{u} Leu^{u} (H5). Alternatively, the non-peptide oligourea helical foldamer has a non-peptide peptidomimetic residue sequence that follows one of the following two helical pentad repeat sequences. Helical pentad repeat sequence 1: oligourea peptidomimetic residues with a hydrophobic side chain in positions *a* and *d* (e.g., Leu^{u}); oligourea peptidomimetic residues with a charged residue in position *b* and *c* (e.g. Glu^{u} and Lys^{u}, respectively); and Tyr^{u} and Ala^{u} in position *e.* Helical pentad repeat sequence 2: oligourea peptidomimetic residues with a hydrophobic side chain in positions a and c (e.g. Leu^{u}); oligourea peptidomimetic residues with a hydrophobic side chain in position e (e.g., Ala^{u} and Pro^{u} residues at the *e* position); and oligourea peptidomimetic residues with a charged side chain in positions *b* and *d* (e.g., Glu^{u} and Lys^{u}, respectively).

In a certain embodiment, the oligourea helical bundle has an internal cavity with a volume in a range of about 300 to about 700 Å³. In another embodiment, the volume of the internal cavity is in a range of 400 to about 600 Å³. In a particular embodiment, the volume of the internal cavity is about 500 Å³.

In certain embodiments, the oligourea compound has a secondary structure similar to a native peptide. As such, the secondary structure can act in a fashion similar to that of the native peptide. For example, the secondary structure of the oligourea compound can be biologically active. By way of further example, the secondary structure can act as a receptor ligand, an effector molecule, an agonist, an antagonist, a modulator of protein-protein interactions, an organocatalyst, or an enzyme. Alteratively, the oligourea compound may have a secondary structure that provides a function not found in nature. For example, the oligourea compound may be a catalyst with tailored substrate specificity not known in nature.

In certain embodiments, the peptide is capable of binding specifically to a target, e.g., a protein such as a receptor or other polypeptide or peptide, or small molecule. In particular, the peptide may comprise an epitope that recognizes another compound, e.g. they can act as receptor ligands, effector molecules, agonists, antagonists, modulators of protein-protein interactions, organocatalysts, or enzymes.

In certain embodiments, the chimeric compounds comprise a peptide portion (i.e., a sequence of α-amino acid residues) contiguous with or coupled to an oligourea portion (i.e., a sequence of oligourea residues). In certain embodiments, the peptide portion comprises at least 2 α-amino acids. In certain additional embodiments, the oligourea portion comprises a non-peptide oligourea helical foldamer, for example, non-peptide oligourea peptidomimetic residues. The non-peptide oligourea helical foldamer can be an aliphatic oligourea. The non-peptide oligourea helical foldamer can be a short amphiphilic α-helicomimetic foldamer, which may include proteinaceous side chains.

In further embodiments, the oligourea compounds at least one non-peptide oligourea peptidomimetic residue of the formula I and oligomers thereof: 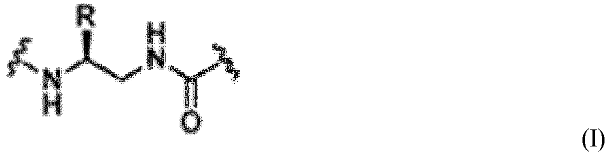 wherein R is independently selected from the group consisting of a hydrogen atom, an amino acid side chain, a (C1-C10) alkyl, (C1-C10) alkenyl, (C1-C10) alkynyl, (C5-C12) monocyclic or bicyclic aryl, (C5-C14) monocyclic or bicyclic aralkyl, (C5-C14) monocyclic or bicyclic heteroalkyl and (C1-C10) monocyclic or bicyclic heteroaryl group comprising up to 5 heteroatoms selected from N, O, and S, said groups being able to be non-substituted or substituted by 1 to 6 substituents further selected from the group consisting of: a halogen atom, an NO₂, OH, amidine, benzamidine, imidazole, alkoxy, (C1-C4) alkyl, NH2, CN, trihalomethyl, (C1-C4) acyloxy, (C1-C4) monoalkylamino, (C1-C4) dialkylamino, guanidino group, bis alkylated and bis acylated guanido group.

In any of the chimeric compound embodiments described herein, the peptide portion may comprise an α-amino acid sequence corresponding to a biologically active peptide or a fragment thereof.

In still additional embodiments, the chimeric compound as described herein is biologically active. The biological activity can stem from the peptide or the oligourea helical bundle. For example, in certain embodiments, the chimeric compounds as described herein are enzymatically active. In still additional embodiments, the chimeric compounds as described herein are configured to bind target proteins. In certain embodiments the target protein is a cytosolic protein. In certain embodiments, the target protein is a membrane protein. In certain embodiments, the membrane protein is a receptor. In still additional embodiments, the receptor is a growth factor receptor or a G-Protein Coupled Receptor (GPCR) or a fragment thereof. In an embodiment, the peptide is biologically active. Alternatively, in another embodiment, the oligourea helical bundle or the non-peptide oligourea helical foldamer is biologically active.

In certain aspects, the description provides chimeric compounds comprising a peptide portion coupled to an oligourea portion comprising a non-peptide oligourea helical foldamers which forms a helical bundle. Surprisingly and unexpectedly non-peptide oligourea helical foldamers as described herein adopt well-defined helical secondary structures akin to that of α-polypeptides, and can enhance or improve the beneficial properties of the cognate or parental "natural" peptide.

In certain aspects, the description provides peptide-oligourea chimeras that adopt stable secondary structures, including, e.g., linear, cyclic, or helicoidal, tertiary structure, and/or quaternary structures, wherein the chimeras comprise a sequence of amino acids (i.e., a polypeptide) contiguous with or coupled to an oligourea sequence of peptidomimetic or amino acid analog residues. In certain embodiments, the amino acid sequence comprises α-amino acids. In an additional embodiments, the chimeric compound comprises an amino acid sequence contiguous with or coupled to one or more oligourea peptidomimetic residues, wherein the peptidomimetic residue includes at least one non-peptide oligourea helical foldamer. In certain embodiments, the chimera comprises two or more non-peptide oligourea helical foldamers.

In certain aspects, the description provides a chimeric ligand compound capable of binding specifically to a target, e.g., a protein such as a receptor or other polypeptide or peptide, or small molecule, similar to the native or natural peptide. In certain embodiments, the chimeric ligand compound comprises a peptide portion coupled to an oligourea portion. In certain embodiments, the peptide portion comprises α-amino acids. In an additional embodiments, the chimeric ligand compound comprises an amino acid sequence contiguous with or coupled to an oligourea portion including one or more oligourea peptidomimetic residues. In certain embodiments the chimeric ligand compound comprises two or more oligourea peptidomimetic residues. In a certain embodiment, the chimeric ligand compound comprises a non-peptide oligourea helical foldamer.

In any of the embodiments described herein, the peptide-oligourea chimeric compound comprises a polypeptide portion including at least one α-, γ-, δ- amino acid, derivative or combination thereof, which is contiguous with or coupled to one or more oligourea peptidomimetic residues. In a preferred embodiment, the peptide-oligourea chimeric compound comprises a non-peptide oligourea helical foldamer. In a particular embodiment, the peptide-oligourea chimeric compound comprises at least one non-peptide oligourea helical foldamer (e.g., one, two, three, four, five, etc.) and/or at least one peptide (e.g., one, two, three, four, five, etc.)

In any of the embodiments described herein, the peptide-oligourea chimeric compound comprises an oligourea portion contiguous with or covalently linked or joined to at least one of the amino terminus (N'), the carboxyl terminus (C'), within the peptide sequence or a combination thereof. In a preferred embodiment, the peptide-oligourea chimeric compound comprises an oligourea portion covalently linked or joined to the C-terminus of the peptide portion.

In any of the embodiments described herein, the peptide-oligourea chimeric compound or the oligourea compound comprises 1, 2, 3, 4, 5, 6, 7, 8, 9, 10, 11, 12, 13, 14, 15, 16 ,17, 18 ,19, 20, 21, 22, 23, 24, 25, 26, 27, 28, 29, 30, 31, 32, 33, 34, 35, 36, 37, 38 ,39, 40, 41, 42, 43, 44, 45, 46, 47, 48, 49, 50, 51, 52, 53, 54, 55, 56, 57, 58, 59, 60, 61, 62, 63 ,64, 65, 66, 67, 68, 69, 70, 71, 72, 73, 74, 75, 76, 77, 78 ,79, 80, 81, 82, 83, 84, 85, 86, 87, 88, 89, 90, 91, 92, 93, 94, 95, 96, 97, 98, 99, 100, or more urea residues.

In another aspect, the description provides oligourea compounds and peptide-oligourea chimeric compounds as described herein further comprising at least one additional chemical modification. In certain embodiments, the chemical modification includes at least one of, for example, acetylation, phosphorylation, methylation, glycosylation, prenylation, isoprenylation, farnesylation, geranylation, pegylation, a disulfide bond, or combination thereof.

In any of the embodiments described herein, the peptide-oligourea chimeric compound comprises a polypeptide portion including at least one α-, γ-, δ- amino acid, derivative or combination thereof, which is contiguous with or coupled to one or more non-peptide oligouera peptidomimetic residues. In a preferred embodiment, the peptide-oligourea chimeric compound comprises a non-peptide oligourea helical foldamer.

In any of the embodiments described herein, the peptide-oligourea chimeric compound comprises an oligourea portion contiguous with or covalently linked or joined to at least one of the amino terminus (N'), the carboxyl terminus (C'), within the peptide sequence or a combination thereof. In a preferred embodiment, the peptide-oligourea chimeric compound comprises an oligourea portion covalently linked or joined to the C-terminus of the peptide portion.

### Pharmaceutical Forms

The compounds as described herein including pharmaceutically acceptable salts thereof are useful for the preparation of a medicament and/or the treatment of disease in a subject. In the case where a salt of a compound is desired and the compound is produced in the form of the desired salt, it can be subjected to purification as such. In the case where a compound is produced in the free state and its salt is desired, the compound is dissolved or suspended in a suitable organic solvent, followed by addition of an acid or a base to form a salt. As such, in an addition aspect the description provides compositions comprising an effective amount of a peptide-oligourea chimera as described herein, and a pharmaceutically acceptable carrier or excipient.

The compounds of the disclosure may optionally be administered with at least one of a pharmaceutically acceptable excipient, pharmacologically active agent or a combination thereof. These novel, unnatural peptidomimetics are resistant or wholly immune to peptidase and protease degradation and are conformationally restrained. Thus, they are useful as tools to model peptide and protein conformations in aqueous solutions. The compounds are also useful as non-enzymatically degradable probes to mimic protein behavior in solution. As such, the description further provides the compositions comprising an effective amount of a chimeric compound as described herein, and a pharmaceutically acceptable carrier or excipient.

Certain compounds of the disclosure and their salts may exist in more than one crystal form and the present disclosure includes each crystal form and mixtures thereof. Certain compounds of the disclosure and their salts may also exist in the form of solvates, for example hydrates, and the present disclosure includes each solvate and mixtures thereof.

Certain compounds of the disclosure may contain one or more chiral centers, and exist in different optically active forms. When compounds of the disclosure contain one chiral center, the compounds exist in two enantiomeric forms and the present disclosure includes both enantiomers and mixtures of enantiomers, such as racemic mixtures. The enantiomers may be resolved by methods known to those skilled in the art, for example by formation of diastereoisomeric salts which may be separated, for example, by crystallization; formation of diastereoisomeric derivatives or complexes which may be separated, for example, by crystallization, gas-liquid or liquid chromatography; selective reaction of one enantiomer with an enantiomer-specific reagent, for example enzymatic esterification; or gas-liquid or liquid chromatography in a chiral environment, for example on a chiral support for example silica with a bound chiral ligand or in the presence of a chiral solvent. It will be appreciated that where the desired enantiomer is converted into another chemical entity by one of the separation procedures described above, a further step may be used to liberate the desired enantiomeric form. Alternatively, specific enantiomers may be synthesized by asymmetric synthesis using optically active reagents, substrates, catalysts or solvents, or by converting one enantiomer into the other by asymmetric transformation.

When a compound of the disclosure contains more than one chiral center, it may exist in diastereoisomeric forms. The diastereoisomeric compounds may be separated by methods known to those skilled in the art, for example chromatography or crystallization and the individual enantiomers may be separated as described above. The present disclosure includes each diastereoisomer of compounds of the disclosure and mixtures thereof.

Certain compounds of the disclosure may exist in different tautomeric forms or as different geometric isomers, and the present disclosure includes each tautomer and/or geometric isomer of compounds of the disclosure and mixtures thereof.

Certain compounds of the disclosure may exist in different stable conformational forms which may be separable. Torsional asymmetry due to restricted rotation about an asymmetric single bond, for example because of steric hindrance or ring strain, may permit separation of different conformers. The present disclosure includes each conformational isomer of compounds of the disclosure and mixtures thereof.

Certain compounds of the disclosure may exist in zwitterionic form and the present disclosure includes each zwitterionic form of compounds of the disclosure and mixtures thereof.

The present disclosure encompasses all possible isomers including tautomers and mixtures thereof. Where chiral carbons lend themselves to two different enantiomers, both enantiomers are contemplated as well as procedures for separating the two enantiomers.

The present disclosure also relates to pharmaceutically acceptable salts, racemates, and optical isomers thereof. The compounds of this disclosure typically contain one or more chiral centers. Accordingly, this disclosure is intended to include racemic mixtures, diasteromers, enantiomers and mixture enriched in one or more steroisomer. The scope of the disclosure as described and claimed encompasses the racemic forms of the compounds as well as the individual enantiomers and non-racemic mixtures thereof.

Many of the compounds of the disclosure may be provided as salts with pharmaceutically compatible counterions (i.e., pharmaceutically acceptable salts).

The term "pharmaceutically acceptable salt" is used throughout the specification to describe, where applicable, a salt form of one or more of the compounds or prodrugs described herein which are presented to increase the solubility of the compound in the gastic juices of the patient's gastrointestinal tract in order to promote dissolution and the bioavailability of the compounds. Pharmaceutically acceptable salts include those derived from pharmaceutically acceptable inorganic or organic bases and acids, where applicable. Suitable salts include those derived from alkali metals such as potassium and sodium, alkaline earth metals such as calcium, magnesium and ammonium salts, among numerous other acids and bases well known in the pharmaceutical art. Sodium and potassium salts are particularly preferred as neutralization salts of the phosphates according to the present disclosure. In a preferred embodiment, the description provides pharmaceutically acceptable salts of the modified peptides as described herein, which retain the biological effectiveness and properties of the parent compounds and which are not biologically or otherwise harmful as the dosage administered. The compounds of this disclosure are capable of forming both acid and base salts by virtue of the presence of amino and carboxy groups respectively.

A "pharmaceutically acceptable counterion" is an ionic portion of a salt that is not toxic when released from the salt upon administration to a recipient. Pharmaceutically compatible salts may be formed with many acids, including but not limited to hydrochloric, sulfuric, acetic, lactic, tartaric, malic, succinic, etc. Salts tend to be more soluble in aqueous or other protonic solvents than are the corresponding free base forms.

Acids commonly employed to form pharmaceutically acceptable salts include inorganic acids such as hydrogen bisulfide, hydrochloric, hydrobromic, hydroiodic, sulfuric and phosphoric acid, as well as organic acids such as para-toluenesulfonic, salicylic, tartaric, bitartaric, ascorbic, maleic, besylic, fumaric, gluconic, glucuronic, formic, glutamic, methanesulfonic, ethanesulfonic, benzenesulfonic, lactic, oxalic, parabromophenylsulfonic, carbonic, succinic, citric, benzoic and acetic acid, and related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, sub erate , sebacate, fumarate, maleate, butyne-I,4-dioate, hexyne-I,6-dioate, benzoate, chlorobenzoate, methylbenzoate, dinitrobenzoate, hydroxybenzoate, methoxybenzoate, phthalate, terephthalate, sulfonate, xylenesulfonate, phenylacetate, phenylpropionate, phenylbutyrate, citrate, lactate, ∼-hydroxybutyrate, glycolate, maleate, tartrate, methanesulfonate, propanesulfonate, naphthalene-I-sulfonate, naphthalene-2-sulfonate, mandelate and the like salts.

Preferred pharmaceutically acceptable acid addition salts include those formed with mineral acids such as hydrochloric acid and hydrobromic acid, and especially those formed with organic acids such as maleic acid. Suitable bases for forming pharmaceutically acceptable salts with acidic functional groups include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines; dicyclohexylamine; tributyl amine; pyridine; N-methyl,N-ethylamine; diethylamine; triethylamine; mono-, bis-, or tris-(2-hydroxy-Iower alkyl amines), such as mono-, bis-, or tris-(2-hydroxyethyl)amine, 2-hydroxy-tert-butylamine, or tris-(hydroxymethyl)methylamine, N,N-di-Iower alkyl-N(hydroxy lower alkyl)-amines, such as N,N-dimethyl-N-(2-hydroxyethyl)amine, or tri-(2-hydroxyethyl)amine; N-methyl-D-glucamine; and amino acids such as arginine, lysine, and the like.

### Prodrugs

The descriptoin also provides prodrug forms of the above described oligourea compounds, wherein the prodrug is metabolized *in vivo* to produce an analog or derivative as set forth above. Indeed, some of the described compounds may be a prodrug for another analog or derivative. The term "prodrug" is well understood in the art and refers to an agent which is converted into the parent drug in vivo by some physiological chemical process (e.g., a prodrug on being brought to the physiological pH is converted to the desired drug form). For example, see Remington 's Pharmaceutical Sciences, 1980, vol. 16, Mack Publishing Company, Easton, Pa., 61 and 424.

Pro-drugs are often useful because, in some situations, they may be easier to administer than the parent drug. They may, for instance, be bioavailable by oral administration whereas the parent drug is not. The prodrug may also have improved solubility in pharmacological compositions over the parent drug. An example, without limitation, of a pro-drug would be a compound of the present disclosure wherein it is administered as an ester (the "pro-drug") to facilitate transmittal across a cell membrane where water solubility is not beneficial, but then it is metabolically hydrolyzed to the carboxylic acid once inside the cell where water solubility is beneficial. Pro-drugs have many useful properties. For example, a pro-drug may be more water soluble than the ultimate drug, thereby facilitating intravenous administration of the drug. A pro-drug may also have a higher level of oral bioavailability than the ultimate drug. After administration,the prodrug is enzymatically or chemically cleaved to deliver the ultimate drug in the blood or tissue.

Exemplary pro-drugs upon cleavage release the corresponding free acid, and such hydrolyzable ester-forming residues of the compounds of this disclosure include but are not limited to carboxylic acid substituents (e.g., -C(O)2H or a moiety that contains a carboxylic acid) wherein the free hydrogen is replaced by (Cl -C4)alkyl, (Cz-C12)alkanoyloxymethyl, (C4-C9)1-(alkanoyloxy)ethyl, I-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, I-methyl-1-10 (alkoxycarbonyloxy)ethyl having from 5 to 8 carbon atoms, N-(alkoxycarbonyl)aminomethyl having from 3 to 9 carbon atoms, 1-(N-(alkoxycarbonyl)amino ) ethyl having from 4 to 10 carbon atoms, 3-phthalidyl, 4-crotonolactonyl, gamma-butyrolacton-4-yl, di-N,N-(C1 -C2)alkylamino(C2-C3)alkyl (suchas ∼-dimethylaminoethyl), carbamoyl-(Cl-C2)alkyl, N ,N -die C1 -C2)-alkylcarbamoyl-(C1-15 C2)alkyl and piperidino-, pyrrolidino- or morpholino(C2-C3)alkyl.

Other exemplary pro-drugs release an alcohol or amine of a compound of the disclosure wherein the free hydrogen of a hydroxyl or amine substituent is replaced by (C1 - C6)alkanoyloxymethyl, 1-((C1-C6)alkanoyloxy)ethyl, I-methyl-1-((C1-C6)alkanoyloxy)ethyl, (Cl -C6)alkoxycarbonyl-oxymethyl, N-(Cl -C6)alkoxycarbonylamino- 20 methyl, succinoyl, (C1 - C6)alkanoyl, a-amino(C l-C4)alkanoyl, arylactyl and a-aminoacyl, or a-aminoacyl-a-aminoacyl wherein said a-aminoacyl moieties are independently any of the naturally occurring L-amino acids found in proteins, -P(O)(OH)2' -P(O)(O(C1 -C6)alkyl)2 or glycosyl (the radical resulting from detachment of the hydroxyl of the hemiacetal of a carbohydrate).

The phrase "protecting group" as used herein means temporary substituents which protect a potentially reactive functional group from undesired chemical transformations. Examples of such protecting groups include esters of carboxylic acids, silyl ethers of alcohols, and acetals and ketals of aldehydes and ketones, respectively. The field of protecting group chemistry has been reviewed (Greene, T.W.; Wuts, P.G.M. Protective 30 Groups in Organic Synthesis, 2nd ed.; Wiley: New York, 1991). Protected forms of the inventive compounds are included within the scope of this disclosure.

The term "chemically protected form," as used herein, pertains to a compound in which one or more reactive functional groups are protected from undesirable chemical reactions, that is, are in the form of a protected or protecting group (also known as a masked or masking group). It may be convenient or desirable to prepare, purify, and/or handle the active compound in a chemically protected form.

By protecting a reactive functional group, reactions involving other unprotected reactive functional groups can be performed, without affecting the protected group; the protecting group may be removed, usually in a subsequent step, without substantially affecting the remainder of the molecule. See, for example, Protective Groups in Organic Synthesis (T. Green and P. Wuts, Wiley, 1991), and Protective Groups in Organic Synthesis (T. Green and P. Wuts; 3rd Edition; John Wiley and Sons, 1999).

For example, a hydroxy group may be protected as an ether (-OR) or an ester (-OC(=O)R), for example, as: a t-butyl ether; a benzyl, benzhydryl (diphenylmethyl), or trityl (triphenylmethyl) ether; a trimethylsilyl or t-butyldimethylsilyl ether; or an acetyl ester (-OC(=O)CH3,-OAc). For example, an aldehyde or ketone group may be protected as an acetal or ketal, respectively, in which the carbonyl group (C(=O)) is converted to a diether (C(OR)2), by reaction with, for example, a primary alcohol. The aldehyde or ketone group is readily regenerated by hydrolysis using a large excess of water in the presence of acid. For example, an amine group may be protected, for example, as an amide (NRC(=O)R) or a urethane (-NRC(=O)OR), for example, as: a methyl amide (-NHC(=O)CH3); a benzyloxy amide (-NHC(=O)OCH2C6HsNHCbz); as a t-butoxy amide (NHC=(=O)OC(CH3)3,-NHBoc); a 2-biphenyl-2-propoxy amide (NHC(=O)OC(CH3)2C6H4C6HsNHBoc), as a 9-fluorenylmethoxy amide (-NHFmoc), as a 6-nitroveratryloxy amide (-NHNvoc), as a 2-trimethylsilylethyloxy amide (-NHTeoc), as a 2,2,2-trichloroethyloxy amide (-NHTroc), as an allyloxy amide (-NHAlloc) , as a 2-(phenylsulfonyl)ethyloxy amide (-NHPsec); or, in suitable cases (e.g., cyclic amines), as a nitroxide radical.

For example, a carboxylic acid group may be protected as an ester or an amide, for example, as: a benzyl ester; a t-butyl ester; a methyl ester; or a methyl amide. For example, a thiol group may be protected as a thioether (-SR), for example, as: a benzyl thioether; or an acetamidomethyl ether (-SCH2NHC(=O)CH3). In at least certain examples, the compounds disclosed herein can be used in the treatment of disorders associated with pathogen infection. Disorders associated with infection by pathogens include, but are not limited to, infection by viruses (DNA viruses, RNA viruses, animal viruses, and the like), bacteria (e.g., gram positive bacteria, gram negative bacteria, acid-fast bacteria, and the like), fungi, parasitic microbes, nematodes, and the like.

The term "pharmaceutically acceptable derivative" is used throughout the specification to describe any pharmaceutically acceptable prodrug form (such as an ester, amide other prodrug group) which, upon administration to a patient, provides directly or indirectly the present compound or an active metabolite of the present compound. The term "independently" is used herein to indicate that the variable, which is independently applied, varies independently from application to application.

The term "treatment" as used herein includes any treatment of a condition or disease in an animal, particularly a mammal, more particularly a human, and includes: (i) preventing the disease or condition from occurring in a subject which may be predisposed to the disease but has not yet been diagnosed as having it; (ii) inhibiting the disease or condition, i.e. arresting its development; relieving the disease or condition, i.e. causing regression of the condition; or (iii) ameliorating or relieving the conditions caused by the disease, i.e. symptoms of the disease.

The term "effective" is used to describe an amount of a compound, composition or component which, when used within the context of its intended use, effects an intended result.

The term "therapeutically effective amount" refers to that amount which is sufficient to effect treatment, as defined herein, when administered to a mammal in need of such treatment. The therapeutically effective amount will vary depending on the subject and disease state being treated, the severity of the affliction and the manner of administration, and may be determined routinely by one of ordinary skill in the art.

Suitable routes for administration include oral, peroral, rectal, vassal, topical (including ocular, buccal and sublingual), vaginal and parental (including subcutaneous, intramuscular, intravitreous, intravenous, intradermal, intrathecal and epidural). The preferred route of administration will depend upon the condition of the patient, the toxicity of the compound and the site of infection, among other considerations known to the clinician.

The therapeutic composition of the disclosure comprises about 1% to about 95% of the active ingredient, single-dose forms of administration preferably comprising about 20% to about 90% of the active ingredient and administration forms which are not single-dose preferably comprising about 5% to about 20% of the active ingredient. Unit dose forms are, for example, coated tablets, tablets, ampoules, vials, suppositories or capsules. Other forms of administration are, for example, ointments, creams, pastes, foams, tinctures, lipsticks, drops, sprays, dispersions and the like. Examples are capsules containing from about 0.05 g to about 1.0 g of the active ingredient.

The pharmaceutical compositions of the present disclosure are prepared in a manner known per se, for example by means of convential mixing, granulating, coating, dissolving or lyophilizing processes.

Preferably, solutions of the active ingredient, and in addition also suspensions or dispersions, especially isotonic aqueous solutions, dispersions or suspensions, are used, it being possible for these to be prepared before use, for example in the case of lyophilized compositions which comprise the active substance by itself or together with a carrier, for example mannitol. The pharmaceutical compositions can be sterilized and/or comprise excipients, for example preservatives, stabilizers, wetting agents and/or emulsifiers, solubilizing agents, salts for regulating the osmotic pressure and/or buffers, and they are prepared in a manner known per se, for example by means of convential dissolving or lyophilizing processes. The solutions or suspensions mentioned can comprise viscosity-increasing substances, such as sodium carboxymethylcellulose, carboxymethylcellulose, dextran, polyvinylpyrrolidone or gelatin.

Pharmaceutically acceptable forms include, for example, a gel, lotion, spray, powder, pill, tablet, controlled release tablet, sustained release tablet, rate controlling release tablet, enteric coating, emulsion, liquid, salts, pastes, jellies, aerosols, ointments, capsules, gel caps, or any other suitable form that will be obvious to one of ordinary skill in the art.

Suspensions in oil comprise, as the oily component, the vegetable, synthetic or semi-synthetic oils customary for injection purposes. Oils which may be mentioned are, in particular, liquid fatty acid esters which contain, as the acid component, a long-chain fatty acid having 8-22, in particular 12-22, carbon atoms, for example lauric acid, tridecylic acid, myristic acid, pentadecylic acid, palmitic acid, margaric acid, stearic acid, arachidinic acid, behenic acid or corresponding unsaturated acids, for example oleic acid, elaidic acid, euric acid, brasidic acid or linoleic acid, if appropriate with the addition of antioxidants, for example vitamin E, .beta.-carotene or 3,5-di-tert-butyl-4-hydroxytoluene. The alcohol component of these fatty acid esters has not more than 6 carbon atoms and is mono- or polyhydric, for example mono-, di- or trihydric alcohol, for example methanol, ethanol, propanol, butanol, or pentanol, or isomers thereof, but in particular glycol and glycerol. Fatty acid esters are therefore, for example: ethyl oleate, isopropyl myristate, isopropyl palmitate, "Labrafil M 2375" (polyoxyethylene glycerol trioleate from Gattefosee, Paris), "Labrafil M 1944 CS" (unsaturated polyglycolated glycerides prepared by an alcoholysis of apricot kernel oil and made up of glycerides and polyethylene glycol esters; from Gattefosee, Paris), "Labrasol" (saturated polyglycolated glycerides prepared by an alcoholysis of TCM and made up of glycerides and polyethylene glycol esters; from Gattefosee, Paris) and/or "Miglyol 812" (triglyceride of saturated fatty acids of chain length C8 to C12 from Huls AG, Germany), and in particular vegetable oils, such as cottonseed oil, almond oil, olive oil, castor oil, sesame oil, soybean oil and, in particular, groundnut oil.

The preparation of the injection compositions is carried out in the customary manner under sterile conditions, as are bottling, for example in ampoules or vials, and closing of the containers.

For example, pharmaceutical compositions for oral use can be obtained by combining the active ingredient with one or more solid carriers, if appropriate granulating the resulting mixture, and, if desired, processing the mixture or granules to tablets or coated tablet cores, if appropriate by addition of additional excipients.

Suitable carriers are, in particular, fillers, such as sugars, for example lactose, sucrose, mannitol or sorbitol cellulose preparations and/or calcium phosphates, for example tricalcium phosphate, or calcium hydrogen phosphate, and furthermore binders, such as starches, for example maize, wheat, rice or potato starch, methylcellulose, hydroxypropylmethylcellulose, sodium carboxymethylcellulose and/or polyvinyl-pyrrolidine, and/or, if desired, desintegrators, such as the above mentioned starches, and furthermore carboxymethyl-starch, cross-linked polyvinylpyrrolidone, alginic acid or a salt thereof, such as sodium alginate. Additional excipients are, in particular, flow regulators and lubricants, for example salicylic acid, talc, stearic acid or salts thereof, such as magnesium stearate or calcium stearate, and/or polyethylene glycol, or derivatives thereof.

Coated tablet cores can be provided with suitable coatings which, if appropriate, are resistant to gastric juice, the coatings used being, inter alia, concentrated sugar solutions, which, if appropriate, comprise gum arabic, talc, polyvinylpyrrolidine, polyethylene glycol and/or titanium dioxide, coating solutions in suitable organic solvents or solvent mixtures or, for the preparation of coatings which are resistant to gastric juice, solutions of suitable cellulose preparations, such as acetylcellulose phthalate or hydroxypropylmethylcellulose phthalate.

By "controlled release" it is meant for purposes of the present disclosure that therapeutically active compound is released from the preparation at a controlled rate or at a specific site, for example, the intestine, or both such that therapeutically beneficial blood levels (but below toxic levels) are maintained over an extended period of time, e.g., providing a 12 hour or a 24 hour dosage form.

The term "rate controlling polymer" as used herein includes hydrophilic polymers, hydrophobic polymers or mixtures of hydrophilic and/or hydrophobic polymers that are capable of retarding the release of the compounds in vivo. In addition, many of the same polymers can be utilized to create an enteric coating of a drug, drug suspension, or drug matrix. It is within the skill of those in the art to modify the coating thickness, permeability, and dissolution characteristics to provide the desired controlled release profile (e.g., drug release rate and locus) without undue experimentation.

Examples of suitable controlled release polymers to be used in this disclosure include hydroxyalkylcellulose, such as hydroxypropylcellulose and hydroxypropylmethylcellulose; poly(ethylene)oxide; alkylcellulose such as ethycellulose and methylcellulose; carboxymethylcellulose; hydrophilic cellulose derivatives; polyethylene glycol; polyvinylpyrrolidone; cellulose acetate; cellulose acetate butyrate; cellulose acetate phthalate; cellulose acetate trimellitate; polyvinylacetate phthalate; hydroxypropylmethylcellulose phthalate; hydroxypropylmethylcellulose acetate succinate; poly(alkyl methacrylate); and poly (vinyl acetate). Other suitable hydrophobic polymers include polymers or copolymers derived from acrylic or methacrylic acid esters, copolymers of acrylic and methacrylic acid esters, zein, waxes, shellac and hydrogenated vegetable oils.

To ensure correct release kinetics, the controlled release preparation of this disclosure contains about 5 and 75% by weight, preferably about 20 and 50% by weight, more preferably about 30 to 45% by weight controlled release polymer(s) and about 1 to 40% by weight, preferably about 3 to 25% by weight active compounds. The controlled release preparation according to the disclosure can preferably include auxiliary agents, such as diluents, lubricants and/or melting binders. Preferably, the excipients are selected to minimize the water content of the preparation. Preferably, the preparation includes an antioxidant. Suitable diluents include pharmaceutically acceptable inert fillers such as microcrystalline cellulose, lactose, dibasic calcium phosphate, saccharides, and/or mixtures of any of the foregoing. The diluent is suitably a water soluble diluent. Examples of diluents include microcrystalline cellulose such as Avicel ph112, Avicel pH101 and Avicel pH102; lactose such as lactose monohydrate, lactose anhydrous, and Pharmatose DCL 21; dibasic calcium phosphate such as Emcompress; mannitol; starch; sorbitol; sucrose; and glucose. Diluents are carefully selected to match the specific formulation with attention paid to the compression properties. Suitable lubricants, including agents that act on the flowability of the powder to be compressed are, for example, colloidal silicon dioxide such as Aerosil 200; talc; stearic acid, magnesium stearate, and calcium stearate. Suitable low temperature melting binders include polyethylene glycols such as PEG 6000; cetostearyl alcohol; cetyl alcohol; polyoxyethylene alkyl ethers; polyoxyethylene castor oil derivatives; polyoxyethylene sorbitan fatty acid esters; polyoxyethylene stearates; poloxamers; and waxes.

To improve the stability in the controlled release preparation, an antioxidant compound can be included. Suitable antioxidants include sodium metabisulfite; tocopherols such as alpha, beta, or delta-tocopherol tocopherol esters and alpha-tocopherol acetate; ascorbic acid or a pharmaceutically acceptable salt thereof; ascorbyl palmitate; alkyl gallates such as propyl gallate, Tenox PG, Tenox s-1; sulphites or a pharmaceutically acceptable salt thereof; BHA; BHT; and monothioglycerol.

The controlled release preparation according to the disclosure preferably can be manufactured by blending the compounds with the controlled release polymer(s) and auxiliary excipients followed by direct compression. Other methods for manufacturing the preparation include melt granulation. Preferred melt granulation techniques include melt granulation together with the rate controlling polymer(s) and diluent(s) followed by compression of the granules and melt granulation with subsequent blending with the rate controlling polymer(s) and diluents followed by compression of the blend. As desired prior to compression, the blend and/or granulate can be screened and/or mixed with auxiliary agents until an easily flowable homogeneous mixture is obtained.

Oral dosage forms of the controlled release preparation according to the disclosure can be in the form of tablets, coated tablets, enterically coated tablets or can be multiparticulate, such as in the form of pellets or mini-tablets. If desired, capsules such as hard or soft gelatin capsules, can contain the multiparticulates. If desired, the multiparticulate oral dosage forms can comprise a blend of at least two populations of pellets or mini-tablets having different controlled-release in vitro and/or in vivo release profiles. If desired, one of the pellet or mini-tablet populations can comprise immediate release multiparticulate, such as multiparticulates formed by conventional means.

If desired, the controlled release matrix tablets or multiparticulates of this disclosure can be coated with a controlled release polymer layer so as to provide additional controlled release properties. Suitable polymers that can be used to form this controlled release layer include the rate controlling polymers listed above.

As desired, the tablets, pellets or mini-tablets according to the disclosure can be provided with a light-protective and/or cosmetic film coating, for example, film-formers, pigments, anti-adhesive agents and plasticizers. Such a film former may consist of fast-dissolving constituents, such as low-viscosity hydroxypropylmethylcelluose, for example Methocel E5 or D14 or Pharmacoat 606 (Shin-Etsu). The film coating may also contain excipients customary in film-coating procedures, such as light-protective pigments, for example iron oxide, or titanium dioxide, anti-adhesive agents, for example talc, and also suitable plasticizers such as PEG 400, PEG 6000, and diethyl phthalate or triethyl citrate.

The controlled release polymer of this disclosure may consist of a hydrogel matrix. For instance, the compounds can be compressed into a dosage form containing a rate controlling polymer, such as HPMC, or mixture of polymers which when wet will swell to form a hydrogel. The rate of release from this dosage form is controlled both by diffusion from the swollen tablet mass and by erosion of the tablet surface over time. The rate of release may be controlled both by the amount of polymer per tablet and by the inherent viscosities of the polymers used.

Dyes or pigments can be admixed to the tablets or coated tablet coatings, for example for identification or characterization of different doses of active ingredient.

Pharmaceutical compositions, which can be used orally, are also hard capsules of gelatin and soft, closed capsules of gelatin and a plasticizer, such as glycerol or sorbitol. The hard capsules can contain the active ingredient in the form of granules, mixed for example with fillers, such as maize starch, binders and/or lubricants, such as talc or magnesium stearate, and stabilizers if appropriate. In soft capsules, the active ingredient is preferably dissolved or suspended in suitable liquid excipients, such as greasy oils, paraffin oil or liquid polyethylene glycols or fatty acid esters of ethylene glycol or propylene glycol, it being likewise possible to add stabilizers and detergents, for example of the polyethylene sorbitan fatty acid ester type.

Other oral forms of administration are, for example, syrups prepared in the customary manner, which comprise the active ingredient, for example, in suspended form and in a concentration of about 5% to 20%, preferably about 10% or in a similar concentration which results in a suitable individual dose, for example, when 5 or 10 ml are measured out. Other forms are, for example, also pulverulent or liquid concentrates for preparing of shakes, for example in milk. Such concentrates can also be packed in unit dose quantities.

Pharmaceutical compositions, which can be used rectally, are, for example, suppositories that comprise a combination of the active ingredient with a suppository base. Suitable suppository bases are, for example, naturally occurring or synthetic triglycerides, paraffin hydrocarbons, polyethylene glycols or higher alkanols.

Compositions which are suitable for parenteral administration are aqueous solutions of an active ingredient in water-soluble form, for example of water-soluble salt, or aqueous injection suspensions, which comprise viscosity-increasing substances, for example sodium carboxymethylcellulose, sorbitol and/or dextran, and if appropriate stabilizers. The active ingredient can also be present here in the form of a lyophilisate, if appropriate together with excipients, and be dissolved before parenteral administration by addition of suitable solvents. Solutions such as are used, for example, for parental administration can also be used as infusion solutions. Preferred preservatives are, for example. Antioxidants, such as ascorbic acid, or microbicides, such as sorbic or benzoic acid.

Ointments are oil-in-water emulsions, which comprise not more than 70%, but preferably 20-50% of water or aqueous phase. The fatty phase consists, in particular, hydrocarbons, for example vaseline, paraffin oil or hard paraffin's, which preferably comprise suitable hydroxy compounds, such as fatty alcohol's or esters thereof, for example cetyl alcohol or wool wax alcohols, such as wool wax, to improve the water-binding capacity. Emulsifiers are corresponding lipophilic substances, such as sorbitan fatty acid esters (Spans), for example sorbitan oleate and/or sorbitan isostearate. Additives to the aqueous phase are, for example, humectants, such as polyalcohols, for example glycerol, propylene glycol, sorbitol and/or polyethylene glycol, or preservatives and odoriferous substances.

Fatty ointments are anhydrous and comprise, as the base, in particular, hydrocarbons, for example paraffin, vaseline or paraffin oil, and furthermore naturally occurring or semi-synthetic fats, for example hydrogenated coconut-fatty acid triglycerides, or, preferably, hydrogenated oils, for example hydrogenated groundnut or castor oil, and furthermore fatty acid partial esters of glycerol, for example glycerol mono- and/or distearate, and for example, the fatty alcohols. They also contain emulsifiers and/or additives mentioned in connection with the ointments which increase uptake of water.

Creams are oil-in-water emulsions, which comprise more than 50% of water. Oily bases used are, in particular, fatty alcohols, for example lauryl, cetyl or stearyl alcohols, fatty acids, for example palmitic or stearic acid, liquid to solid waxes, for example isopropyl myristate, wool wax or beeswax, and/or hydrocarbons, for example vaseline (petrolatum) or paraffin oil. Emulsifiers are surface-active substances with predominantly hydrophilic properties, such as corresponding nonionic emulsifiers, for example fatty acid esters of polyalcohols or ethyleneoxy adducts thereof, such as polyglyceric acid fatty acid esters or polyethylene sorbitan fatty esters (Tweens), and furthermore polyoxyethylene fatty alcohol ethers or polyoxyethylene fatty acid esters, or corresponding ionic emulsifiers, such as alkali metal salts of fatty alcohol sulfates, for example sodium lauryl sulfate, sodium cetyl sulfate or sodium stearyl sulfate, which are usually used in the presence of fatty alcohols, for example cetyl stearyl alcohol or stearyl alcohol. Additives to the aqueous phase are, inter alia, agents which prevent the creams from drying out, for example polyalcohols, such as glycerol, sorbitol, propylene glycol and/or polyethylene glycols, and furthermore preservatives and odoriferous substances.

Pastes are creams and ointments having secretion-absorbing powder constituents, such as metal oxides, for example titanium oxide or zinc oxide, and furthermore talc and/or aluminum silicates, which have the task of binding the moisture or secretions present.

Foams are administered from pressurized containers and they are liquid oil-in-water emulsions present in aerosol for. As the propellant gases, halogenated hydrocarbons, such as chlorofluoro-lower alkanes, for example dichlorofluoromethane and dichlorotetrafluoroethane, or, preferably, non-halogenated gaseous hydrocarbons, air, N.sub.2 O, or carbon dioxide are used. The oily phases used are, inter alia, those mentioned above for ointments and creams, and the additives mentioned there are likewise used.

Tinctures and solutions usually comprise an aqueous-ethanolic base to which, humectants for reducing evaporation, such as polyalcohols, for example glycerol, glycols and/or polyethylene glycol, and re-oiling substances, such as fatty acid esters with lower polyethylene glycols, i.e. lipophilic substances soluble in the aqueous mixture to substitute the fatty substances removed from the skin with the ethanol, and, if necessary, other excipients and additives, are admixed.

### Methods of Treatment

The disclosure also relates to a process or method for treatment of the disease states. The oligourea compounds or chimeric compounds can be administered prophylactically or therapeutically as such or in the form of pharmaceutical compositions, preferably in an amount, which is effective against the diseases mentioned. With a warm-blooded animal, for example a human, requiring such treatment, the compounds are used, in particular, in the form of pharmaceutical composition. A daily dose of about 0.1 to about 5 g, preferably 0.5 g to about 2 g, of a compound of the present disclosure is administered here for a body weight of about 70 kg.

The description provides methods of treating a disease or disorder or ameliorating the effects of the same comprising the steps of administering to an individual in need thereof, a composition comprising an effective amount of a chimeric compound or a oligourea compound as described herein, and a pharmaceutically acceptable carrier or excipient, wherein the composition is effective for treating, preventing or ameliorating the effects of the disease or disorder.

The compounds described above are used for the manufacture of a medication for use in the treatment of a disease, disorder or condition. The term "disease involving deregulation of cell proliferation and/or angiogenesis" means, in the context of the disclosure, any human or animal disease affecting one or more organs. Exemplary diseases include, but are not limited to, rheumatoid arthritis, osteoarthritis, juvenile chronic arthritis, Lyme arthritis, psoriatic arthritis, reactive arthritis, spondyloarthropathy, systemic lupus erythematosus, Crohn's disease, ulcerative colitis, inflammatory bowel disease, insulin dependent diabetes mellitus, thyroiditis, asthma, allergic diseases, psoriasis, dermatitis sclerodenna, atopic dermatitis, graft versus host disease, organ transplant rejection, acute or chronic immune disease associated with organ transplantation, sarcoidosis, atherosclerosis, disseminated intravascular coagulation, Kawasaki's disease, Grave's disease, nephrotic syndrome, chronic fatigue syndrome, Wegener's granulomatosis, Henoch-Schoenlein purpurea, microscopic vasculitis of the kidneys, chronic active hepatitis, uveitis, septic shock, toxic shock syndrome, sepsis syndrome, cachexia, infectious diseases, parasitic diseases, acquired immunodeficiency syndrome, acute transverse myelitis, Huntington's chorea, Parkinson's disease, Alzheimer's disease, stroke, primary biliary cirrhosis, hemolytic anemia, malignancies, heart failure, myocardial infarction, Addison's disease, sporadic, polyglandular deficiency type I and polyglandular deficiency type II, Schmidt's syndrome, adult (acute) respiratory distress syndrome, alopecia, alopecia areata, seronegative arthopathy, arthropathy, Reiter's disease, psoriatic arthropathy, ulcerative colitic arthropathy, enteropathic synovitis, chlamydia, yersinia and salmonella associated arthropathy, spondyloarthopathy, atheromatous disease/arteriosclerosis, atopic allergy, autoimmune bullous disease, pemphigus vulgaris, pemphigus foliaceus, pemphigoid, linear IgA disease, autoimmune haemolytic anaemia, Coombs positive haemolytic anaemia, acquired pernicious anaemia, juvenile pernicious anaemia, myalgic encephalitis/Royal Free Disease, chronic mucocutaneous candidiasis, giant cell arteritis, primary sclerosing hepatitis, cryptogenic autoimmune hepatitis, Acquired Immunodeficiency Disease Syndrome, Acquired Immunodeficiency Related Diseases, Hepatitis C, common varied immunodeficiency (common variable hypogammaglobulinaemia), dilated cardiomyopathy, female infertility, ovarian failure, premature ovarian failure, fibrotic lung disease, cryptogenic fibrosing alveolitis, post-inflammatory interstitial lung disease, interstitial pneumonitis, connective tissue disease associated interstitial lung disease, mixed connective tissue disease associated lung disease, systemic sclerosis associated interstitial lung disease, rheumatoid arthritis associated interstitial lung disease, systemic lupus erythematosus associated lung disease, dermatomyositis/polymyositis associated lung disease, Sjodgren's disease associated lung disease, ankylosing spondylitis associated lung disease, vasculitic diffuse lung disease, haemosiderosis associated lung disease, drug-induced interstitial lung disease, radiation fibrosis, bronchiolitis obliterans, chronic eosinophilic pneumonia, lymphocytic infiltrative lung disease, postinfectious interstitial lung disease, gouty arthritis, autoimmune hepatitis, type-1 autoimmune hepatitis (classical autoimmune or lupoid hepatitis), type-2 autoimmune hepatitis (anti-LKM antibody hepatitis), autoimmune mediated hypoglycemia, type B insulin resistance with acanthosis nigricans, hypoparathyroidism, acute immune disease associated with organ transplantation, chronic immune disease associated with organ transplantation, osteoarthrosis, primary sclerosing cholangitis, idiopathic leucopenia, autoimmune neutropenia, renal disease NOS, glomerulonephritides, microscopic vasulitis of the kidneys, lyme disease, discoid lupus erythematosus, male infertility idiopathic or NOS, sperm autoimmunity, multiple sclerosis (all subtypes), insulin-dependent diabetes mellitus, sympathetic ophthalmia, pulmonary hypertension secondary to connective tissue disease, Goodpasture's syndrome, pulmonary manifestation of polyarteritis nodosa, acute rheumatic fever, rheumatoid spondylitis, Still's disease, systemic sclerosis, Takayasu's disease/arteritis, autoimmune thrombocytopenia, idiopathic thrombocytopenia, autoimmune thyroid disease, hyperthyroidism, goitrous autoimmune hypothyroidism (Hashimoto's disease), atrophic autoimmune hypothyroidism, primary myxoedema, phacogenic uveitis, primary vasculitis and vitiligo. The human antibodies, and antibody portions of the disclosure can be used to treat autoimmune diseases, in particular those associated with inflammation, including, rheumatoid spondylitis, allergy, autoimmune diabetes, autoimmune uveitis.

The methods herein include administering to the subject (including a subject identified as in need of such treatment) an effective amount of a compound described herein, or a composition described herein to produce a desired effect. Identifying a subject in need of such treatment can be in the judgment of the subject or a health care professional and can be subjective (e.g., opinion) or objective (e.g., measurable by a test or diagnostic method). The therapeutic methods of the disclosure, which include prophylactic treatment, in general comprise administration of a therapeutically effective amount of at least one of the compounds herein, such as a compound of the formulae herein to a subject (e.g., animal, human) in need thereof, including a mammal, particularly a human. Such treatment will be suitably administered to subjects, particularly humans, suffering from, having, susceptible to, or at risk for a disease, disorder, or symptom thereof. Determination of those subjects "at risk" can be made by any objective or subjective determination by a diagnostic test or opinion of a subject or health care provider (e.g., genetic test, enzyme or protein marker, Marker (as defined herein), family history, and the like).

In another aspect, the present description provides methods of making and using the peptide-oligourea chimeric compounds or the oligourea compounds as described herein. For example, the peptide-oligourea chimeric compounds or the oligourea compound as described herein can be used as a diagnostic agent or a therapeutic agent for the treatment of a disease or condition.

In one embodiment, the disclosure provides a method of monitoring treatment progress. The method includes the step of determining a level of diagnostic marker (Marker) (e.g., any target delineated herein modulated by a compound herein, a protein or indicator thereof, etc.) or diagnostic measurement (e.g., screen, assay) in a subject suffering from or susceptible to a disorder or symptoms thereof associated with protein-expression related disease (including misfolding), in which the subject has been administered a therapeutic amount of a compound herein sufficient to treat the disease or symptoms thereof. The level of Marker determined in the method can be compared to known levels of Marker in either healthy normal controls or in other afflicted patients to establish the subject's disease status. In certain embodiments, a second level of Marker in the subject is determined at a time point later than the determination of the first level, and the two levels are compared to monitor the course of disease or the efficacy of the therapy. In certain embodiments, a pre-treatment level of Marker in the subject is determined prior to beginning treatment according to this disclosure; this pre-treatment level of Marker can then be compared to the level of Marker in the subject after the treatment commences, to determine the efficacy of the treatment.

The active compound is included in the pharmaceutically acceptable carrier or diluent in an amount sufficient to deliver to a patient a therapeutically effective amount for the desired indication, without causing serious toxic effects in the patient treated. A preferred dose of the active compound for all of the herein-mentioned conditions is in the range from about 10 ng/kg to 300 mg/kg, preferably 0.1 to 100 mg/kg per day, more generally 0.5 to about 25 mg per kilogram body weight of the recipient/patient per day. A typical topical dosage will range from 0.01-5% wt/wt in a suitable carrier. The compound is conveniently administered in any suitable unit dosage form, including but not limited to one containing less than 1mg, 1 mg to 3000 mg, preferably 5 to 500 mg of active ingredient per unit dosage form. An oral dosage of about 25-250 mg is often convenient. The active ingredient is preferably administered to achieve peak plasma concentrations of the active compound of about 0.00001-30 mM, preferably about 0.1-30 µM.

This may be achieved, for example, by the intravenous injection of a solution or formulation of the active ingredient, optionally in saline, or an aqueous medium or administered as a bolus of the active ingredient. Oral administration is also appropriate to generate effective plasma concentrations of active agent. The concentration of active compound in the drug composition will depend on absorption, distribution, inactivation, and excretion rates of the drug as well as other factors known to those of skill in the art. It is to be noted that dosage values will also vary with the severity of the condition to be alleviated. It is to be further understood that for any particular subject, specific dosage regimens should be adjusted overtime according to the individual need and the professional judgment of the person administering or supervising the administration of the compositions, and that the concentration ranges set forth herein are exemplary only and are not intended to limit the scope or practice of the claimed composition. The active ingredient may be administered at once, or may be divided into a number of smaller doses to be administered at varying intervals of time.

### Methods of Preparation

In another aspect, the present description provides methods of making and using the compounds of the disclosure. For example, in one embodiment, the description provides a method of making an oligourea compound of the disclosure comprising fabricating a oligourea helical bundle or super-helical nanotube with a non-peptide oligourea peptidomimetic residue sequence selected from the group consisting of: a helical pentad repeat (*a, b, c, d, e*): oligourea peptidomimetic residues with a hydrophobic side chain in positions *a* and *d* (e.g., Leu^{u}), oligourea peptidomimetic residues with a charged residue in position b and *c* (e.g. Glu^{u} and Lys^{u}, respectively), and Tyr^{u} and Ala^{u} in position e; and a helical pentad repeat (*a, b, c, d, e*): oligourea peptidomimetic residues with a hydrophobic side chain in positions a and c (e.g. Leu^{u}), oligourea peptidomimetic residues with a hydrophobic side chain in position e (e.g., Ala^{u} and Pro^{u} residues at the e position), and oligourea peptidomimetic residues with a charged side chain in positions b and *d* (e.g., Glu^{u} and Lys^{u}, respectively). In another embodiment, the oligourea helical bundle with a non-peptide oligourea peptidomimetic residue sequence selected from the group consisting of: Leu^{U} Glu^{U} Lys^{U} Leu^{U} Tyr^{U} Leu^{U} Glu^{U} Lys^{U} Leu^{U} Ala^{U} Leu^{U} (H1); Leu^{U} Glu^{U} Leu^{U} Lys^{U} Pro^{U} Leu^{U} Glu^{U} Leu^{U} Lys^{U} Ala^{U} (H2); Leu^{U} Glu^{U} Lys^{U} Leu^{U} Tyr^{U} Asn^{U} Glu^{U} Lys^{U} Leu^{U} Ala^{U} Leu^{U} (H3); Ser^{U} Glu^{U} Lys^{U} Leu^{U} Tyr^{U} Leu^{U} Glu^{U} Lys^{U} Leu^{U} Ala^{U} Leu^{U} (H4); and Ala^{U} Leu^{U} Lys^{U} Leu^{U} Glu^{U} Tyr^{U} Leu^{U} Glu^{U} Leu^{U} Lys^{U} Ala^{U} Leu^{U} (H5).

Additional, exemplary methods for performing the synthesis of chimeric compounds as described herein are provided below.

It is understood that the examples and embodiments described herein are for illustrative purposes only and that various substitutions, modifications or changes in light thereof will be suggested to persons skilled in the art and are included within the spirit and purview of this application and are considered within the scope of the appended claims. The following examples are given by way of example of the preferred embodiments, and are in no way considered to be limiting to the disclosure. For example, the relative quantities of the ingredients may be varied to achieve different desired effects, additional ingredients may be added, and/or similar ingredients may be substituted for one or more of the ingredients described. All publications, patents, and patent applications cited herein are hereby incorporated by reference in their entirety for all purposes.

### Examples

**Design of amphiphilic oligourea foldamers for aqueous self-assembly.** Two short oligourea sequences that result in amphiphilic structures with well-defined and distinct hydrophobic and charged regions when folded as canonical 2.5-helices were designed. In certain embodiments, the sequestration of hydrophobic side-chains provide the driving force for self-assembly in acqueous conditions and the complementary charged side-chains facilitate inter-helical interactions. The primary sequences of oligoureas H1 or H2 are shown in Figure 1B or Figure 1C. H1 and H2 of Figures 1C and 1B primarily differ in their distribution of the charged and non-charged side-chains. By analogy to α-peptides known to form helical budles (Hill, R. B., Raleigh, D. P., Lombardi, A. & DeGrado, W. F. De novo design of helical bundles as models for understanding protein folding and function. Acc. Chem. Res. 33, 745-754 (2000); and Woolfson, D. N. The design of coiled-coil structures and assemblies. Adv. Protein Chem. 70, 79-112 (2005)), the helix of H1 contains a hydrophobic face formed from two contiguous position so the helical pentad repeat (*a* and *d*) composed entirely of leucine-type urea residues (Leu^{u}-superscript 'u' denotes urea residue), flanked on either side by charged residues: glutamate-type urea residues (Glu^{u}) at position *b*, and lysine-type urea residues (Lys^{u}) at position c (Fig. 1A and B). The fifth and final helical pentad position (*e*) - situated opposite the hydrophobic face - is composed of tyrosine-type (Tyr^{u}) and alanine-type (Ala^{u}) urea residues. In contrast, the helix of **H2** contains a more extended hydrophobic face formed from three contiguous non-charged helical positions, composed of Leu^{u} residues at the *a* and *c* positions, and Ala^{u} and Pro^{u} residues at the e position (Fig. 1C) (in contrast to proline amino acid residues, urea proline-type [Pro^{u}] residues have been shown to be helico-compatible in Fremaux, J., et al., G. Condensation approach to aliphatic oligourea foldamers: helices with N-(pyrrolidin-2-ylmethyl)ureido junctions. Angew. Chem. Int. Ed Engl. 50, 11382-11385 (2011)). The remaining two contiguous helical positions - *b* and *d -* are composed of charged Glu^{u} and Lys^{u} residues, respectively. Oligoureas **H1** and **H2** were synthesized chemically following previously reported procedures (Douat-Casassus, C., et al. Microwave-enhanced solid-phase synthesis of N,N'-linked aliphatic oligoureas and related hybrids. Org. Lett. 14, 3130-3133 (2012); and Fremaux, J., et al. Condensation approach to aliphatic oligourea foldamers: helices with N-(pyrrolidin-2-ylmethyl)ureido junctions. Angew. Chem. Int. Ed Engl. 50, 11382-11385 (2011)).

Circular dichroism (CD) experiments showed both molecules to be moderately helical in aqueous conditions at relatively low concentrations (< 10 µM), and revealed a clear stabilizing influence of increasing foldamer concentration (from 3.125 µM to 200 µM) on the molar ellipticity of both molecules (Fig. 1D and Fig. 2) - a strong indicator of the existence of quaternary assemblies (Daniels, D. S., Petersson, E. J., Qiu, J. X. & Schepartz, A. High-resolution structure of a beta-peptide bundle. J. Am. Chem. Soc. 129, 1532-1533 (2007)). Next, crystals of oligoureas **H1** and **H2** were grown from aqueous crystallisation conditions and X-ray structures determined with resolutions of 1.25 Å and 1.4 Å for **H1** and **H2**, respectively.

**High-resolution crystallographic studies of oligoureas H1 and H2.** The crystal structure of **H1** reveals a non-cylindrical helical-bundle formed from the assembly of six well-defined canonical oligourea helices, with a hydrophobic Leu^{u}-rich core and a charged, hydrated exterior (Fig. 1B and E). As anticipated, hydrophobic interactions appear to play a key role in helical bundle assembly, with all of the leucine-type side-chains orientated towards the central hydrophobic core of the bundle, the majority of them interlocking in a manner highly reminiscent of the 'knobs-into-holes' (KIH) type packing of natural α-helical bundles (Hill, R. B., et al. De novo design of helical bundles as models for understanding protein folding and function. Acc. Chem. Res. 33, 745-754 (2000); Woolfson, D. N. The design of coiled-coil structures and assemblies. Adv. Protein Chem. 70, 79-112 (2005); Harbury, P. B., et al. High-resolution protein design with backbone freedom. Science 282, 1462-1467 (1998); and O'Shea, E. K., et al. X-ray structure of the GCN4 leucine zipper, a two-stranded, parallel coiled coil. Science 254, 539-544 (1991)) (Fig. 1D and Fig. 3). The helical bundle is formed from three **H1** dimers, each dimer being composed of two anti-parallel helices held together by: 1) Leu^{u}-Leu^{u} packing interactions and 2) a mirrored hydrogen bond between the amine of the Lys^{u}₈ side-chain and the free carbonyl group of the C-terminal Leu^{u}₁₁ residue (Fig. 3). Three such dimers interact primarily through further hydrophobic Leu^{u}-Leu^{u} interactions to form a complete discrete hexameric helical bundle - a stoichiometry which is rare among α-peptide helical bundles (Zaccai, N. R. et al. A de novo peptide hexamer with a mutable channel. Nat. Chem. Biol. 7, 935-941 (2011)), and unheard of amongst foldamer helical bundles. No salt-bridges are observed within the oligourea helical bundle - despite the inclusion of four chargeable residues per helix - highlighting the significant role played by hydrophobic forces in bundle assembly. Also unexpected was the discovery of an isolated cavity within the hydrophobic core of the bundle (Fig. 1E). The cavity has a volume of 491.3 Å³ and is therefore theoretically well-suited for the encapsulation of guest molecules in the size-range of, for example, pharmaceutical compounds.

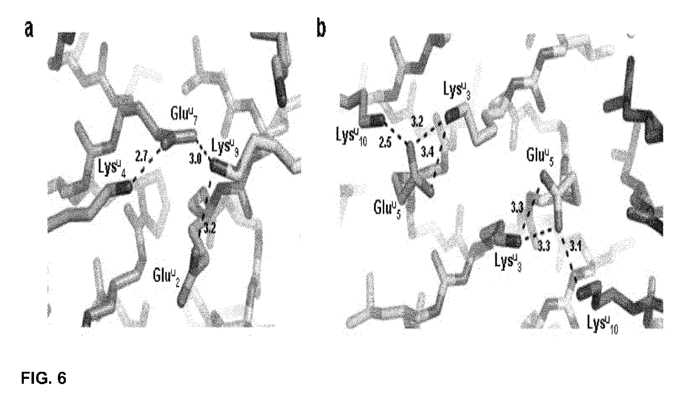

In contrast to oligourea **H1**, the crystal structure of **H2** reveals higher-order assemblies of a dramatically different arrangement, with the **H2** oligourea helices organizing to create an extended channel-type assembly with a highly charged, water-filled interior pore with an internal diameter of 17 Å (Fig. 1C and F). The exterior surface is composed entirely of hydrophobic Leu^{u}, Ala^{u} and Pro^{u} residues, which pack in a dense and precise manner, with the interior pore composed entirely of charged Lys^{u} and Glu^{u} residues. The channel-type assembly is composed of two intertwined right-handed super-helices, with each super-helix composed of individual canonical oligourea helices packed in a staggered side-by-side manner (with 12 oligourea helices per super-helical turn), locked together by a combination of hydrophobic packing interactions and extensive inter-helical salt-bridges (Fig. IF and Figs. 4 and 5). Indeed, salt-bridges appear to play a major role in stabilizing the channel structure, as the majority of the chargeable side-chains are involved in intermolecular electrostatic contacts, forming intricate networks of salt-bridges linking adjacent and distal oligourea molecules (Fig. 6). The hydrophilic pore of the channel is heavily hydrated, and analysis of the electron density maps reveals the presence of both well-ordered and mobile water molecules (Fig. IF, inset). This evidence of molecular mobility within the pore raises the possibility that such channels could potentially be of use within the context of, for example, ion conductance (Lear, J. D., et al. Synthetic amphiphilic peptide models for protein ion channels. Science 240, 1177-1181 (1988)). In order to more fully understand the nature of the assemblies observed crystallographically, a series of biophysical and solution analyses on molecules **H1** and **H2**.

**Biophysical characterization and molecular basis of bundle formation of oligourea H1.** The stoichiometry of the **H1** helical bundle evidenced by the crystal structure was investigated by native electrospray ionization mass spectrometry (ESI-MS), a technique well-suited for the detection and investigation of non-covalent assemblies (Lai, Y.-T. et al. Structure of a designed protein cage that self-assembles into a highly porous cube. Nat. Chem. 6, 1065-1071 (2014); and Hernández, H. & Robinson, C. V. Determining the stoichiometry and interactions of macromolecular assemblies from mass spectrometry. Nat. Protoc. 2, 715-726 (2007)). Native ESI-MS analysis of a 100 µM solution of **H1** (in 20 mM aqueous ammonium acetate) reveals a predominant multimer species with an m/z of 2083.4 - attributed unambiguously to a hexameric species ([**H1**₆]⁵⁺), and therefore in excellent agreement with the crystal structure (Fig. 7A). In order to corroborate this result, a third oligourea (**H3**) was synthesized as a negative control. **H3** is analogous to **H1**, but bears an asparagine-type residue (Asn^{u}) at position six (Fig. 7A, top). This mutation results in the insertion of a highly hydrophilic residue (Asn^{u}) within an otherwise fully hydrophobic helical face, and was believed it would impair the ability of this foldamer to self-assemble. The analysis of **H3** by ESI-MS under the same conditions as **H1** revealed no such predominant hexameric species (Fig. 7A), suggesting the hexameric species present in the **H1** spectrum to be significant. CD-monitored thermal melting experiments support the ESI-MS data, with **H3** revealing a significantly reduced thermal melting profile compared to **H1** (Fig. 7A). A Tₘ of 45.9°C can be derived from the melting profile of **H1** shown in Fig. 7A, suggesting that the helical bundle formed by **H1** has a certain degree of molten-globule-like character, which may be explained by the absence of salt-bridges evident from the crystal structure.

With the aim of investigating atomic-scale details of intermolecular interactions in solution, **H1** and **H3** were studied by high-field NMR. NOESY analysis of **H3** (at a concentration of 200 µM in either 100% D₂O or in 98% D₂O, 20 mM sodium acetate, pH 4.0) indicates a predominantly monomeric species, with no evidence of specific intermolecular interactions (Fig. 7B and Fig. 8). Variable-concentration CD analysis of oligourea **H3** (from 3.125 µM to 200 µM) shows this molecule unexpectedly displays a propensity to self-assemble only slightly lower than that of **H1** (Fig. 9A-9C), suggesting that **H3** may still self-associate to some degree in aqueous conditions, albeit into highly-dynamic and unstable assemblies of low stoichiometry. The relative size of **H3** compared to **H1** in aqueous conditions was investigated further by DOSY analysis, which indeed indicates - albeit qualitatively - that **H3** is noticeably smaller than **H1** in aqueous conditions (Fig. 9D). In contrast to **H3**, NOESY analysis of **H1** under the same conditions provides clear evidence of specific, stable helix-helix intermolecular interactions - for example, strong NOE cross-peaks can be seen corresponding to interactions between the aromatic δ or ε protons of Tyr^{u}₅ side-chains and the aliphatic δ1 or δ2 protons of Leu^{u}₁₁ side-chains (Fig. 7B, left and Fig. 8). As Tyr^{u}₅ and Leu^{u}₁₁ are positioned on opposite faces of the helix (Fig. 1B), these NOE signals necessarily correspond to specific intermolecular interactions. Similarly strong intermolecular NOEs can also be seen between the aliphatic protons of Leu^{u}₁ side-chains and those of Glu^{u}₂ side-chains from a different chain (Fig. 7B, right and Fig. 8). Importantly, these intermolecular NOEs correlate remarkably well with the crystal structure of **H1** (Fig. 7B, insets), and provide strong evidence that the helical bundle of **H1** observed in the crystal state is indeed representative of the quaternary structure of this foldamer in solution. Additional NMR data (including detailed peak assignments for **H1** and **H3**) is shown in Figs. 8 and 10).

In order to test whether the hexameric helical bundle arrangement formed by oligourea **H1** was structurally robust, we designed and synthesized an analogue of **H1** bearing a serine-type urea residue (Ser^{u}) in place of Leu^{u}₁ (oligourea **H4**) (Fig. 7A). Leu^{u}₁ was chosen for modification for at least the following reasons: 1) from the crystal structure of **H1** it is clear that, although this residue is orientated towards the hydrophobic core of the bundle, it does not appear to contribute extensively to the KIH-like packing of the remaining Leu^{u} residues (Fig. 3A), and 2) as a means to corroborate the intermolecular NOE Leu^{u}₁-Glu^{u}₂ interactions of **H1** described above. As with oligourea **H1**, a crystal structure was determined for **H4** (refined to 1.69 Å), revealing a hexameric helical bundle almost identical to that formed from **H1** (Cα r.m.s.d., 0.379 Å) (Fig. 7C). The incorporation of six Ser^{u} urea residues within the hexameric **H4** helical bundle results in the formation of water-bridged networks at the three-way N-terminal helix junctions, thereby increasing the number of indirect inter-helical contacts compared to the **H1** bundle (Fig. 7C, right). As with the **H1**-bundle, the **H4**-bundle also contains an isolated hydrophobic cavity, with a comparable yet slightly larger volume of 519.5 Å³. Variable-concentration CD experiments, CD-monitored thermal melting, native ESI-MS, and NMR studies all demonstrate **H4** to possess a tendency to self-assemble similar yet noticeably reduced compared to **H1**, suggesting an important, but non-essential role, played by Leu^{u}₁ in bundle formation (Fig. 11). In addition, the NOE peak corresponding to intermolecular Leu^{u}₁-Glu^{u}₂ interactions is absent in the **H4** NOESY spectra, confirming the assignment of this interaction in the **H1** spectra.

**Solution and imaging studies of oligourea H2.** The dramatically different quaternary assembly formed by **H2** compared to **H1** in the crystal state was corroborated by solution and biophysical studies. Native ESI-MS analysis of **H2** (in 20 mM aqueous ammonium acetate) revealed a significant reduction in discrete multimeric species in the spectra as compared to **H1** (Fig. 12A - *cf.* ion counts in Fig. 7A, recorded in identical conditions). MS/MS analysis indicates that this background noise is composed of **H2** monomers (Fig. 12B) - in good agreement with the presence of non-discrete assemblies. Analysis of **H2** by negative staining electron microscopy (EM) and cryo-EM under similar buffered sample conditions revealed the presence of extended homogeneous hollow tubular nanostructures with diameters of 7 and 4.6 nm, respectively (Fig. 13A and Fig. 14 and 15). These observations by EM are fully consistent with the molecular arrangement observed in the crystal. The detection of tubular structures by cryo-EM - a true solution technique - provides particularly strong evidence in support of the ability of **H2** to self-assemble into channel-type structures in aqueous conditions. NMR analysis of **H2** (in 100 % D₂O at pH 5.5) revealed an absence of distinct peaks within NOESY spectra, suggesting **H2** to be almost fully multimeric/aggregated in these conditions. The equivalent NMR analysis of **H2** performed at a lower pH of 4 - a pH at which the Glu^{u} side-chains would be expected to be protonated (and hence unable to form salt-bridges) - showed **H2** to be predominantly monomeric (Supplementary Fig. 12C), further demonstrating the importance of electrostatic contacts in **H2** self-assembly as evidenced by the crystal structure (see Figs. 12C and 12D and legend for further details and discussion). By regulating the formation of intermolecular salt-bridges, pH may act as a control mechanism for channel/tubule formation.

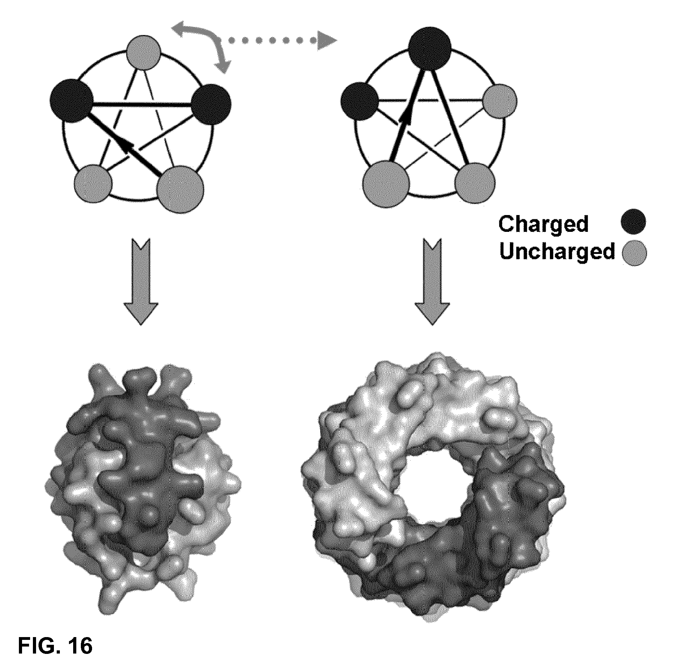

**Principles of control of oligourea aqueous self-assembly and channel pore diameter.** The key difference between oligoureas **H1** and **H2** relates to the distribution of the charged and uncharged residues, thus, the continuous charged helical face and the extended uncharged helical face of **H2** appear to be critical design elements that shift the self-assemblies from discrete helical bundles to tubular nano-structures. In order to test this hypothesis, an additional oligourea - **H5** - was synthesized, bearing uncharged residues at three contiguous helical pentad positions (two composed of Leu^{u} residues [*b* and *d*] and a third composed of Ala^{u} and Tyr^{u} residues [*a*]) and a continuous charged helical face composed of charged Lys^{u} and Glu^{u} residues (positions e and *c)* (Fig. 13B). Variable-concentration CD analysis revealed **H5** to possess a strong tendency to self-assemble in solution (Figs. 12E-12G), whilst native ESI-MS spectra revealed a clear absence of distinct multimeric species, accompanied by a significant level of high-mass background noise composed of **H5** monomers (as shown by MS/MS analysis) (Figs. 12H and 12I). Atomic-scale details of the assemblies indicated by the CD and native ESI-MS data were provided by a 1.47 Å crystal structure of **H5**, revealing a multi-stranded super-helical channel fundamentally similar to that formed from **H2**, yet differing noticeably in super-helix chain stoichiometry, with the **H5** channel formed from six intertwined right-handed super-helices (Fig. 13B). Each super-helix of the **H5** channel requires 24 oligourea molecules per complete super-helical turn. This, coupled with the increased super-helical chain stoichiometry, leads to an increase in the internal pore diameter of the **H5** channel - from 17 Å (channel **H2**) to 25 Å (channel **H5**) (Fig. 13C and 13D). Despite significant differences in the sequences of **H2** and **H5**, the individual super-helix chains of channels **H2** and **H5** are constructed in remarkably similar fashions, involving the staggered side-by-side packing of oligourea helices, locked together by a combination of hydrophobic interactions and extensive salt-bridge networks (Figs. 4, 5, 6, and 13B). As with the **H2** channel, the **H5** channel also has a densely packed, hydrophobic exterior (composed of Leu^{u}, Ala^{u} and Tyr^{u} residues) and a highly charged and hydrated interior pore. Oligourea **H5** therefore not only supports the proposition that uniting the charged and uncharged helical positions through sequence modification to form continuous charged and uncharged helical faces results in the formation of non-discrete channel-type structures (Fig. 16), but also indicates that the diameter of the channel pores can be modulated through the length and sequence of the foldamer building unit.

### A 1.7 Å crystal structure of H1+ reveals an artificial octameric helical bundle

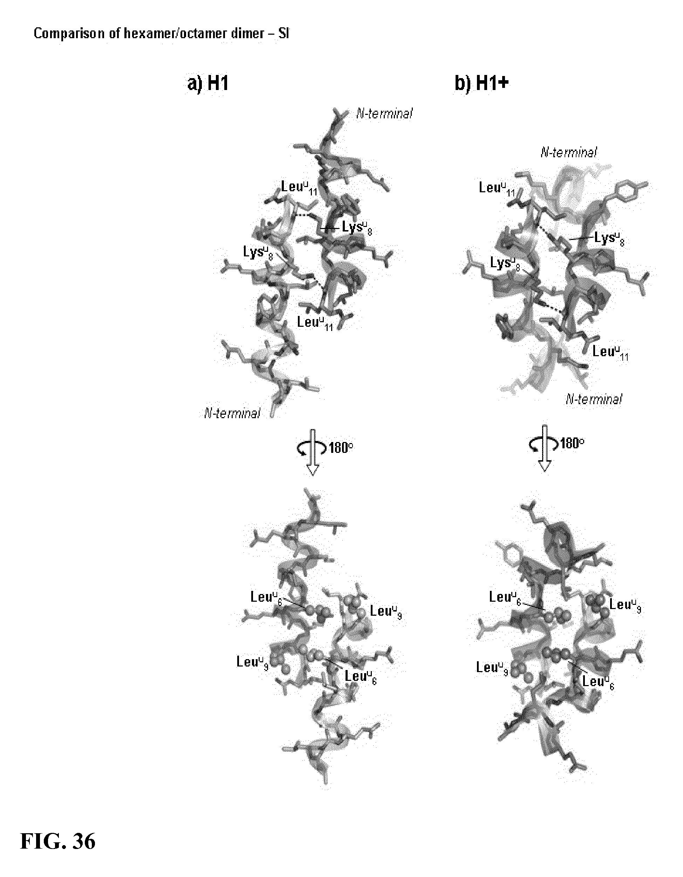

While variable-concentration CD studies showed **H1** and **H1**⁺ to display a similar tendency to self-assemble in H₂O, we wanted to investigate whether there were any significant structural consequences to removing a negative charge from the foldamer. Crystals of **H1**+ suitable for X-ray diffraction studies were obtained under aqueous conditions. A crystal structure of **H1**⁺ was determined and refined to 1.7 Å, revealing a dramatically different arrangement to the previously reported crystal structure of **H1**, with **H1**⁺ forming tightly-packed, globular octameric helical bundle. (See Figure 36). All leucine-type (Leu^{u}) side-chains of **H1**⁺ are orientated towards a central hydrophobic core, with all non-Leu^{u} residues - including the Lys^{u}, Glu^{u} and Gln^{u} residues - orientated towards the solvent. Despite the increase in helix stoichiometry of the **H1**⁺ octameric helical bundle compared to the hexameric helical bundle formed by **H1**, the two different helical assemblies are in fact constructed in remarkably similar fashions - indeed, both helical bundles could be considered to be constructed from the same helix dimer. This dimer - seen in both hexameric and octameric helical bundles - involves the anti-parallel arrangement of two helices, held together by a combination of hydrophobic Leu^{u}-Leu^{u} interactions and a mirrored hydrogen bond between the amine of the Lys^{u}₈ residues and the free carbonyl of Leu^{u}₁₁. Both the hexameric and octameric bundle are constructed from the side-to-side packing of three or four of these dimers (respectively). As a consequence, the Leu^{u}-Leu^{u} packing interactions within the hexameric and octameric bundle cores are highly conserved between the two arrangements, suggesting that the Leu^{u}₆-Leu^{u}₉-Leu^{u}₉-Leu^{u}₆ arrangement is a favourable hydrophobic packing motif

The octameric bundle appears to be more compact than the hexamer, and interestingly, the globular nature of **H1**⁺ appears to be the indirect result of the formation of extensive inter-helical (i.e. intra-bundle) salt-bridges. Indeed, the octameric arrangement contains the maximum possible number of salt bridges possible for this sequence (eight). The salt-bridges are all inter-helical and are formed between the Glu^{u}₂ and Lys^{u}₃ side-chains of neighbouring helices. Interestingly, in order for these salt-bridges to form, the helices need to 'bend' significantly in a directional manner. Indeed, all eight helices of the octameric bundle do not conform to the expected canonical oligourea helix geometry, but instead are all 'bent' in a directional and highly conserved manner. Surprisingly, the bending of the oligourea helices results in the insertion of water molecules into the subsequently enlarged helix grooves, and, crucially, the helical hydrogen bonding is not broken, but rather the water molecules bridge the carbonyl oxygen of residue *n* to the NHs of residue *n+2.* The insertion of one bridging water molecule has been observed previously in the crystal structure of a similarly bent oligourea 8-mer obtained in non aqueous conditions. Each helix of the octameric bundle has between 1 and 3 water molecules inserted between residues Glu^{u}₂ and Leu^{u}₄, and between residues Leu^{u}₄ and Leu^{u}₆. These intra-helical waters are therefore inserted within a common helical face, resulting in a considerable bending of the helix around a single axis (relative to the canonical helices of the hexameric **H1** bundle). The consequence of these intra-helical water-bridges and helical bending is that the octameric structure appears far more compact and globular than the hexamer, with the three N-terminal residues (Leu^{u}₁, Glu^{u}₂ and Lys^{u}₃) forming a 'cap' to the hydrophobic core. The bending of **H1**⁺ helices as a result of the insertion of relay water molecules is highly reminiscent of α-helices in proteins where curvature is known to play a significant role on packing interactions (Bhattacharyya, 1997 #2320).

Interestingly, as with the hexameric **H1** helical bundle, the octameric **H1**⁺ bundle also contains a void space within the hydrophobic core. Unlike the **H1** helical bundle however, the **H1**⁺ bundle void space is continuous with the solvent, tempting one to consider its future use as an active site (following further engineering of the foldamer).

The direct role of the Gln^{u} residues is subtle yet crucial - although these side-chains are situated in close proximity to one another on the surface of the H1+ bundle, they do not appear to play any major role in the octamer assembly, are appear to contribute to water-soluble only. However, the role of the Glu^{u} residues in the octameric helical bundle may be critical, if indirect: the positions of the Glu^{u}₇ side-chains within the hexameric helical bundle preclude the possibility of the Glu^{u} side-chains being charged, hence no salt-bridges can be formed in the hexameric bundle. With this energetically unfavourable like-charge clash removed in the **H1**+ helical bundle, the remaining chargeable side-chains of **H1**+ are free to become charged, and hence take part in salt-bridge formation.

### Additional crystal structures of H1+ all reveal octameric helical bundles and reveal ion co-ordination sites

In addition to the crystal structure of **H1**⁺ described above (in space group *P*2₁2₁2₁), crystals of H1+ were obtained in three additional space groups: *P*3₂21, *C*2 and *P*422. Structures determined for each of these crystal forms (to resolutions of x, x and x, respectively) all reveal octameric helical bundle arrangements almost identical to the structure described above. Where resolution allows, intra-helical waters can be seen in the electron density maps of these additional crystal structures, indicating a remarkable conservation of this structural motif. The ability of **H1**⁺ to form the same helical bundle in fundamentally different space groups is significant, and gives one confidence that the octameric arrangement for **H1**⁺ described herein is not an artefact of crystal packing effects.

In addition, the crystal structure of **H1**⁺ in space group P422 reveals two chloride ions coordinated within the octameric bundle - each one octahedrally bound by eight free N-terminal NHs of four helices. These chlorides effectively 'close' the internal cavity, and also appear to stabilise the N-terminal regions in addition to the stability afforded by the salt-bridges (as indicated by B-factor comparison). The fact that chloride coordination is observed in just one of the four octameric crystal structures reported here suggests that ion binding is permissable but non-essential for folding. Anion binding properties have recently been reported for a series neutral oligourea foldamers in organic-solvent conditions. ¹H-NMR titration studies in organic solvents (MeCN, MeCN/DMSO) revealed that the interaction between aliphatic oligoureas and anions (oxyanions > Cl⁻) was site specific by involving the urea NHs localized at the positive pole of the helix that do not participate to the intramolecular hydrogen bonding network. (ref)-our results here suggest that ion coordination by oligoureas may well be possible in aqueous conditions, and offers enticing possibilities with respect to guest coordination. This mode of binding parallels that found in proteins where anion binding sites are often located at the amino terminus of an α-helix where the interaction with the positive pole of the helix dipole contributes to anion stabilization in addition to contacts with main chain amide NHs (and some polar side chains) (Copley, 1994 #3279; Dutzler, 2002 #723).

### METHODS

Data Deposition. Atomic coordinates and structure factors have been deposited in the Cambridge Crystallographic Data Centre with accession codes: 1030455, 1030456, 1030457 and 1030454, for structures **H1**, **H2**, **H4** and **H5**, respectively, and can be obtained free of charge upon request (www.ccdc.cam.ac.uk/data_request/).

**Chemistry.** Oligoureas **H1**, **H3**, **H4** and **H5** were synthesized on solid support using azide-protected succinimidyl carbamate building blocks following previously reported procedures (Douat-Casassus, C., et al. Microwave-enhanced solid-phase synthesis of N,N'-linked aliphatic oligoureas and related hybrids. Org. Lett. 14, 3130-3133 (2012)). **H2** was assembled in solution using Boc-protected succinimidyl carbamate building blocks and a fragment condensation approach (Fremaux, J., et al. Condensation approach to aliphatic oligourea foldamers: helices with N-(pyrrolidin-2-ylmethyl)ureido junctions. Angew. Chem. Int. Ed Engl. 50, 11382-11385 (2011)). Full details of chemical synthesis and purification can be found in the supplementary information.

**Circular dichroism.** Circular dichroism (CD) experiments were performed on a Jasco J-815 spectrometer. Variable concentration experiments were performed on oligoureas **H1-5** in double-distilled H₂O (ddH₂O) as well as buffered conditions (20 mM sodium acetate at pH 4.0 for **H1** and **H3** and 20 mM sodium phosphate at pH 7.4 for **H2**) starting from oligourea concentrations of 200 µM followed by serial two-fold dilutions. Data were recorded at 20°C between wavelengths of 180 and 250 nm at 0.5 nm intervals at a speed of 50 nm/min with an integration time of 2 seconds. CD-monitored thermal melting experiments were performed on oligoureas **Hl-5** in pure water (and **H1** in 20 mM sodium acetate at pH 4.0) at a concentration of 200 µM. For these experiments, oligourea samples were heated from 5°C to 90°C using a gradient of 1°C.min⁻¹. The CD signal at 202 nm was monitored for these experiments. The Tₘ value for **H1** (in pure water) was determined by fitting CD-monitored thermal melting data to a simple two-state Boltzmann unfolding model using Origin 8.6 (adjusted R-square of fit: 0.99943).

**X-ray crystallography**. For crystallisation trials, lyophilized powders of oligoureas **H1-H5** were dissolved to final concentrations of 10 mg/ml in ddH₂O. Crystallisation trials were performed at 20°C in standard aqueous hanging drops composed of 0.5 µL of oligourea solution plus 0.5 µL of crystallisation reagent. Crystals were obtained for all oligourea molecules except **H3**. X-ray diffraction data were collected at the European Synchrotron Radiation Facility (ESRF) on beam lines ID23-2 (**H1** and **H2**) and ID29 (**H5**) and at the SOLEIL synchrotron on beam line PROXIMA-1 (**H4**). Data were processed using XDS (Kabsch, W. XDS. Acta Crystallogr. D Biol. Crystallogr. 66, 125-132 (2010)) and CCP4 (Winn, M. D. et al. Overview of the CCP4 suite and current developments. Acta Crystallogr. D Biol. Crystallogr. 67, 235-242 (2011)). The structures were solved by molecular replacement using previously reported oligourea crystal structures as search models (Fischer, L. et al. The canonical helix of urea oligomers at atomic resolution: insights into folding-induced axial organization. Angew. Chem. Int. Ed Engl. 49, 1067-1070 (2010); and Fremaux, J., Fischer, L., Arbogast, T., Kauffmann, B. & Guichard, G. Condensation approach to aliphatic oligourea foldamers: helices with N-(pyrrolidin-2-ylmethyl)ureido junctions. Angew. Chem. Int. Ed Engl. 50, 11382-11385 (2011)) using Phaser (McCoy, A. J. et al. Phaser crystallographic software. J. Appl. Crystallogr. 40, 658-674 (2007)) from the CCP4 suite (Winn, M. D. et al. Overview of the CCP4 suite and current developments. Acta Crystallogr. D Biol. Crystallogr. 67, 235-242 (2011)). Model building and restrained refinement were performed in Coot (Emsley, P., Lohkamp, B., Scott, W. G. & Cowtan, K. Features and development of Coot. Acta Crystallogr. D Biol. Crystallogr. 66, 486-501 (2010)) and Refmac5 (Murshudov, G. N. et al. REFMAC5 for the refinement of macromolecular crystal structures. Acta Crystallogr. D Biol. Crystallogr. 67, 355-367 (2011)), respectively. Resolutions and R_{work}/R_{free} factors of final refined models are: 1.25 Å, 15.5 / 22.6 % (**H1**), 1.40 Å, 17.9 / 19.8 % (**H2**), 1.69 Å, 19.3 / 27.7 % **(H4)** and 1.47 Å, 17.6 / 24.4 % **(H5).** Additional crystallisation, data collection and refinement details can be found in the supplementary information.

For crystallisation trials, lyophilized powders of oligoureas **H1-H5** were dissolved to final concentrations of 10 mg/ml in double-distilled H₂O (ddH₂O). Crystallisation trials were performed at 20 °C in standard aqueous hanging drops composed of 0.5 µL of oligourea solution plus 0.5 µL of crystallisation reagent. Crystals were obtained for all oligourea molecules except **H3**. Data collection and refinement statistics for crystal structures of oligoureas **H1, H2, H4** and **H5** can be found in Table S1. Further details of crystallisation, data collection and refinement are given below.

Crystals of oligourea **H1** were grown using a crystallisation reagent composed of 20 % isopropanol, 200 mM calcium chloride and 100 mM sodium acetate buffer (pH 4.6). For data collection, a single crystal was cryo-protected in a solution composed of 25 % glycerol, 13.3 % isopropanol, 133 mM calcium chloride and 67 mM sodium acetate (pH 4.6) and flash frozen in liquid nitrogen. X-ray diffraction data were collected on beam line ID23-2 at the European Synchrotron Radiation Facility (ESRF), and processed using XDS (Kabsch, W. XDS. Acta Crystallogr. D Biol. Crystallogr. 66, 125-132 (2010)) and CCP4 (Winn, M. D. et al. Overview of the CCP4 suite and current developments. Acta Crystallogr. D Biol. Crystallogr. 67, 235-242 (2011)). The structure was solved by molecular replacement using a previously reported oligourea crystal structure as a search model (CCDC code: 750017; Fischer, L. et al. The canonical helix of urea oligomers at atomic resolution: insights into folding-induced axial organization. Angew. Chem. Int. Ed Engl. 49, 1067-1070 (2010)), using Phaser (McCoy, A. J. et al. Phaser crystallographic software. J. Appl. Crystallogr. 40, 658-674 (2007)) from the CCP4 suite (Winn, M. D. et al. Overview of the CCP4 suite and current developments. Acta Crystallogr. D Biol. Crystallogr. 67, 235-242 (2011)). Geometric restraints for maximum likelihood restrained refinement were generated using the PRODRG server (Schüttelkopf, A. W. & van Aalten, D. M. F. PRODRG: a tool for high-throughput crystallography of protein-ligand complexes. Acta Crystallogr. D Biol. Crystallogr. 60, 1355-1363 (2004)). Model building and restrained refinement were performed in Coot (Emsley, P., Lohkamp, B., Scott, W. G. & Cowtan, K. Features and development of Coot. Acta Crystallogr. D Biol. Crystallogr. 66, 486-501 (2010)) and Refmac5 (Murshudov, G. N. et al. REFMAC5 for the refinement of macromolecular crystal structures. Acta Crystallogr. D Biol. Crystallogr. 67, 355-367 (2011)), respectively. Twinning was detected using Xtriage from the Phenix software suite (Adams, P. D. et al. PHENIX: a comprehensive Python-based system for macromolecular structure solution. Acta Crystallogr. D Biol. Crystallogr. 66, 213-221 (2010)), and was subsequently accounted for during the refinement process. The data are hemihedrally twinned, with relative twin fractions of 0.508 and 0.492 for domains defined by operators H, K, L and K, H, -L, respectively. The final model was refined (including anisotropic displacement parameters) to a resolution of 1.25 Å, with R_{work} and R_{free} factors of 15.5 % and 22.6 % respectively.

Oligourea **H2** crystallized in a surprisingly large number of different crystallisation conditions - many of which differed considerably in pH, precipitant and ion content - yet all crystals obtained **of H2** were isomorphous (i.e. they were all of the same space group and unit cell). The structure reported here was solved from diffraction data collected on a crystal grown from a crystallisation reagent composed of 30 % 2-methyl-2,4-pentanediol, 200 mM sodium chloride and 100 mM sodium acetate buffer (pH 4.6). This crystal/dataset was selected purely on the basis of resolution. The crystal was flash frozen directly in liquid nitrogen and diffraction data collected on beam line ID23-2 at the ESRF. Diffraction data were processed as above. The structure was solved by molecular replacement using a previously reported pyrrolidine-containing oligourea crystal structure as a search model (CCDC code: 836811; Fremaux, J., Fischer, L., Arbogast, T., Kauffmann, B. & Guichard, G. Condensation approach to aliphatic oligourea foldamers: helices with N-(pyrrolidin-2-ylmethyl)ureido junctions. Angew. Chem. Int. Ed Engl. 50, 11382-11385 (2011)) using Phaser (McCoy, A. J. et al. Phaser crystallographic software. J. Appl. Crystallogr. 40, 658-674 (2007)) from the CCP4 suite (Winn, M. D. et al. Overview of the CCP4 suite and current developments. Acta Crystallogr. D Biol. Crystallogr. 67, 235-242 (2011)). Model building and restrained refinement were performed in Coot (Emsley, P., Lohkamp, B., Scott, W. G. & Cowtan, K. Features and development of Coot. Acta Crystallogr. D Biol. Crystallogr. 66, 486-501 (2010)) and Refmac5 (Murshudov, G. N. et al. REFMAC5 for the refinement of macromolecular crystal structures. Acta Crystallogr. D Biol. Crystallogr. 67, 355-367 (2011)), respectively. The final model was refined (including anisotropic displacement parameters) to a resolution of 1.40 Å, with R_{work} and R_{free} factors of 17.9 % and 19.8 %, respectively.

Crystals of oligourea **H4** were grown using a crystallisation reagent composed of 15 % isopropanol, 1 M sodium chloride, 200 mM calcium chloride and 100 mM sodium acetate buffer (pH 4.6). Data were collected on beam line PROXIMA 1 at SOLEIL Synchrotron and diffraction data processed in XDS and CCP4. The structure was solved by molecular replacement using a truncated version of a single helix from the crystal structure of **H1** as a search model (using Phaser; McCoy, A. J. et al. Phaser crystallographic software. J. Appl. Crystallogr. 40, 658-674 (2007)). Model building and restrained refinement were performed as described for **H1.** Twinning was detected using Xtriage from the Phenix software suite (Adams, P. D. et al. PHENIX: a comprehensive Python-based system for macromolecular structure solution. Acta Crystallogr. D Biol. Crystallogr. 66, 213-221 (2010)), and was subsequently accounted for during the refinement process. The data are hemihedrally twinned, with relative twin fractions of 0.513 and 0.487 for domains defined by operators H, K, L and K, H, -L, respectively. The final model was refined (including isotropic displacement parameters) to a resolution of 1.69 Å, with R_{work} and R_{free} factors of 19.3 and 27.7, respectively.

Crystals of oligourea **H5** were grown using a crystallisation reagent composed of 20 % Jeffamine M600 and 100 mM sodium HEPES buffer (pH 7.5). Crystals were cryo-protected in a solution composed of 25 % glycerol, 13.3 % Jeffamine M600 plus 67 mM sodium HEPES buffer (pH 7.5) and flash frozen in liquid nitrogen. Diffraction data were collected on beam line ID29 at the ESRF, and processed as above. The structure was solved by molecular replacement using a single modified helix from the crystal structure of **H1** as a search model, using Phaser (McCoy, A. J. et al. Phaser crystallographic software. J. Appl. Crystallogr. 40, 658-674 (2007).) from the CCP4 suite (Winn, M. D. et al. Overview of the CCP4 suite and current developments. Acta Crystallogr. D Biol. Crystallogr. 67, 235-242 (2011)). Model building and restrained refinement were performed as described for oligoureas **H1, H2** and **H4.** The final model was refined (including anisotropic displacement parameters) to a resolution of 1.47 Å, with R_{work} and R_{free} factors of 17.6 % and 24.4 %, respectively.

Atomic coordinates and structure factors for all four crystal structures have been deposited in the Cambridge Crystallographic Data Centre (CCDC) with accession codes 1030455, 1030456, 1030457 and 1030454 for structures **H1, H2, H4** and **H5**, respectively (see Table S1). These data are available free of charge upon request (www.ccdc.cam.ac.uk/).

Cavity analysis for structures **H1** and **H4** was performed using SURFNET (Laskowski, R. A. SURFNET: a program for visualizing molecular surfaces, cavities, and intermolecular interactions. J. Mol. Graph. 13, 323-330 (1995)), using a 1.4 Å probe radius. All figures were prepared using PyMOL (DeLano, W. L. The PyMOL Molecular Graphics System. (DeLano Scientific, San Carlos, CA, USA, 2002)).

**Native electrospray ionization mass spectrometry.** Native electrospray ionization mass spectrometry (ESI-MS) experiments were performed on an Agilent 6560 DTIMS-Q-TOF spectrometer (Agilent Technologies, Santa Clara, CA), with the dual-ESI source operated in positive ion mode. Oligoureas **H1-H5** were analyzed at a concentration 100 µM in 20 mM ammonium acetate, with a syringe pump flow rate of 180 µL/h. The trapping funnel RF amplitude was set to 150 V to improve softness. The data were analyzed using the Agilent MassHunter software (version B.07).

**NMR spectroscopy.** Spectra were recorded on a Bruker Avance 700 MHz or 800 MHz spectrometer equipped with a standard or cryogenic triple resonance gradient probe, respectively. Samples were measured at a concentration of 200 µM in 450 uL water with 10 % or 100 % D₂O, at both pH 4.0 and 5.5, and in 98 % D₂O plus 20 mM sodium acetate at pH 4.0, at a temperature of 293 K. 1D spectra used a 3-9-19 WATERGATE sequence for suppression of the water signal. Assignment of proton chemical shifts used 2D 1H,1H-TOCSY spectra collected in D₂O with and without 20 mM sodium acetate (pH 4.0) with a mixing time of 80 ms and a sweepwidth of 8000 Hz in both dimensions with 1024 x 180 complex points, and centered on the water signal. Side-chain assignments were supported by natural abundance 2D 1H,13C-HSQC spectra collected with 512 x 128 complex points using a 13C sweepwidth of 16000 Hz centered on 40 ppm. Structure characterization used 2D 1H,1H-NOESY spectra with a mixing time of 200 ms and a sweepwidth of 8000 Hz in both dimensions centered on the water signal, and using 1024 x 256, 1024 x 160 or 1024 x 128 complex points. NMR spectra were collected with the software Topspin 2.1 or 3.2 and processed with NMRPipe/Draw⁴⁷. Chemical shift assignment and figure preparation used Sparky 3 (T. D. Goddard & D. G. Kneller, University of California, San Francisco, USA). Further details of experimental NMR conditions can be found in the appropriate figure legends.

**Electron microscopy imaging.** For negative staining electron microscopy (EM) studies, a 5 µl sample of **H2** at a concentration of 200 µM (in 50 mM sodium HEPES buffer, pH 7.5) was deposited for 2 minutes on carbon copper grids submitted to a glow discharge (Elmo, Cordouan technologies). After a brief wash using distilled water, they were stained with 2 % uranyl acetate and dried with filter paper. Grids were observed with a FEI Tecnai F20 electron microscope. For cryo-EM studies, the sample was deposited onto a lacey carbon glow discharged copper grids. After removing the excess of solution with filter paper, grids were rapidly plunged into a liquid ethane bath cooled with liquid nitrogen using EM GP (Leica). Specimens were observed at -170 °C using a cryo holder (Gatan, USA), with a FEI Tecnai F20 electron microscope operating at 200 kV under low-dose conditions. Images were acquired with a USC1000 2k x 2k Gatan camera.

**Table 1. Data collection and refinement statistics for X-ray crystal structures of oligoureas H1, H2, H4 and H5.**

| **Oligourea** | **H1** | **H2** | **H4** | **H5** |
|---|---|---|---|---|
| Sequence | LuEuKuLuYuLuEuKuLuAu Lu | LuEuLuKuPuLuEuLuKu Au | SuEuKuLuYuLuEuKuLuAu Lu | AuLuKuLuEuYuLuEuLuKuAu Lu |
| Data Collection | | | | |
| Space group | *P* 6₃ | *P* 6₁22 | *P* 6₃ | *P* 622 |
| a, b, c (Å) | 34.00, 34.00, 37.69 | 38.12, 38.12, 54.98 | 33.52,33.2, 38.02 | 50.36, 50.36, 42.87 |
| α, β, γ (°) | 90.0, 90.0, 120.0 | 90.0, 90.0, 120.0 | 90.0, 90.0, 120.0 | 90.0, 90.0, 120.0 |
| Resolution (A) | 37.69 - 1.25 (1.32 - 1.25) | 33.01 - 1.40 (1.48 - 1.40) | 29.03 - 1.69 (1.79 - 1.69) | 43.62 - 1.47 (1.56 - 1.47) |
| Rₘₑₐₛ (%) | 4.7 (67.7) | 83 (60.5) | 6.7 (66.0) | 43 (62.9) |
| I / σ | 28.86 (4.01) | 24.77 (6.16) | 11.22 (2.24) | 37.77 (4.62) |
| Reflections (total) | 72787 | 72322 | 8749 | 114408 |
| Reflections (unique) | 6938 | 5060 | 2709 | 5847 |
| Completenes s (%) | 99.7 (99.5) | 99.7 (99.5) | 97.8 (93.8) | 99.8 (98.6) |
| Redundancy | 10.5 (10.2) | 14.3 (14.4) | 3.2 (3.1) | 19.6(18.2) |
| Refinement | | | | |
| Resolution (Å) | 8.70 - 1.25 | 19.06 - 1.40 | 29.03 - 1.69 | 43.62 - 1.47 |
| R_{work} / R_{free} (%) | 15.5 / 22.6 | 17.9 / 19.8 | 193 / 27.7 | 17.6 / 24.4 |
| Atoms | 279 | 246 | 259 | 295 |
| Waters | 30 | 18 | 18 | 37 |
| Overall B-factor (Å²) | 13.93 | 14.89 | 29.86 | 26.92 |
| R.m.s. deviations | | | | |
| Bond-lengths (Å) | 0.015 | 0.010 | 0.017 | 0.015 |
| Bond-angles (°) | 1.737 | 2.020 | 2.184 | 1.688 |
| CCDC code | 1030455 | 1030456 | 1030457 | 1030454 |

| | | | | |
|---|---|---|---|---|
| Values in brackets refer to highest resolution shells. R_{work}: ∑\|Fₒ - F_{c}\|/ ∑Fₒ. R_{free}: R-factor calculated for 5 % of the data. Rₘₑₐₛ: redundancy independent R-factor¹⁵. | | | | |

### SYNTHESIS OF EXEMPLARY OLIGOUREAS

Two different strategies have been adopted to synthesize the oligoureas studied in this work: compound **H2** was assembled in solution, using Boc-protected succinimidyl carbamate building blocks and a fragment condensation approach (Fremaux, J., et al.. Condensation approach to aliphatic oligourea foldamers: helices with N-(pyrrolidin-2-ylmethyl)ureido junctions. Angew. Chem. Int. Ed Engl. 50, 11382-11385 (2011)); whereas compounds **H1, H3, H4** and **H5** were prepared by microwave assisted solid phase synthesis, starting from N₃-protected succinimidyl carbamate building blocks (Douat-Casassus, C., et al. Microwave-enhanced solid-phase synthesis of N,N'-linked aliphatic oligoureas and related hybrids. Org. Lett. 14, 3130-3133 (2012)).

### Boc-protected building block synthesis for oligourea synthesis in solution

The building blocks containing Ala- (1), Leu- (2) and Lys-type (3) side chains were synthesized as previously reported (Fremaux, J., et al.. Condensation approach to aliphatic oligourea foldamers: helices with N-(pyrrolidin-2-ylmethyl)ureido junctions. Angew. Chem. Int. Ed Engl. 50, 11382-11385 (2011)). The characterization of the Lys-type building block obtained starting from Boc-L-Lys(2-ClZ)-OH is reported here as an example. Furthermore, the strategy followed to obtain the Glu-type building block **(4)** is also described.

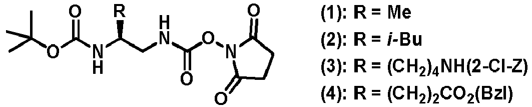

### Boc-Lys: 5-tert-Butoxycarbonylamino-6-(2,5-dioxo-pyrrolidin-1-yloxycarbonylamino)-hexyl]-carbamic acid 2-chloro-benzyl ester (3)

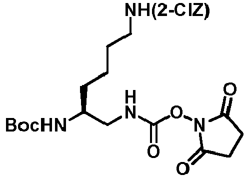

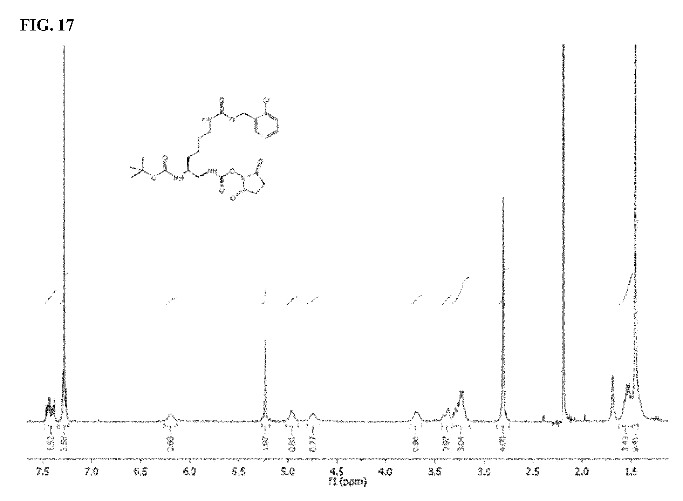

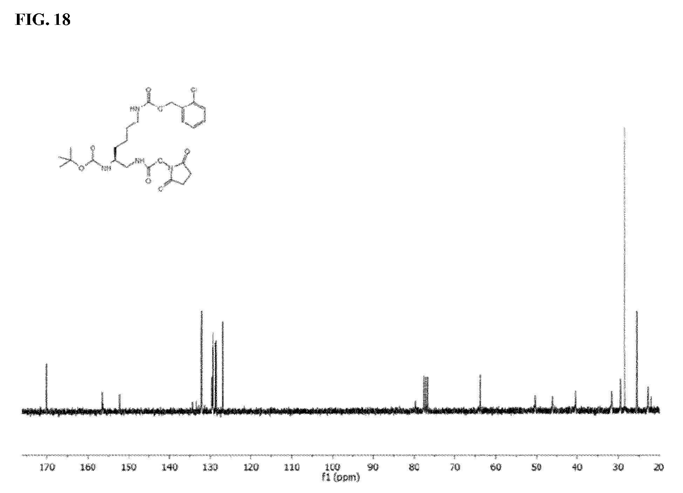

Boc-L-Lys(2-ClZ)-OH (5 g, 20.06 mmol) was transformed according to a procedure previously described (Fremaux, J., et al.. Condensation approach to aliphatic oligourea foldamers: helices with N-(pyrrolidin-2-ylmethyl)ureido junctions. Angew. Chem. Int. Ed Engl. 50, 11382-11385 (2011)). The product (3) was obtained as a white solid after recrystallization from EtOAc with cyclohexane, with an overall yield of 38 % after 4 steps. ¹H NMR (CDCl₃, 300 MHz) δ: 7.48-7.34 (m, 4H), 6.18 (s, 1H), 5.22 (s, 2H), 4.96 (s, 1H), 4.73 (s, 1H), 3.69 (s, 1H), 3.46-3.15 (m, 4H), 2.80 (s, 4H), 1.64-1.47 (m, 6H) 1.46 (s, 9H); ¹³C NMR (75 MHz, CDCl₃) δ: 170.10, 156.48, 152.23, 132.18, 132.05, 129.73, 129.45, 129.32, 128.65, 128.48, 126.90, 63.81, 50.42, 40.38, 31.54, 29.48, 28.35, 25.46, 22.64; ESI-MS (ESI+) *m*/*z*: 563.19 [M+Na]⁺ (Fig. 17 and 18).

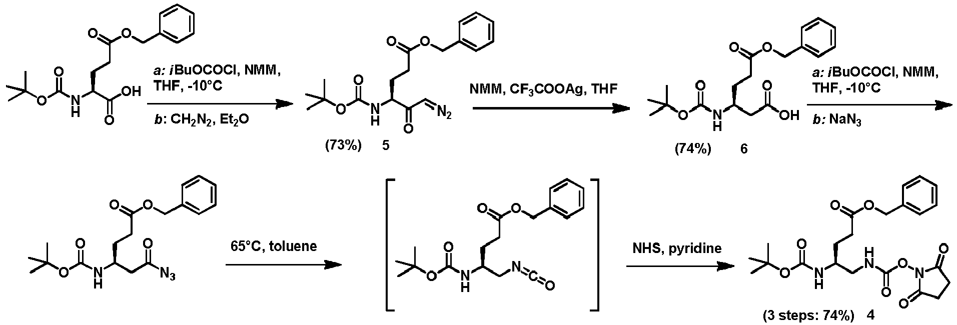

### 4-tert-Butoxycarbonylamino-6-diazo-5-oxo-hexanoic acid benzyl ester (5)

Boc-Glu(OBn)-OH (5.06 g, 15.00 mmol) was dissolved in anhydrous THF (80 mL) under an atmosphere of N₂, and cooled to -10 °C. NMM (2.14 ml, 19.50 mmol) was added, followed by IBCF (2.95 ml, 22.50 mmol). The reaction was stirred at -10°C for 1 h. The white precipitate formed was eliminated by filtration. The filtrate was cooled to 0 °C and freshly prepared diazomethane in diethyl ether was added (2 eq, prepared from Diazald and KOH). The mixture was warmed up to room temperature and stirred overnight. The reaction was then quenched with the addition of acetic acid (1 ml), and the mixture was vigorously stirred (750 rpm) for two hours. An excess of acetic acid was then added (1 ml), and the mixture was then neutralised with a saturated aqueous NaHCO₃ solution (70 ml) and stirred until the effervescence disappeared. The organic phase was separated and washed with 1 N KHSO₄ (aq) (60 ml), brine (60 ml), dried over MgSO₄, and the solvents were evaporated *in vacuo*. The crude obtained was purified by flash chromatography on silica gel (gradient cyclohexane-30% ethyl acetate), to afford the pure product as a yellow solid (3.94 g, 73 %). ¹H NMR (CDCl₃, 300 MHz) δ: 7.46-7.29 (m, 5H), 5.47 (s, 1H), 5.25 (m, 1H), 5.14 (s, 2H), 4.26 (m, 1H), 2.60-2.38 (m, 2H), 2.23-2.10 (m, 1H), 1.93-1.77 (m, 1H), 1.45 (s, 9H).

### 3-tert-Butoxycarbonylamino-hexanedioic acid 6-benzyl ester (6)

The diazoketone **(5)** (3.00 g, 8.30 mmol), was dissolved in a mixture 5:1 of THF/H₂0 (60 ml), and cooled in an ice bath in a flask covered with aluminium foil to protect from the light. Silver trifluoroacetate (0.37 g, 1.66 mmol) was then added, followed by a dropwise addition of NMM (2.28 ml, 20.75 mmol). The reaction mixture was stirred for 4.5 hrs and warmed to room temperature. After completion was reached, THF was evaporated, IN KHSO₄ (aq) (40 ml) was added, and the precipitate observed was removed by filtration. The aqueous solution was extracted with ethyl acetate, and the organic phase was washed with IN KHSO₄ (aq) (twice), dried over MgSO₄, and the solvents were evaporated *in vacuo*. Trituration of the yellow crude oil with diethyl ether gave the desired compound as a white solid (2.17 g, 74 %). ¹H NMR (CDCl₃, 300 MHz) δ: 7.42-7.31 (m, 5H), 5.13 (s, 2H), 4.00 (m, 1H), 4.03-3.90 (m, 1H), 2.70-2.56 (m, 2H), 2.54-2.40 (m, 2H), 1.98-1.85 (m, 2H), 1.45 (s, 9H).

### Boc-Glu: 4-tert-Butoxycarbonylamino-5-(2,5-dioxo-pyrrolidin-1-yloxycarbonylamino)-pentanoic acid benzyl ester (4)

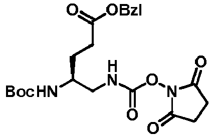

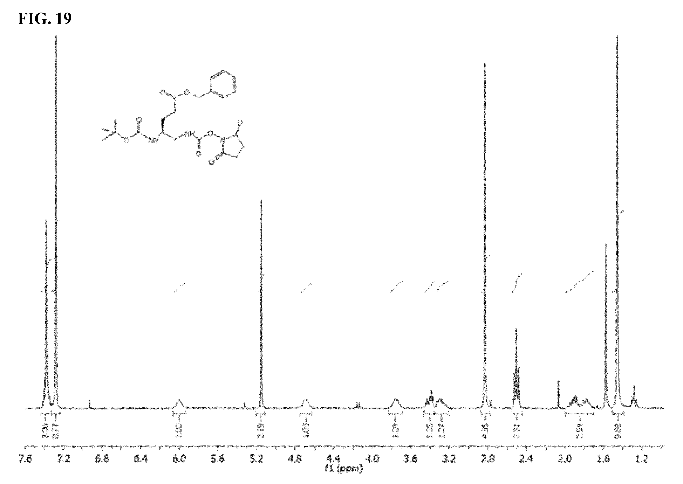

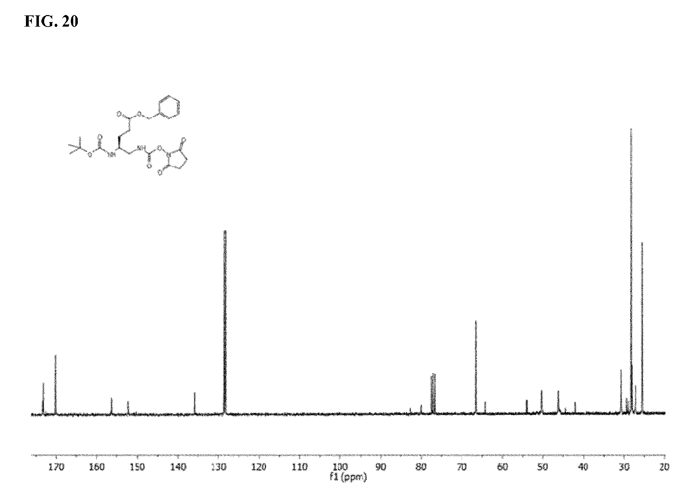

The acid **(6)** (0.64 g, 1.82 mmol) was reacted as previously reported (Fischer, L. et al. Succinimidyl Carbamate Derivatives from N-Protected α-Amino Acids and Dipeptides-Synthesis of Ureidopeptides and Oligourea/Peptide Hybrids. Eur. J. Org. Chem. 2007, 2511-2525 (2007)). The desired product (4) was obtained as a white solid after precipitation from EtOAc with hexane (0.63 g, 74 %). ¹H NMR (CDCl₃, 300 MHz) δ: 7,45-7,33 (m, 5H, CHAr), 6,08 (s, 1H, NH), 5,14 (s, 2H, CH₂Ar), 4,74 (m, 1H, NH), 3,82-3,68 (m, 1H, CHN), 3,45-3,22 (m, 2H, CH₂N), 2,83 (s, 4H, CH₂), 2,55-2.44 (m, 2H, CH₂), 1,98-1,69 (m,2H, CH₂), 1,45 (s, 9H, Boc). ¹³C NMR (CDCl₃, 75 MHz) δ: 173.07, 170.17, 156.41, 152.27, 135.80, 128.59, 128.27, 128.22, 80.00, 66.51, 50.26, 46.27, 30.73, 28.30, 27.17, 25.48; ESI-MS (ESI+) *m*/*z*: 486.20 [M+Na]⁺ (Fig. 19 and 20).

### Synthesis of oligourea H2

The couplings of Boc-protected OSu activated building blocks were performed according to the general procedure previously reported (Fremaux, J., Fischer, L., Arbogast, T., Kauffmann, B. & Guichard, G. Condensation approach to aliphatic oligourea foldamers: helices with N-(pyrrolidin-2-ylmethyl)ureido junctions. Angew. Chem. Int. Ed Engl. 50, 11382-11385 (2011)). The characterization of each intermediate and a scheme of the synthetic strategy adopted are described herein.

### FRAGMENT 1:

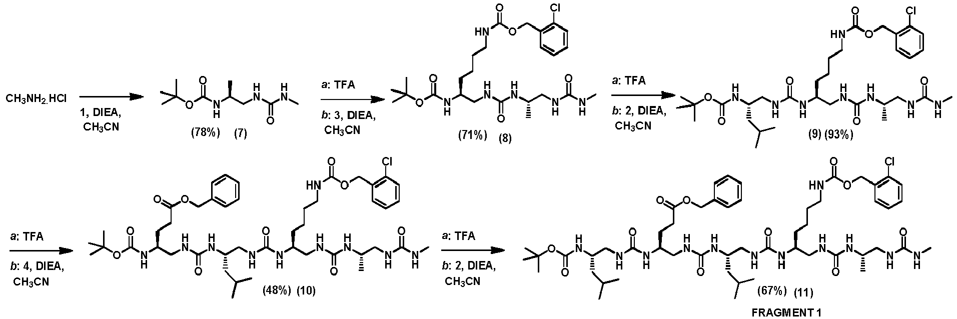

### Boc-Ala^{u}-NHMe (7)

The compound was prepared as previously reported (Fischer, L. et al. The canonical helix of urea oligomers at atomic resolution: insights into folding-induced axial organization. Angew. Chem. Int. Ed Engl. 49, 1067-1070 (2010)) from the Boc-Ala type building block (1) (0.260 g, 3.81 mmol), to afford compound (7) as a white solid (0.531 g, crude yield 90 %) after aqueous work up. ¹H NMR (DMSO d6, 300 MHz) δ: 6.65 (m, 1H, NH), 5.88 (m, 1H, NH), 5.80 (m, 1H, NH), 3.46-3.35 (m, 1H, CHN), 3.05-2.84 (m, 2H, CH₂N), 2.53 (d, 3H, CH₃N), 1.37 (s, 9H, Boc), 0.95 (d, *J* = 6.6 Hz, 3H, CH₃).

### Boc-Lys(2ClZ)^{u}-Ala^{u}-NHMe (8)

Compound **(8)** was prepared from **(3)** (1.38 g, 2.54 mmol) and **(7)** (0.620 g, 2.68 mmol), as described in the general procedure previously reported (Fremaux, J., Fischer, L., Arbogast, T., Kauffmann, B. & Guichard, G. Condensation approach to aliphatic oligourea foldamers: helices with N-(pyrrolidin-2-ylmethyl)ureido junctions. Angew. Chem. Int. Ed Engl. 50, 11382-11385 (2011)). Oligourea **(8)** was obtained as a white solid (1.06 g, 71 %) after purification by silica gel flash chromatography (DCM 9:MeOH 1). ¹H NMR (CD₃CN, 300 MHz) δ: 7.51-7.32 (m, 4H, CH_{Ar}), 5.83 (m, 1H, NH), 5.60 (m, 1H, NH), 5.38-5.25 (m, 2H, NH), 5.18 (s, 2H, CH₂OCO), 5.12-5.02 (m, 2H, NH), 3.74-3.63 (m, 1H, CHN), 3.61-3.50 (m, 1H, CHN), 3.25-3.07 (m, 4H, CH₂N), 3.06-2.91 (m, 2H, CH₂N), 2.66 (d, *J* = 4.7 Hz, 3H, CH₃N), 1.58-1.46

(m, 2H, CH₂), 1.45 (s, 9H, Boc), 1.42-1.29 (m, 4H, CH₂), 1.07 (d, *J* = 6.7 Hz, 3H, CH₃); ESI-MS (ESI+) *m*/*z*: 579.27 [M+Na]⁺, 1134.8 [2M+Na]⁺.

### Boc-Leu^{u}-Lys(2ClZ)^{u}-Ala^{u}-NHMe (9)

Compound **(9)** was prepared from **(2)** (0.64 g, 1.80 mmol) and **(8)** (1.00 g, 1.80 mmol) as described in the general procedure previously reported (Fremaux, J., et al. Condensation approach to aliphatic oligourea foldamers: helices with N-(pyrrolidin-2-ylmethyl)ureido junctions. Angew. Chem. Int. Ed Engl. 50, 11382-11385 (2011)). Oligourea **(9)** was obtained as a white solid (1.25 g, 93 %) after purification by silica gel flash chromatography (DCM 9:MeOH 1). ¹H NMR (CD₃OH, 300 MHz) δ: 7.49-7.29 (m, 4H, CH_{Ar}), 7.12 (m, 1H, NH), 6.53 (d, *J* = 9.6 Hz, 1H, NH), 6.04 (m, 1H, NH), 5.94 (m, 1H, NH), 5.89 (m, 1H, NH), 5.81 (m, 1H, NH), 5.19 (s, 2H, CH₂OCO), 3.92-3.72 (m, 3H, CHN), 3.57-3.40 (m, 2H, CH₂N), 3.20-3.11 (m, 2H, CH₂N), 2.90-2.79 (m, 2H, CH₂N), 2.72 (d, *J* = 3.9 Hz, 3H, CH₃N), 2.68-2.44 (m, 2H, CH₂N), 1.76-1.61 (m, 1H, CH), 1.60-1.51 (m, 2H, CH₂), 1.47 (s, 9H, Boc), 1.44-1.15 (m, 6H, CH₂), 1.08 (d, *J* = 6.7 Hz, 3H, CH₃), 0.99-0.87 (m, 6H, CH₃); ESI-MS (ESI+) *m*/*z*: 699.2 [M+Na]⁺, 721.3 [M+Na]⁺.

### Boc-Glu(OBn)^{u}-Leu^{u}-Lys(2ClZ)^{u}-Ala^{u}-NHMe (10)

Compound **(10)** was prepared from **(4)** (0.86 g, 1.86 mmol) and **(9)** (1.30 g, 1.86 mmol) as described in the general procedure previously reported (Fremaux, J., et al. Condensation approach to aliphatic oligourea foldamers: helices with N-(pyrrolidin-2-ylmethyl)ureido junctions. Angew. Chem. Int. Ed Engl. 50, 11382-11385 (2011)). Oligourea (10) was obtained as a white solid (0.86 g, 48 %) after purification by silica gel flash chromatography (DCM 9:MeOH 1). ¹H NMR (CD₃OH, 300 MHz) δ: 7.51-7.28 (m, 9H, CH_{Ar}), 7.10 (m, 1H, NH), 6.66 (d, *J* = 9.7 Hz, 1H, NH), 6.40 (m, 1H, NH), 6.28 (m, 1H, NH), 6.20 (m, 1H, NH), 6.08-5.99 (m, 2H, NH), 5.93 (m, 1H, NH), 5.82-5.75 (m, 2H, NH), 5.19 (s, 2H, CH₂OCO), 5.13 (s, 2H, CH₂OCO), 4.01-3.66 (m, 4H, CHN), 3.60-3.42 (m, 4H, CH₂N), 3.16-3.07 (m, 2H, CH₂N), 2.86-2.75 (m, 1H, CH₂N), 2.73 (d, *J* = 3.9 Hz, 3H, CH₃N), 2.68-2.51 (m, 2H, CH₂N), 2.49-2.40 (m, 2H, CH₂COO), 2.40-2.32 (m, 1H, CH₂N), 1.91-1.78 (m, 1H, CH), 1.76-1.51 (m, 4H, CH₂), 1.47 (s, 9H, Boc), 1.42-1.16 (m, 6H, CH₂), 1.08 (d, *J* = 6.7 Hz, 3H, CH₃), 0.96-0.88 (m, 6H, CH₃); ESI-MS (ESI+) *m*/*z*: 947.3 [M+H]⁺, 969.4 [M+Na]⁺.\

### Boc-Leu^{u}-Glu(OBz)^{u}-Leu^{u}-Lys(2ClZ)^{u}-Ala^{u}-NHMe (11)

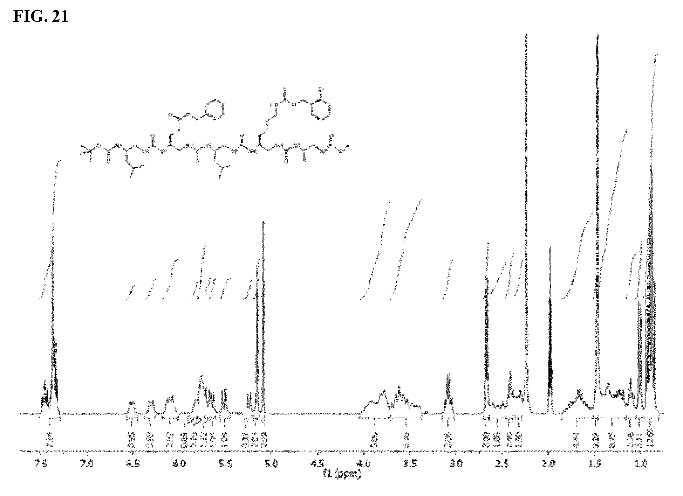

Compound **(11)** was prepared from **(2)** (0.113 g, 0.317 mmol) and **(10)** (0.300 g, 0.317 mmol) as previously described (Fremaux, J., et al. Condensation approach to aliphatic oligourea foldamers: helices with N-(pyrrolidin-2-ylmethyl)ureido junctions. Angew. Chem. Int. Ed Engl. 50, 11382-11385 (2011)). Oligourea **(11)** was obtained as a white solid (0.23 g, 67 %) after purification by silica gel flash chromatography (DCM 9:MeOH 1). ¹H NMR (CD₃OH, 300 MHz) δ: 7.51-7.32 (m, 9H, CH_{Ar}), 6.51 (m, 1H, NH), 6.30 (m, 1H, NH), 6.12 (m, 1H, NH), 6.07 (m, 1H, NH), 5.82 (m, 1H, NH), 5.80-5.72 (m, 3H, NH), 5.69 (d*, J* = 10.6 Hz, 1H, NH), 5.64 (d, *J* = 10.4 Hz, 1H, NH), 5.52 (d, *J* = 9.9 Hz, 1H, NH), 5.24 (d, *J* = 10.1 Hz, 1H, NH), 5.16 (s, 2H, CH₂OCO), 5.09 (s, 2H, CH₂OCO), 4.04-3.72 (m, 5H, CHN), 3.70-3.38 (m, 5H, CH₂N), 3.13-3.05 (m, 2H, CH₂N), 2.67 (d, *J* = 4.7 Hz, 3H, CH₃N), 2.65-2.46 (m, 2H, CH₂N), 2.45-2.37 (m, 2H, CH₂COO), 2.37-2.28 (m, 3H, CH₂N), 1.83-1.53 (m, 4H, CH-CH₂), 1.46 (s, 9H, Boc), 1.44-1.16 (m, 8H, CH₂), 1.15-1.07 (m, 2H, CH₂), 1.01 (d, *J* = 6.7 Hz, 3H, CH₃), 0.95-0.84 (m, 12H, CH₃); ESI-MS (ESI+) *m*/*z*: 1089.5 [M+H]⁺, 1111.5 [M+Na]⁺ (Fig. 21).

### FRAGMENT 2:

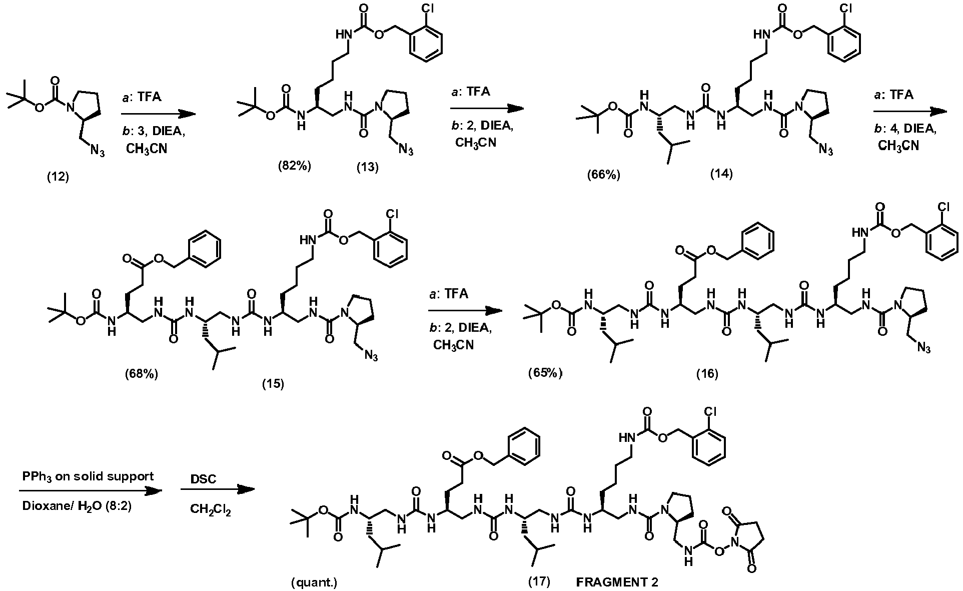

### Boc Lys(2ClZ)^{u}-Pro^{u}-N₃ (13)

Compound **(13)** was prepared from **(3)** (0.43 g, 2.65 mmol) and **(12)** (0.600 g, 2.65 mmol) as previously described (Fremaux, J., et al. Condensation approach to aliphatic oligourea foldamers: helices with N-(pyrrolidin-2-ylmethyl)ureido junctions. Angew. Chem. Int. Ed Engl. 50, 11382-11385 (2011)). Oligourea **(13)** was obtained as a white solid (1.2 g, 82 %) after purification by silica gel flash chromatography (DCM 9 : MeOH 1). The synthesis of compound **(12)** was also previously reported (Fremaux, J., et al. Condensation approach to aliphatic oligourea foldamers: helices with N-(pyrrolidin-2-ylmethyl)ureido junctions. Angew. Chem. Int. Ed Engl. 50, 11382-11385 (2011)). ¹H NMR (CD₃CN, 300 MHz) δ: 7.74-7.33 (m, 4H, CH_{Ar}), 5.82 (m, 1H, NH), 5.51 (s, 1H, NH), 5.32 (m, 1H, NH), 5.17 (s, 2H, CH₂OCO), 4.04-3.97 (m, 1H, CHN), 3.59-3.52 (m, 1H, CHN), 3.48 (dd, *J* = 12.1, 6.5 Hz, 1H, CH₂N), 3.37 (dd, *J* = 12.1, 3.8 Hz, 1H, CH₂N), 3.33-3.23 (m, 2H, CH₂N), 3.21-3.09 (m, 4H, CH₂N), 2.03-1.78 (m, 4H, CH₂), 1.56-1.45 (m, 3H, CH₂), 1.43 (s, 9H, Boc), 1.41-1.30 (m, 3H, CH₂); ESI-MS (ESI+) *m*/*z*: 279.09 [M+2H]²⁺, 574.25 [M+Na]⁺.

### Boc Leu-Lys(2ClZ)^{u}-Pro^{u}-N₃ (14)

Compound **(14)** was prepared from **(2)** (0.77 g, 2.17 mmol) and **(13)** (1.20 g, 2.17 mmol) as previously described (Fremaux, J., et al. Condensation approach to aliphatic oligourea foldamers: helices with N-(pyrrolidin-2-ylmethyl)ureido junctions. Angew. Chem. Int. Ed Engl. 50, 11382-11385 (2011)). Oligourea **(14)** was obtained as a white solid (1.00 g, 66 %) after purification by silica gel flash chromatography (DCM 9:MeOH 1). ¹H NMR (CD₃CN, 300 MHz) δ: 7.53-7.35 (m, 4H, CH_{Ar}), 5.87 (m, 1H, NH), 5.68-5.53 (m, 2H, NH), 5.39 (m, 1H, NH), 5.18 (s, 2H, CH₂OCO), 5.12 (m, 1H, NH), 4.07-3.98 (m, 1H, CHN), 3.70-3.56 (m, 2H, CHN), 3.48 (dd, *J* = 12.1, 6.6 Hz, 1H, CH₂N), 3.38 (dd, *J* = 12.1, 3.8 Hz, 1H, CH₂N), 3.34-3.26 (m, 2H, CH₂N), 3.21-2.99 (m, 6H, CH₂N), 1.96-1.77 (m, 4H, CH₂), 1.72-1.47 (m, 4H, CH₂), 1.43 (s, 9H, Boc), 1.40-1.20 (m, 5H, CH₂), 0.95-0.88 (m, 6H, CH₃); ESI-MS (ESI+) *m*/*z*: 694.38 [M+H]⁺, 716.36 [M+Na]⁺.

### Boc Lys(2ClZ)^{u}-Leu-Lys(2ClZ)^{u}-Pro^{u}-N₃ (15)

Compound **(15)** was prepared from **(4)** (0.63 g, 1.36 mmol) and **(14)** (0.95 g, 1.36 mmol) as previously described (Fremaux, J., et al. Condensation approach to aliphatic oligourea foldamers: helices with N-(pyrrolidin-2-ylmethyl)ureido junctions. Angew. Chem. Int. Ed Engl. 50, 11382-11385 (2011)). Oligourea **(15)** was obtained as a white solid (0.88 g, 68 %) after purification by silica gel flash chromatography (DCM 9:MeOH 1). ¹H NMR (CD₃CN, 300 MHz) δ: 7.51-7.32 (m, 9H, CH_{Ar}), 6.00-5.88 (m, 2H, NH), 5.67 (m, 1H, NH), 5.54-5.38 (m, 3H, NH), 5.16 (s, 2H, CH₂OCO), 5.11 (s, 2H, CH₂OCO), 4.06-4.00 (m, 1H, CHN), 3.86-3.61 (m, 3H, CHN), 3.51-3.24 (m, 7H, CH₂N), 3.15-3.07 (m, 2H, CH₂N), 2.96-2.80 (m, 2H, CH₂N),2.72-2.57 (m, 1H, CH₂N), 2.45-2.38 (m, 2H, CH₂COO), 1.95-1.73 (m, 4H, CH₂), 1.73-1.59 (m, 2H, CH₂), 1.52-1.45 (m, 3H, CH-CH₂), 1.44 (s, 9H, Boc), 1.39-1.29 (m, 4H, CH₂),1.28-1.17 (m, 2H, CH₂), 0.96-0.85 (m, 6H, CH₃); ESI-MS (ESI+) *m*/*z*: 942.49 [M+H]⁺, 980.45 [M+Na]⁺.

### Boc Leu-Lys(2ClZ)^{u}-Leu-Lys(2ClZ)^{u}-Pro^{u}-N₃ (16)

Compound **(16)** was prepared from **(2)** (0.32 g, 0.90 mmol) and **(15)** (0.85 g, 0.90 mmol as previously described (Fremaux, J., et al. Condensation approach to aliphatic oligourea foldamers: helices with N-(pyrrolidin-2-ylmethyl)ureido junctions. Angew. Chem. Int. Ed Engl. 50, 11382-11385 (2011)). Oligourea **(16)** was obtained as a white solid (0.64 g, 65 %) after purification by silica gel flash chromatography (DCM 9:MeOH 1). ¹H NMR (CD₃CN, 300 MHz) δ: 7.50-7.33 (m, 9H, CH_{Ar}), 6.12 (m, 1H, NH), 5.99-5.87 (m, 2H, NH), 5.75 (d, *J* = 8.7 Hz, 1H, NH), 5.57-5.48 (m, 2H, NH), 5.43-5.37 (m, 2H, NH), 5.17 (s, 2H, CH₂OCO), 5.10 (s, 2H, CH₂OCO), 4.97 (d, *J* = 10.3 Hz, 1H, NH), 4.08-4.02 (m, 1H, CHN), 3.95-3.74 (m, 3H, CHN), 3.73-3.50 (m, 4H, CHN-CH₂N), 3.49-3.29 (m, 5H, CH₂N), 3.14-3.06 (m, 2H, CH₂N), 2.85-2.75 (m, 1H, CH₂N), 2.58-2.45 (m, 1H, CH₂N), 2.44-2.36 (m, 2H, CH₂COO), 2.37-2.28 (m, 2H, CH₂N), 1.93-1.53 (m, 8H, CH-CH₂), 1.45 (s, 9H, Boc), 1.43-1.10 (m, 10H, CH₂), 0.95-0.86 (m, 12H, CH₃); ESI-MS (ESI+) *m*/*z*: 1084.4 [M+H]⁺, 1106.5 [M+Na]⁺.

### Boc Leu^{u}-Glu(OBn)^{u}-Leu^{u}-Lys(2ClZ)^{u}-Pro^{u}-NHOSu (17)

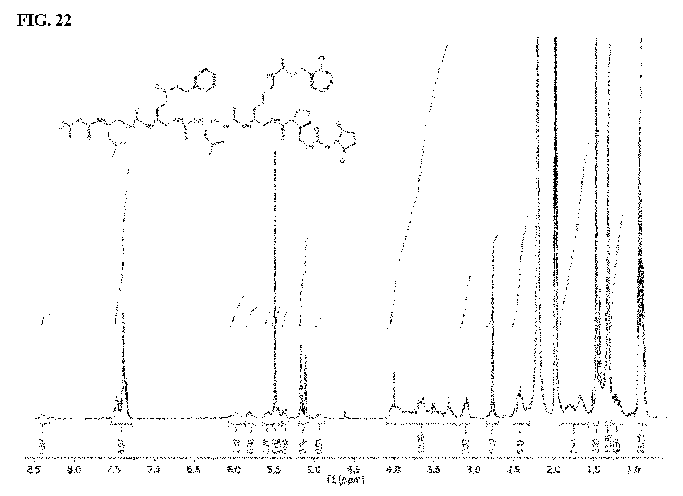

Compound (17) (0.30 g, 0.260 mmol) was dissolved in a mixture 8:2 dioxane/H₂O (8 ml). PL-TPP resin (1.5 mmol/g, 0.200 g) was added and the mixture was stirred under microwave irradiation (50W, 50°C, CEM Discover) for 2 h. After completion, the resin was removed by filtration, washed with dioxane and H₂O, and the filtrate was evaporated *in vacuo*. The amine obtained was used without further purification. Disuccinimidyl carbonate (0.080 g, 0.31 mmol) was suspended in dry DCM (10 mL) and a DCM solution of the amine was added portion wise. The reaction mixture was stirred at room temperature under a N₂ atmosphere for 4 hr. After that time, the white precipitate was eliminated by filtration and washed with DCM. The filtrate was washed with IN KHSO₄ (aq), water, dried over Na₂SO₄, and concentrated under reduced pressure. The pure product **(17)** was precipitated from concentrated DCM solution as a white solid by adding Et₂O (0.340 g, quantitative). ¹H NMR (CD₃CN, 300 MHz) δ: 8.40 (s, 1H, NHOSu), 7.51-7.31 (m, 9H, CH_{Ar}), 6.09-5.89 (m, 2H, NH), 5.80 (m, 1H, NH), 5.57 (m, 1H, NH), 5.46 (m, 1H, NH),5.37 (d, J = 10.2 Hz, 1H, NH) 5.16 (s, 2H, CH₂OCO), 5.11 (s, 2H, CH₂OCO), 4.93 (m, 1H, NH), 4.07-3.21 (m, 14H, CHN-CH₂N), 3.18-3.04 (m, 2H, CH₂N), 2.76 (s, 4H, CH₂), 2.55-2.27 (m, 6H, CH₂N), 1.95-1.50 (m, 8H, CH-CH₂), 1.46 (s, 9H, Boc), 1.42-1.11 (m, 10H, CH₂), 0.98-0.83 (m, 12H, CH₃); ESI-MS (ESI+) *m*/*z*: 1199.4 [M+H]⁺, 1221.6 [M+Na]⁺ (Fig. 22).

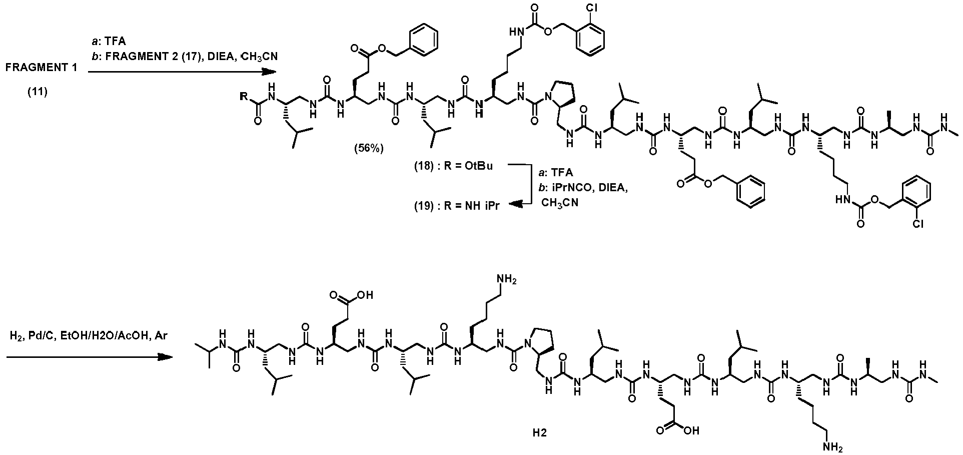

### Boc-Leu^{u}-Glu(OBn)^{u}-Leu^{u}-Lys(2ClZ)^{u}-Pro^{u}-Leu^{u}-Glu(OBn)^{u}-Leu^{u}-Lys(2ClZ)^{u}-Ala^{u}-NHMe (18)

Compound **(11)** (0.130 g, 0.119 mmol) was dissolved in TFA (3 ml) and stirred for 45 min at room temperature. After Boc deprotection was completed, the mixture was concentrated under reduced pressure, co-evaporated with cyclohexane. The crude product was then dissolved in DMF (3 ml), then DIEA (0.06 ml, 0.36 mmol) and **(17)** (0.143 g, 0.119 mmol) were added, and the reaction mixture was then stirred under microwave irradiation (50°C, 25W) for 3 hrs. After completion, the solvent was evaporated *in vacuo*, the crude was dissolved in EtOAc and washed with saturated aqueous NaHCO₃, IN KHSO₄ (aq) and brine. The organic layer was then dried over Na₂SO₄ and the solvents were evaporated *in vacuo*. The crude product was purified by silica gel flash chromatography (DCM 9:MeOH 1) to afford compound **(18)** as a white solid (0.140 g, 56 %). ¹H NMR (CD₃CN, 300 MHz) δ: 7.50-7.31 (m, 18H, CH_{Ar}), 7.13 (m, 1H, NH), 6.90 (m, 1H, NH), 6.76 (m, 1H, NH), 6.62 (m, 1H, NH), 6.48-6.30 (m, 3H, NH), 6.10-5.84 (m, 8H, NH), 5.80-5.70 (m, 2H, NH), 5.68-5.58 (m, 2H, NH), 5.54-5.51 (m, 1H, NH), 5.37 (d, *J* = 10.0 Hz, 1H, NH), 5.16 (s, 2H, CH₂OCO), 5.14 (s, 2H, CH₂OCO), 5.10 (s, 2H, CH₂OCO), 5.9 (s, 2H, CH₂OCO), 4.93 (d, *J* = 10.3 Hz, 1H, NH), 4.33-4.24 (m, 1H, CHN), 4.09-3.40 (m, 19H, CHN-CH₂N), 3.30-3.19 (m, 2H, CH₂N), 3.13-2.92 (m, 4H, CH₂N), 2.84-2.71 (m, 1H, CH₂N), 2.67 (d, *J* = 4.6 Hz, 3H, CH₃N), 2.65-2.53 (m, 2H, CH₂N), 2.52-2.20 (m, 12H, CH₂N-CH₂COO), 1.95-1.50 (m, 16H, CH-CH₂), 1.48 (s, 9H, Boc), 1.43-1.05 (m, 16H, CH₂),1.01 (d*, J* = 6.7 Hz, 3H, CH₃), 0.95-0.82 (m, 24H, CH₃); ESI-MS (ESI+) *m*/*z:* 1060.4 [M+2Na]²⁺.

### iPr-NHCO-Leu^{u}-Glu(OBn)^{u}-Leu^{u}-Lys(2ClZ)^{u}-Pro^{u}-Leu^{u}-Glu(OBn)^{u}-Leu^{u}-Lys(2ClZ)^{u}-Ala^{u}-NHMe (19)

Boc-protected oligourea **(18)** (0.065 g, 0.031 mmol) was dissolved in TFA (3 ml) and stirred for 45 min. After Boc deprotection was completed, the mixture was concentrated under reduced pressure, and TFA was co-evaporated with cyclohexane. The crude product was then dissolved in a CH₃CN/DMF mixture (4:1, 2.5 ml), then DIEA (0.016 ml, 0.094 mmol) and isopropyl isocyanate (0.005 ml, 0.05 mmol) were added, and the mixture was stirred under microwave irradiation (50 °C, 25 W) for 3 hrs. After completion, the solvents were evaporated *in vacuo*, the crude product was dissolved in EtOAc and treated with saturated aqueous NaHCO₃, IN KHSO₄ (aq) and brine. The organic layer was then dried over Na₂SO₄ and the solvents were evaporated *in vacuo*. Crude compound **(19)** was used as such in the next step. ESI-MS (ESI+) *m*/*z*: 1049.4 [M+2Na]²⁺.

### iPr-NHCO-Leu^{u}-Glu^{u}-Leu^{u}-Lys^{u}-Pro^{u}-Leu^{u}-Glu^{u}-Leu^{u}-Lys^{u}-Ala^{u}-NHMe (H2)

Compound **(19)** (0.065 g, 0.031 mmol) was dissolved in a mixture 5:2:1 of EtOH/H₂O/Acetic acid and stirred under an atmosphere of Ar. Pd/C 10 % (0.006 g) was added, Ar was replaced by H₂ and the mixture was stirred at room temperature for 24 hrs. The Pd/C was then removed by filtration on Millipore paper and washed with the solvent mixture used for the reaction. EtOH was completely evaporated *in vacuo*, the crude product was freeze-dried and purified by semi-preparative HPLC (25-70 % CH₃CN 0.1 % TFA in H₂O 0.1 % TFA, 20 min; Macherey-Nagel Nucleodur 100-16 C18 ec, 10 x 250). Pure **H2** was freeze-dried and TFA was exchanged with HCl by repeated lyophilisations in 0.1 N HCl. ESI-MS (ESI+) *m*/*z*: 771.6

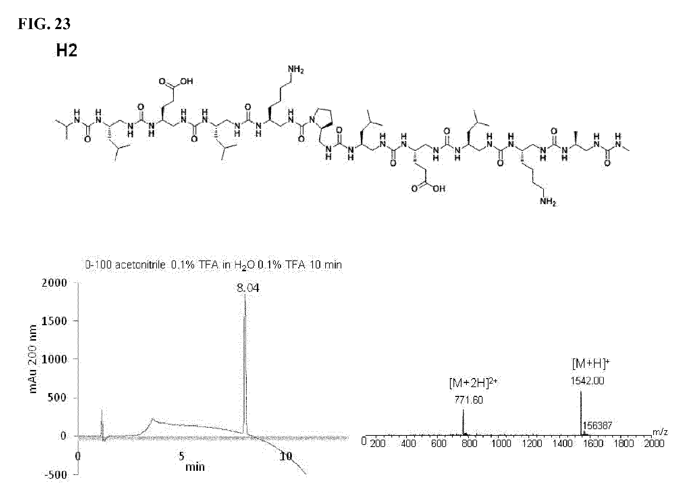

[M+2H]²⁺, 1541.9 [M+H]⁺; HPLC: Rₜ= 8.04 min (0-100 % CH₃CN 0.1 % TFA in H₂O 0.1 % TFA, 10 min, Macherey-Nagel, Nucleodur cc 70/4 100-3 C18 ec, 4.6 x 100, 1 ml/min) (Fig. 23).

### Azido building block synthesis for oligourea synthesis on solid support

The building blocks containing Ser-, Glu- and Lys-type side chains were synthesized as previously reported (Douat-Casassus, C., et al. Microwave-enhanced solid-phase synthesis of N,N'-linked aliphatic oligoureas and related hybrids. Org. Lett. 14, 3130-3133 (2012)), following path A, starting from the *N*-Cbz protected (L) amino acid. The Asn type building block was synthesized following path B described in the same report (Douat-Casassus, C., et al. Microwave-enhanced solid-phase synthesis of N,N'-linked aliphatic oligoureas and related hybrids. Org. Lett. 14, 3130-3133 (2012)), starting from the *N*-Fmoc protected amino acid. Fmoc deprotection was performed using DBU, as previously described (Zhang, Z. & Fan, E. Solid-phase and solution-phase syntheses of oligomeric guanidines bearing peptide side chains. J. Org. Chem. 70, 8801-8810 (2005)).

### N₃-Ser: (R)-2,5-dioxopyrrolidin-1-yl (2-azido-3-(tert-butoxy)propyl)carbamate (20)

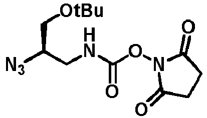

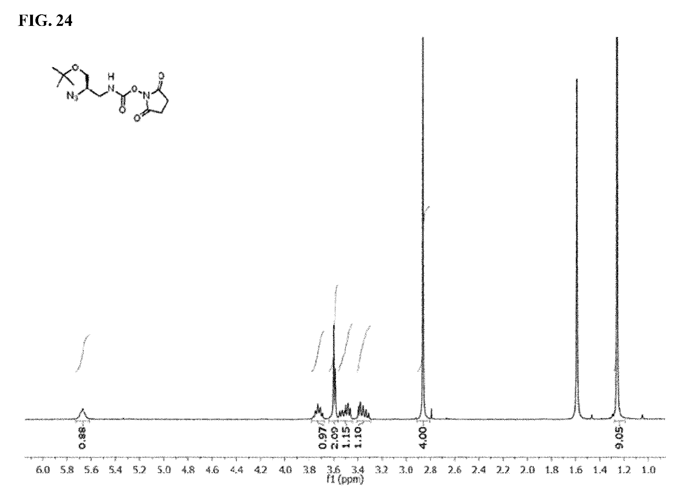

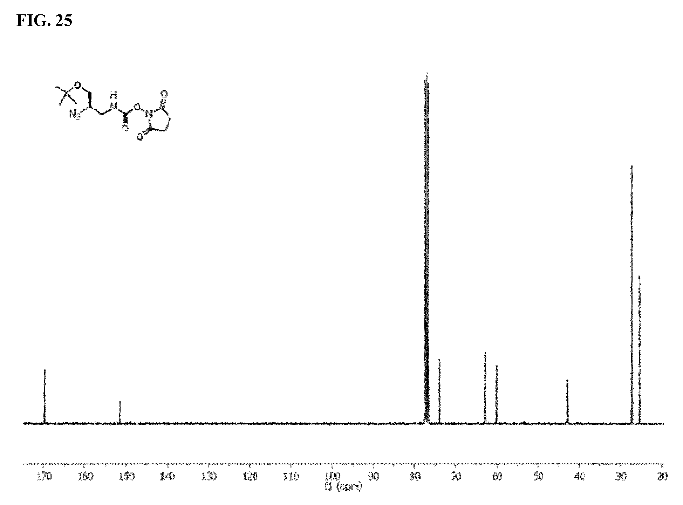

The pure product was obtained as a pale yellow solid, with an overall yield of 41% after 6 steps. ¹H NMR (CDCl₃, 300 MHz) δ: 5.67 (bs, 1H), 3.67 - 3.69 (m, 1H), 3.59 (d, *J* = 5.1 Hz, 2H), 3.50 (ddd, *J* = 13.8, 6.2, 4.9 Hz, 1H), 3.42 - 3.29 (m, 1H), 2.86 (s, 4H), 1.26 (s, 9H); ¹³C NMR (CDCl₃, 75 MHz) δ: 169.73, 151.54, 74.06, 62.81, 60.21, 43.00, 27.27, 25.48; HRMS (ESI+) *m*/*z:* calcd for C₁₂H₁₉N₅O₅Na [M+H]⁺ 336.1278, found 336.1285 (Fig. 24 and 25).

### N₃-Glu: (S)-tert-butyl4-azido-5-((((2,5-dioxopyrrolidin-1-yl)oxy)carbonyl)amino) pentanoate (21)

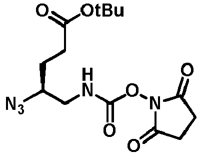

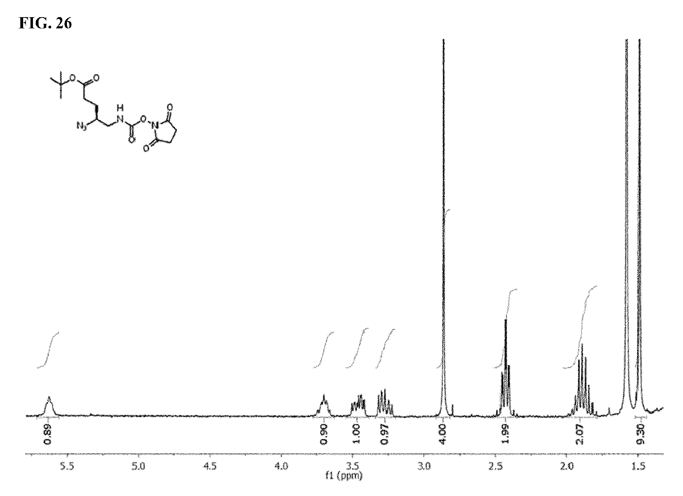

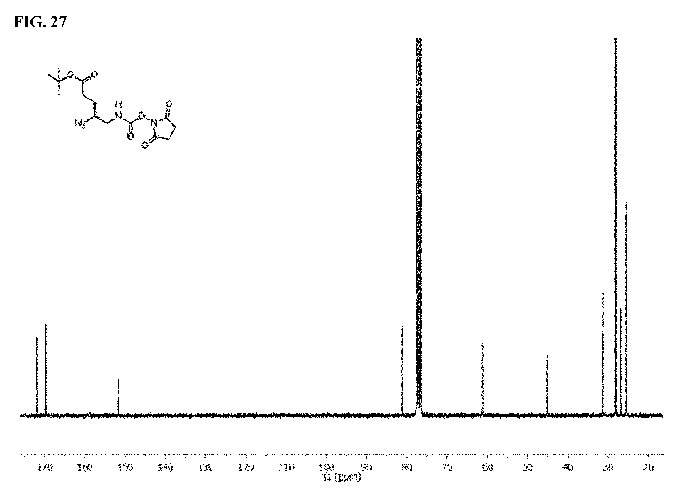

The pure product was obtained as an off white solid, with an overall yield of 38% after 6 steps. ¹H NMR (CDCl₃, 300 MHz) δ: 5.63 (bs, 1H), 3.74 - 3.66 (m, 1H), 3.46 (ddd, *J* = 14.2, 6.5, 4.5 Hz, 1H), 3.34 - 3.21 (m, 1H), 2.86 (s, 4H), 2.43 (td, *J* = 7.1, 1.9 Hz, 2H), 2.01 - 1.78 (m, 2H), 1.49 (s, 9H), 1.23 (d, *J* = 6.2 Hz, 3H); ¹³C NMR (CDCl₃, 75 MHz) δ: 171.97, 169.73, 151.67, 81.08, 61.06, 45.02, 31.27, 28.08, 26.84, 25.48; HRMS (ESI+) *m*/*z*: calcd for C₁₄H₂₂N₅O₆ [M+H]⁺ 356.1572, found 356.1572 (Fig. 26 and 27).

### N₃-Lys: (S)-2,5-dioxopyrrolidin-1-yl (2-azido-5-N-tert-butoxycarbonylaminohexyl) carbamate (22)

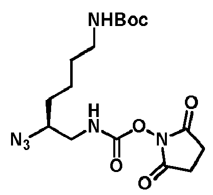

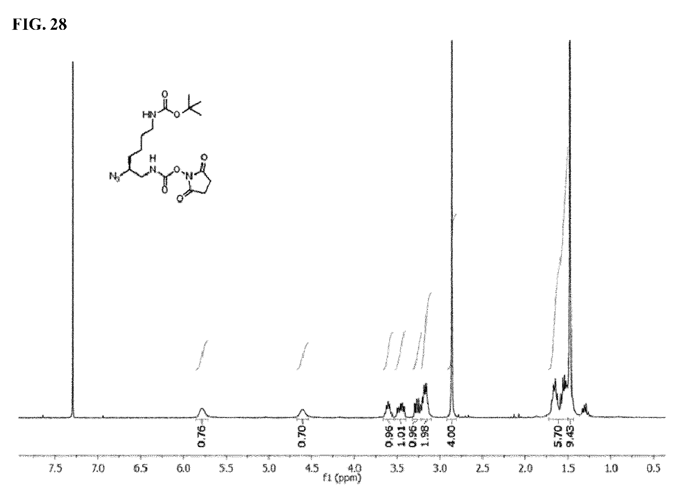

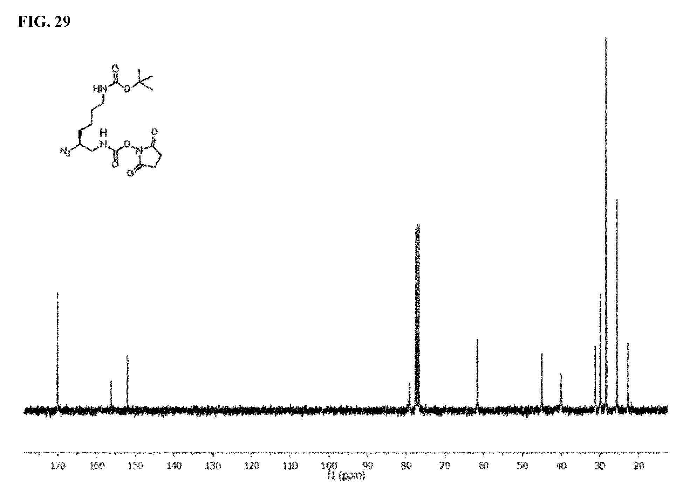

The pure product was obtained as an off white solid, with an overall yield of 35% after 6 steps. ¹H NMR (CDCl₃, 300 MHz) δ: 5.78 (bs, 1H), 4.61 (bs, 1H), 3.67 - 3.54 (m, 1H), 3.46 (ddd, *J* = 13.9, 6.5, 4.4 Hz, 1H), 3.32 - 3.21 (m, 1H), 3.17 (dd, *J* = 12.2, 6.1 Hz, 2H), 2.83 (s, 4H), 1.69 - 1.45 (m, 6H), 1.48 (s, 9H); ¹³C NMR (CDCl₃, 75 MHz) δ: 170.10, 156.19, 151.92, 79.24, 61.66, 45.01, 40.00, 31.17, 29.78, 28.42, 25.48, 22.71; HRMS (ESI+) *m*/*z*: calcd for C₁₆H₂₇N₆O₆ [M+H]⁺ 399.1994, found 399.1993 (Fig. 28 and 29).

### N₃-Asn: (S)-2,5-dioxopyrrolidin-1-yl (2-azido-4-oxo-4-(tritylamino)butyl)carbamate (23)

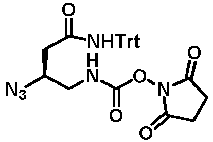

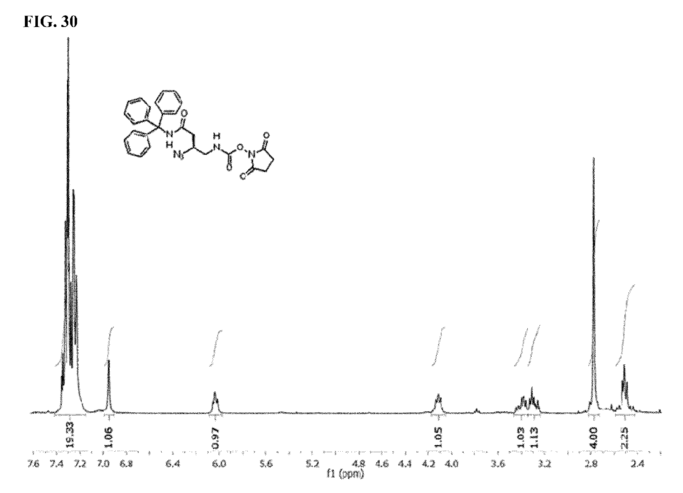

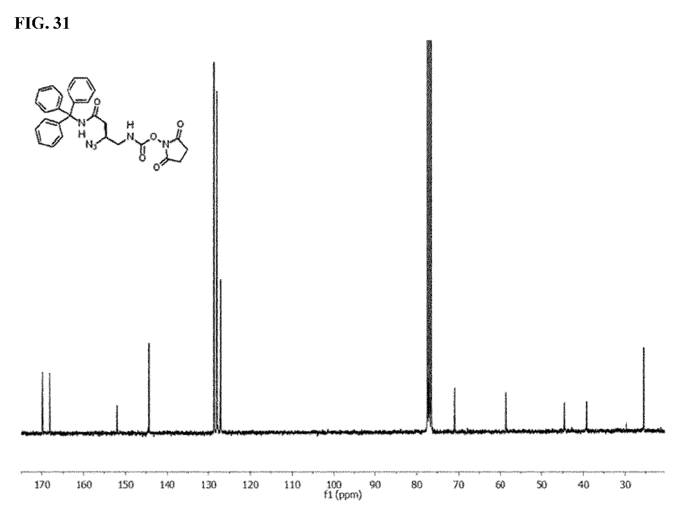

The pure product was obtained as a pale yellow solid, with an overall yield of 27 % after 6 steps. ¹H NMR (CDCl₃, 300 MHz) δ: 7.36-7.23 (m, 15H), 6.95 (s, 1H), 6.04 (t, *J* = 6.2 Hz, 1H), 4.16 - 4.08 (m, 1H), 3.40 (ddd, *J* = 12.5 , 7.1, 5.3 Hz, 1H), 3.29 (dt, *J* = 14.2, 5.2 Hz, 1H), 2.78 (s, 4H), 2.59 - 2.42 (m, 2H); ¹³C NMR (CDCl₃, 75 MHz) δ: 169.84, 168.18, 152.04, 144.30, 128.70, 128.04, 127.16, 70.94, 58.53, 44.64, 39.24, 25.45. HRMS (ESI+) *m*/*z*: calcd for C₂₈H₂₇N₆O₅ [M+H]⁺ 527.2045, found 527.2037 (Fig. 30 and 31).

### Solid phase synthesis of oligoureas H1, H3, H4 and H5

Compounds **H1, H3, H4** and **H5** were assembled following previously reported procedures (Douat-Casassus, C., et al. Microwave-enhanced solid-phase synthesis of N,N'-linked aliphatic oligoureas and related hybrids. Org. Lett. 14, 3130-3133 (2012)) in a polypropylene SPE tube (CEM) using microwave assisted solid phase synthesis (CEM DiscoveryBio) on NovaPeg Rink Amide resin (Novabiochem) using variable scales (0.02-0.1 mmol). The syntheses were monitored using the chloranil test. Unless stated otherwise, the reagents were purchased from Sigma Aldrich. Coupling: N₃-OSu activated building blocks (3 eq) were dissolved in DMF (Carlo Erba Reagents) in the presence of DIEA (6 eq) and reacted with the resin at 50 W, 50 °C for 40 min. Azide reduction: the resin was swelled using a mixture of 7/3 1,4-dioxane/H₂O and reacted with a solution of Me₃P 1M in THF (10 eq) at 50 W, 50 °C for 15 min under an atmosphere of N₂. This procedure was repeated two to four times, until a positive chloranil test was observed. N-terminus isopropylation: the final isopropyl protection was performed by reacting the resin with isopropyl isocyanate (3 eq) in the presence of DIEA (6 eq) in DMF at 50 W, 50 °C for 10 min. This procedure was repeated if necessary. Resin cleavage and purification: the dried resin was treated with a mixture of 95:2.5:2.5 TFA: H₂O:TIS and shaken at room temperature for 2.5 hrs (or overnight in the case of oligoureas containing Ser(tBu) side chains). The crude products were precipitated as TFA salts with Et₂O and purified with the appropriate gradient by semipreparative HPLC (Dionex Ultimate 3000, column Macherey-Nagel Nucleodur 100-16 C18 ec, 10 x 250, solvents acetonitrile/water 0.1 % TFA, 4 ml/min). Analytic HPLC characterizations were performed on Macherey-Nagel column, Nucleodur cc 70/4 100-3 C18 ec, 4.6 x 100, 1 ml/min, solvents acetonitrile/water 0.1 % TFA, 1 ml/min. The compounds were freeze-dried and TFA was exchanged with HCl by repeated lyophilisations in 0.1 N HCl.

### iPr-NHCO-Leu^{u}-Glu^{u}-Lys^{u}-Leu^{u}-Tyr^{u}-Leu^{u}-Glu^{u}-Lys^{u}-Leu^{u}-Ala^{u}-Leu^{u}-NH₂ (H1)

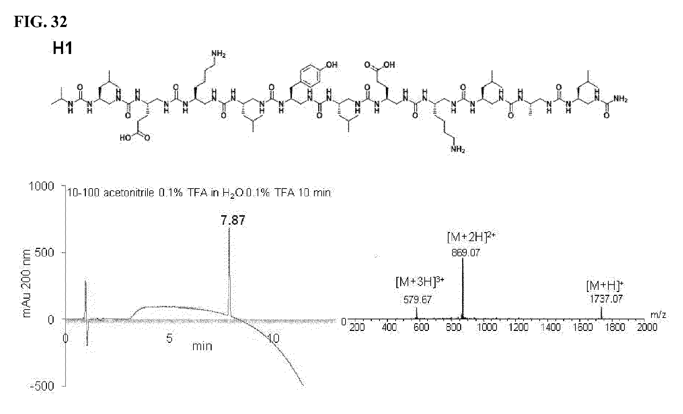

ESI-MS (ESI+) *m*/*z*: 579.67 [M+3H]³⁺, 869.07 [M+2H]²⁺, 1737.07 [M+H]⁺; HPLC: Rₜ= 7.87 min (10-100% CH₃CN 0.1 % TFA in H₂O 0.1 % TFA, 10 min, C18) (Fig. 32).

### iPr-NHCO-Leu^{u}-Glu^{u}-Lys^{u}-Leu^{u}-Tyr^{u}-Asn^{u}-Glu^{u}-Lys^{u}-Leu^{u}-Ala^{u}-Leu^{u}-NH₂ (H3)

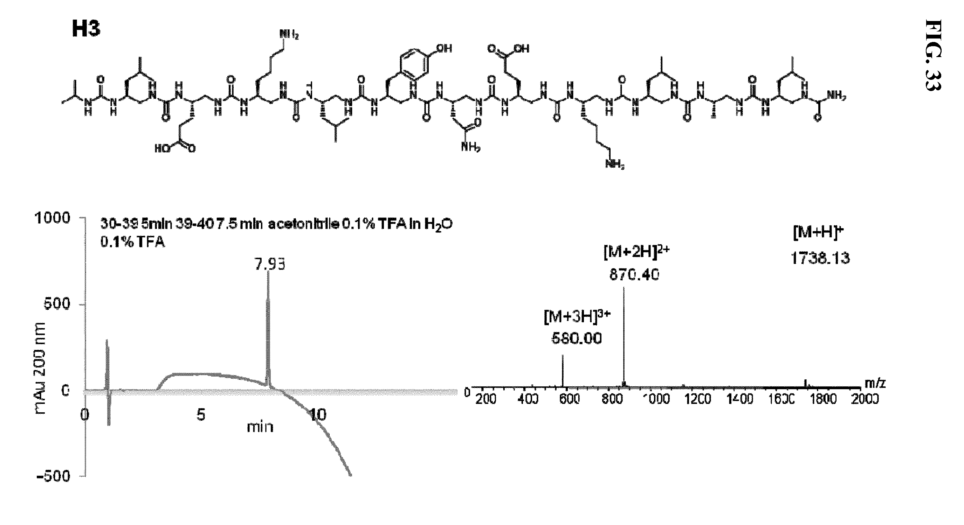

ESI-MS (ESI+) *m*/*z*: 580.00 [M+3H]³⁺, 870.40 [M+2H]²⁺, 1738.13 [M+H]⁺; HPLC: Rₜ= 7.93 min (30-39 5 min 39-40 7.5 min CH₃CN 0.1 % TFA in H₂O 0.1 % TFA, C18) (Fig. 33).

### iPr-NHCO-Ser^{u}-Glu^{u}-Lys^{u}-Leu^{u}-Tyr^{u}-Leu^{u}-Glu^{u}-Lys^{u}-Leu^{u}-^{u}-Leu^{u}-NH₂ (H4)

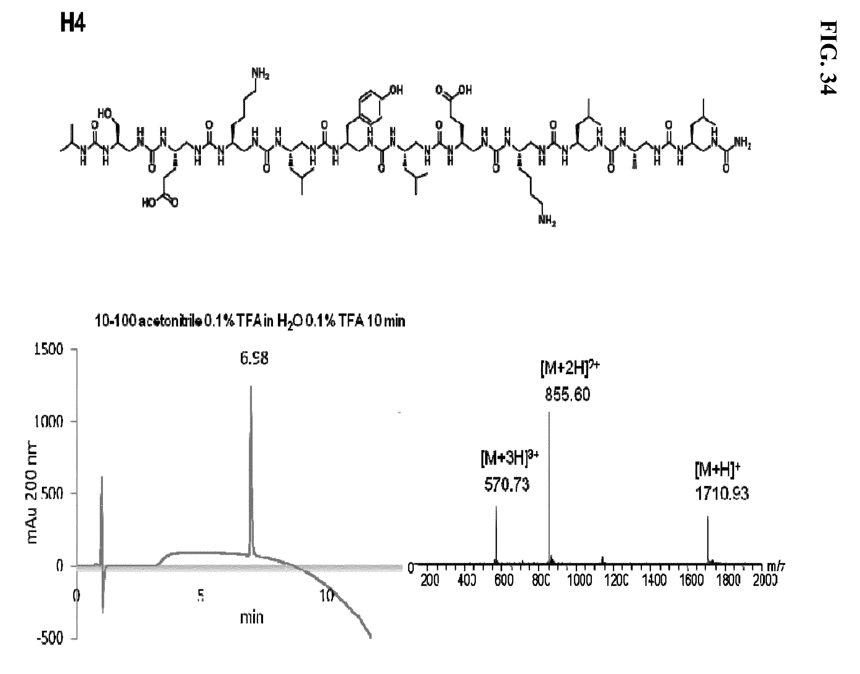

ESI-MS (ESI+) *m*/*z*: 570.73 [M+3H]³⁺, 855.60 [M+2H]²⁺, 1710.93 [M+H]⁺; HPLC: Rₜ= 6.98 min (10-100 % CH₃CN 0.1 % TFA in H₂O 0.1 % TFA, 10 min, C18) (Fig. 34).

### iPr-NHCO-Ala^{u}-Leu^{u}-Lys^{u}-Leu^{u}-Glu^{u}-Tyr^{u}-Leu^{u}-Glu^{u}-Leu^{u}-Lys^{u}-Ala^{u}-Leu^{u}-NH₂ (H5)

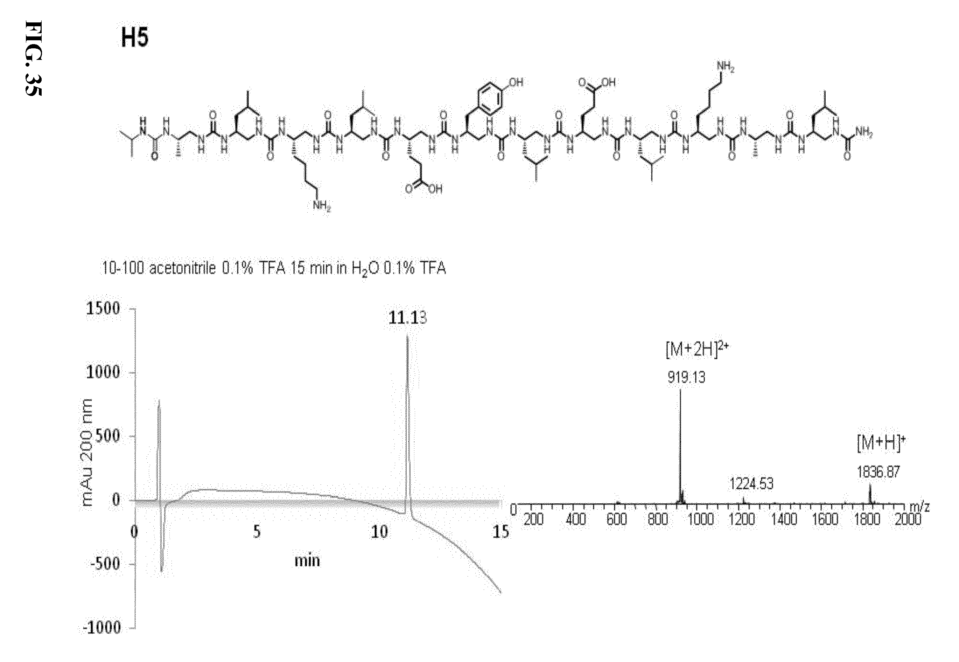

ESI-MS (ESI+) *m*/*z* 919.13 [M+2H]²⁺, 1836.87 [M+H]⁺; HPLC: Rₜ= 11.13 min (10-100 % CH₃CN 0.1 % TFA in H₂O 0.1 % TFA, 15 min, C18) (Fig. 35).

Thus, the experimental results demonstrate that peptide-oligourea chimeric foldamers (compounds having a polypeptide portion contiguous with or linked to oligomers of amino acids having an N, N'-linked urea bridging unit) demonstrate enhanced or improved properties relative to the parental or cognate "natural" peptide. Oligoureas can be derived from building blocks with any desired amino acid side chain. In particular, the chimeric compounds as described herein demonstrate regular and persistant helical conformations and improved helix stability. Because the chimeric foldamers as described herein can adopt desired secondary structures similar to native peptides, including, e.g., linear, cyclic or helicoidal structures, they can serve as, for example, receptor ligands, effector molecules, agonists, antagonists, modulators of protein-protein interactions, organocatalysts or enzymes.

While preferred embodiments of the disclosure have been shown and described herein, it will be understood that such embodiments are provided by way of example only. Numerous variations, changes and substitutions will occur to those skilled in the art without departing from the spirit of the disclosure. Accordingly, it is intended that the appended claims cover all such variations as fall within the spirit and scope of the disclosure.

The contents of all references, patents, pending patent applications and published patents, cited throughout this application are hereby expressly incorporated by reference.

Those skilled in the art will recognize, or be able to ascertain using no more than routine experimentation, many equivalents to the specific embodiments of the disclosure described herein. Such equivalents are intended to be encompassed by the following claims. It is understood that the detailed examples and embodiments described herein are given by way of example for illustrative purposes only, and are in no way considered to be limiting to the disclosure. Various modifications or changes in light thereof will be suggested to persons skilled in the art and are included within the spirit and purview of this application and are considered within the scope of the appended claims. For example, the relative quantities of the ingredients may be varied to optimize the desired effects, additional ingredients may be added, and/or similar ingredients may be substituted for one or more of the ingredients described. Additional advantageous features and functionalities associated with the systems, methods, and processes of the present disclosure will be apparent from the appended claims. Moreover, those skilled in the art will recognize, or be able to ascertain using no more than routine experimentation, many equivalents to the specific embodiments of the disclosure described herein. Such equivalents are intended to be encompassed by the following claims.

## Claims

1. A compound comprising a non-peptide oligourea helical foldamer.

2. The compound of claim 1, wherein the non-peptide oligourea helical foldamer is selected from the group consisting of:
an aliphatic non-peptide oligourea helical foldamer;
a short amphiphilic α-helicomimetic foldamer with proteinaceous side-chains;
a non-peptide oligourea peptidomimetic residue sequence selected from the group consisting of:
Leu^{u} Glu^{u} Lys^{u} Leu^{u} Tyr^{u} Leu^{u} Glu^{u} Lys^{u} Leu^{u} Ala^{u} Leu^{u} (H1);
Leu^{u} Glu^{u} Leu^{u} Lys^{u} Pro^{u} Leu^{u} Glu^{u} Leu^{u} Lys^{u} Ala^{u} (H2);
Leu^{u} Glu^{u} Lys^{u} Leu^{u} Tyr^{u} Asn^{u} Glu^{u} Lys^{u} Leu^{u} Ala^{u} Leu^{u} (H3) Ser^{u} Glu^{u} Lys^{u} Leu^{u} Tyr^{u} Leu^{u} Glu^{u} Lys^{u} Leu^{u} Ala^{u} Leu^{u} (H4);
Ala^{u} Leu^{u} Lys^{u} Leu^{u} Glu^{u} Tyr^{u} Leu^{u} Glu^{u} Leu^{u} Lys^{u} Ala^{u} Leu^{u} (H5);
Leu^{u} Glu^{u} Lys^{u} Leu^{u} Tyr^{u} Leu^{u} Asn^{u} Lys^{u} Leu^{u} Ala^{u} Leu^{u} (H6); and
Leu^{u} Glu^{u} Lys^{u} Leu^{u} Tyr^{u} Leu^{u} Gln^{u} Lys^{u} Leu^{u} Ala^{u} Leu^{u} (H7),
wherein the oligourea helical foldamer has an isolated cavity, a pH-responsive water-filled channel with controllable pore diameter or a pH-responsive superhelical channel with a water-filled pore within the hydrophobic core of a bundle.

3. The compound of claim 1, wherein the oligourea helical foldamer is a helical bundle, or a helical bundle that self-assembles into a three-dimensional nanostructure in aqueous conditions.

4. The compound of claim 1, wherein the compound has a secondary structure similar to a native peptide.

5. The compound of claim 4, wherein the secondary structure acts as a receptor ligand, an effector molecule, an agonist, an antagonist, a modulator of protein-protein interactions, an organocatalyst, or an enzyme.

6. The compound of claim 1, wherein the compound has a secondary structure that provides functions not found in nature.

7. The compound of claim 1, wherein the compound is biologically active.

8. The compound of claim 1, further comprising a peptide that is fused to, contiguous with, or conjugated to the oligourea helical bundle.

9. The compound of claim 8, wherein the peptide segment comprises an amino acid sequence corresponding to a biologically active peptide or a fragment thereof.

10. The compound of claim 9, wherein the peptide is a peptide α-helix.

11. The compound of claim 10, wherein the peptide α-helix includes an epitope that recognizes another compound.

12. The compound of claim 11, wherein the epitope acts as a receptor ligand, an effector molecule, an agonist, an antagonist, a modulator of protein-protein interactions, an organocatalyst, or an enzyme.

13. The compound of claim 1, wherein the oligourea helical bundle encapsulates a drug or substrate.

14. The compound of claim 1, wherein the oligourea helical bundle transfers substances across a membrane.

15. The compound of claim 1, wherein the oligourea helical bundle has an internal cavity with a volume in a range of about 400-600 Å³.

16. A peptide-oligourea chimeric compound, comprising:
a peptide; and
a oligourea helical bundle.

17. The compound of claim 16, wherein the oligourea helical bundle self-assembles into its three-dimensional nanostructure under aqueous conditions.

18. The compound of claim 16, wherein the peptide is a peptide α-helix.

19. The compound of claim 16, wherein:
the non-peptide oligourea helical foldamer includes aliphatic olgioureas;
the non-peptide oligourea helical foldamer is a short amphiphilic α-helicomimetic foldamer with proteinaceous side-chains;
the oligourea helical bundle has a non-peptide oligourea peptidomimetic residue sequence selected from the group consisting of:
Leu^{u} Glu^{u} Lys^{u} Leu^{u} Tyr^{u} Leu^{u} Glu^{u} Lys^{u} Leu^{u} Ala^{u} Leu^{u} (H1);
Leu^{u} Glu^{u} Leu^{u} Lys^{u} Pro^{u} Leu^{u} Glu^{u} Leu^{u} Lys^{u} Ala^{u} (H2);
Leu^{u} Glu^{u} Lys^{u} Leu^{u} Tyr^{u} Asn^{u} Glu^{u} Lys^{u} Leu^{u} Ala^{u} Leu^{u} (H3) Ser^{u} Glu^{u} Lys^{u} Leu^{u} Tyr^{u} Leu^{u} Glu^{u} Lys^{u} Leu^{u} Ala^{u} Leu^{u} (H4); or
Ala^{u} Leu^{u} Lys^{u} Leu^{u} Glu^{u} Tyr^{u} Leu^{u} Glu^{u} Leu^{u} Lys^{u} Ala^{u} Leu^{u} (H5);
Leu^{u} Glu^{u} Lys^{u} Leu^{u} Tyr^{u} Leu^{u} Asn^{u} Lys^{u} Leu^{u} Ala^{u} Leu^{u} (H6); and
Leu^{u} Glu^{u} Lys^{u} Leu^{u} Tyr^{u} Leu^{u} Gln^{u} Lys^{u} Leu^{u} Ala^{u} Leu^{u} (H7),
wherein the oligourea helical bundle has an isolated cavity, a pH-responsive water-filled channels with controllable pore diameters or a pH-responsive superhelical channel with water-filled pores within the hydrophobic core of the bundle.

20. The compound of claim 16, wherein the oligourea helical bundle has an internal cavity with a volume in a range of about 400-600 Å³.

21. A therapeutic composition comprising an effective amount of the compound of claim 1 and a pharmaceutically acceptable carrier or excipient.

22. A method of treating a disease in a patient comprising administering to an individual in need thereof, and effective amount of the composition of claim 19, wherein the composition is effective in ameliorating the disease or condition.

23. A method of synthesizing a oligourea compound comprising the steps of:
fabricating a oligourea helical bundle with a non-peptide oligourea peptidomimetic residue sequence selected from the group consisting of :
a helical pentad repeat (*a, b, c, d, e):*
oligourea peptidomimetic residues with a hydrophobic side chain in positions *a* and *d* (e.g., Leu^{u});
oligourea peptidomimetic residues with a charged residue in position *b* and *c* (e.g. Glu^{u} and Lys^{u}, respectively); and
Tyr^{u} and Ala^{u} in position e;
a helical pentad repeat (*a, b, c, d, e):*
oligourea peptidomimetic residues with a hydrophobic side chain in positions a and c (e.g. Leu^{u});
oligourea peptidomimetic residues with a hydrophobic side chain in position e (e.g., Ala^{u} and Pro^{u} residues at the e position); and
oligourea peptidomimetic residues with a charged side chain in positions *b* and *d* (e.g., Glu^{u} and Lys^{u}, respectively);
Leu^{u} Glu^{u} Lys^{u} Leu^{u} Tyr^{u} Leu^{u} Glu^{u} Lys^{u} Leu^{u} Ala^{u} Leu^{u} (H1);
Leu^{u} Glu^{u} Leu^{u} Lys^{u} Pro^{u} Leu^{u} Glu^{u} Leu^{u} Lys^{u} Ala^{u} (H2);
Leu^{u} Glu^{u} Lys^{u} Leu^{u} Tyr^{u} Asn^{u} Glu^{u} Lys^{u} Leu^{u} Ala^{u} Leu^{u} (H3) Ser^{u} Glu^{u} Lys^{u} Leu^{u} Tyr^{u} Leu^{u} Glu^{u} Lys^{u} Leu^{u} Ala^{u} Leu^{u} (H4);
Ala^{u} Leu^{u} Lys^{u} Leu^{u} Glu^{u} Tyr^{u} Leu^{u} Glu^{u} Leu^{u} Lys^{u} Ala^{u} Leu^{u} (H5);
Leu^{u} Glu^{u} Lys^{u} Leu^{u} Tyr^{u} Leu^{u} Asn^{u} Lys^{u} Leu^{u} Ala^{u} Leu^{u} (H6); and
Leu^{u} Glu^{u} Lys^{u} Leu^{u} Tyr^{u} Leu^{u} Gln^{u} Lys^{u} Leu^{u} Ala^{u} Leu^{u} (H7).


SEQUENCE LISTING

<110>  Centre National De La Recherche Scientifique (CNRS)
       Guichard, Gilles

<120>  QUATERNARY ASSEMBLIES OF WATER-SOLUBLE NON-PEPTIDE HELICAL
       FOLDAMERS, THEIR USE AND PRODUCTION THEREOF

<130>  1513195.129US0

<140>  TBD
<141>  2016-08-29

<150>  US 62/212,590
<151>  2015-09-01

<160>  7

<170>  PatentIn version 3.5

<210>  1
<211>  11
<212>  PRT
<213>  Artificial Sequence

<220>
<223>  Synthetic


<220>
<221>  MISC_FEATURE
<222>  (1)..(11)
<223>  At least one of 1-11 is an oligourea peptidomimetic residue

<400>  1

Leu Glu Lys Leu Tyr Leu Glu Lys Leu Ala Leu
1               5                   10


<210>  2
<211>  10
<212>  PRT
<213>  Artificial Sequence

<220>
<223>  Synthetic


<220>
<221>  MISC_FEATURE
<222>  (1)..(10)
<223>  At least one of 1-10 is an oligourea peptidomimetic residue

<400>  2

Leu Glu Leu Lys Pro Leu Glu Leu Lys Ala
1               5                   10


<210>  3
<211>  11
<212>  PRT

<213>  Artificial Sequence

<220>
<223>  Synthetic


<220>
<221>  MISC_FEATURE
<222>  (1)..(11)
<223>  At least one of 1-11 is an oligourea peptidomimetic residue

<400>  3

Leu Glu Lys Leu Tyr Asn Glu Lys Leu Ala Leu
1               5                   10


<210>  4
<211>  11
<212>  PRT
<213>  Artificial Sequence

<220>
<223>  Synthetic


<220>
<221>  MISC_FEATURE
<222>  (1)..(11)
<223>  At least one of 1-11 is an oligourea peptidomimetic residue

<400>  4

Ser Glu Lys Leu Tyr Leu Glu Lys Leu Ala Leu
1               5                   10


<210>  5
<211>  12
<212>  PRT
<213>  Artificial Sequence

<220>
<223>  Synthetic


<220>
<221>  MISC_FEATURE
<222>  (1)..(12)
<223>  At least one of 1-12 is an oligourea peptidomimetic residue

<400>  5

Ala Leu Lys Leu Glu Tyr Leu Glu Leu Lys Ala Leu
1               5                   10


<210>  6
<211>  11
<212>  PRT
<213>  Artificial Sequence

<220>

<223>  Synthetic

<220>
<221>  MISC_FEATURE
<222>  (1)..(11)
<223>  At least one of 1-11 is an oligourea peptidomimetic residue

<400>  6

Leu Glu Lys Leu Tyr Leu Asn Lys Leu Ala Leu
1               5                   10

<210>  7
<211>  11
<212>  PRT
<213>  Artificial Sequence

<220>
<223>  Synthetic

<220>
<221>  MISC_FEATURE
<222>  (1)..(11)
<223>  At least one of 1-11 is an oligourea peptidomimetic residue

<400>  7

Leu Glu Lys Leu Tyr Leu Gln Lys Leu Ala Leu
1               5                   10